(19) Europäisches Patentamt
European Patent Office
Office européen des brevets

(11) Publication number: 0 498 680 A1

# (12) EUROPEAN PATENT APPLICATION

(21) Application number: **92301100.1**

(22) Date of filing: **10.02.92**

(51) Int. Cl.[5]: **C07D 207/16,** C07K 5/06, C07D 401/12, C07D 403/12, C07D 405/12, C07D 409/12, A61K 31/40, C07D 413/12, C07D 417/12, C07D 217/26

(30) Priority: **08.02.91 JP 17341/91**

(43) Date of publication of application: **12.08.92 Bulletin 92/33**

(84) Designated Contracting States: **AT BE CH DE DK ES FR GB GR IT LI LU MC NL PT SE**

(71) Applicant: **Sankyo Company Limited**
**5-1 Nihonbashi Honcho 3-chome Chuo-ku**
**Tokyo (JP)**

(72) Inventor: **Yabe, Yuichiro**
**c/o Sankyo Company Limited, 2-58 Hiromachi 1-chome**
**Shinagawa-ku, Tokyo 140 (JP)**

Inventor: **Sakurai, Mitsuya**
**c/o Sankyo Company Limited, 2-58 Hiromachi 1-chome**
**Shinagawa-ku, Tokyo 140 (JP)**
Inventor: **Higashida, Susumu**
**c/o Sankyo Company Limited, 2-58 Hiromachi 1-chome**
**Shinagawa-ku, Tokyo 140 (JP)**
Inventor: **Komai, Tomoaki**
**c/o Sankyo Company Limited, 2-58 Hiromachi 1-chome**
**Shinagawa-ku, Tokyo 140 (JP)**
Inventor: **Nishigaki, Takashi**
**c/o Sankyo Company Limited, 2-58 Hiromachi 1-chome**
**Shinagawa-ku, Tokyo 140 (JP)**
Inventor: **Handa, Hiroshi**
**17-16, Sakurajosui 1-chome**
**Setagaya-ku, Tokyo 156 (JP)**

(74) Representative: **Gibson, Christian John Robert et al**
**MARKS & CLERK 57/60 Lincoln's Inn Fields**
**London WC2A 3LS (GB)**

## (54) New beta-amino-alpha-hydroxycarboxylic acids and their use.

(57) The compounds of the invention are β-amino-α-hydroxycarboxylic acid derivatives having the formula (I):

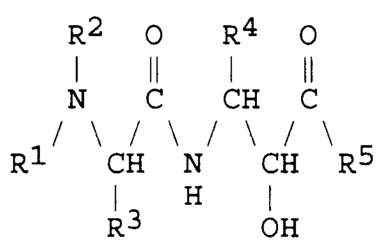

(I)

wherein:

$R^1$ represents a group of formula $R(Z)_xA$-

wherein R is hydrogen, or a substituted or unsubstituted alkyl, alkenyl, alkynyl, aryl or heterocyclic group or R is a group of formula $-NR^aR^b$ wherein $R^a$ and $R^b$ are hydrogen atoms, alkyl groups, unsubstituted or substituted cycloalkyl, heterocyclic, aralkenyl or aryl groups,

Z represents oxygen or sulphur;

A represents a group of formula -CO-, -COCO-, -SO-, $-SO_2$- or -CS-; and

x is the cipher 0 or the integer 1;

$R^2$ represents a hydrogen atom, or a substituted or unsubstituted alkyl group;

$R^3$ is hydrogen, or a substituted or unsubstituted alkyl, alkenyl, alkynyl, aryl or heterocyclic group;

EP 0 498 680 A1

$R^4$ is a hydrogen atom, a substituted or unsubstituted alkyl, aryl or heterocyclic group ;

and

$R^5$ represents a group of formula $-B-(CO)-Y-R^c_y$ , wherein

B represents a heterocyclic group unsubstituted or substituted with at least one alkyl group ;

Y represents an oxygen atom, a nitrogen atom or a sulphur atom ;

y is the integer 1 when Y represents an oxygen atom or a sulphur atom, and 2 when Y represents a nitrogen atom ; and

$R^c$ represents

is hydrogen, or a substituted or unsubstituted alkyl, alkenyl, alkynyl, aryl or heterocyclic group ;

and, where there are two groups or atoms represented by $R^c$, they are the same or different ; and and pharmaceutically acceptable salts and esters thereof.

The present invention relates to a series of new β-amino-α-hydroxycarboxylic acid derivatives which are of particular value in the treatment of retroviral infections, in particular AIDS, as well as to a process for their production and the use of the compounds in such treatment.

The human immunodeficiency virus (HIV) has been the target of various research efforts for some considerable years, but there is still no definitive treatment for the disease condition it causes, acquired immune deficiency syndrome (AIDS).

One main reason that research has yet to reveal a cure or treatment for AIDS is that the virus belongs to that group of viruses known as the retroviruses. These viruses do not replicate as normal viruses. Normal viruses infect an organism and take over the machinery of the host cells. The host cells are then forced to produce the virus in large quantities.

Retroviruses, on the other hand, go one step further. The first action on invading a cell is to incorporate the genetic material of the virus into the genetic material of the cell. The two sets of genetic material then become impossible to tell apart. Thus, where there is the possibility of targetting the invader when the infection is by a normal virus, there is no possibilty when the infectious agent is a retrovirus, as the virus becomes a part of the host.

However, the retrovirus must use enzymes unique to the retroviruses in order to insert its genetic material into that of the host, and must also provide unique products when the mature virus is being assembled in the cell. These unique products, therefore, provide one route by which retroviruses can be attacked.

Replication in retroviruses involves extensive post-translation processing of precursor polyproteins by proteolytic enzymes made by the virus. Mature viral proteins which are essential for virus assembly and function are the result of this processing. It has been shown that interruption of the processing has an inhibitory effect on the production of virus particles.

HIV protease is encoded by the pol gene on the HIV genome, as are the reverse transcriptase, an endonuclease and RNaseH. In the absence of any one of these enzymes, HIV cannot successfully replicate.

Azidothymidine (AZT) is a well known anti-HIV agent, and has been approved for AIDS therapy. AZT works by interfering with HIV reverse transcriptase. This function of HIV is unique to retroviruses, and is used by the virus to incorporate viral genetic material into the host chromosomes. Interfering with this function should then block infection. However, it is also well known that AZT is associated with certain undesirable side-effects, such as hematological toxicity, and so there is still a requirement to identify further, useful anti-HIV agents.

A key step in the replication of HIV takes place when the HIV-1 protease cleaves specific bonds in the precursor gag and pol proteins to produce the mature proteins necessary for production of infectious viral particles. HIV protease has been targeted as a possible intervention point in the treatment of AIDS, and attempts have been made to produce compounds which inhibit this protease.

Compounds having HIV protease inhibiting activity have been described, for example, in J. Med. Chem. [33, 2687 (1990)], where $C_2$ symmetric homodimers are described as having high specificity and good anti-HIV activity in vitro. Compounds described in J. Med. Chem [34, 1222 (1991)], contain hydroxyethylamines derived from phenylalanyl-proline, and apparently have good HIV protease inhibitory activity. Hydroxyethylamine dipeptidyl isosteres, which were designed to mimic the tetrahedral hydrolysis intermediate of tyrosyl-proline or phenylalanyl-proline, which are partial sequences cleaved by HIV protease, are described in J. Med. Chem. [33, 1285 (1990)] and Science [248, 358 (1990)].

Further amino acid derivatives having HIV protease inhibiting activity are described in European Patent Publication No's. 346 847, 356 223, 337 714, 374 098 and 386 611.

Known amino acid derivatives, having activity as renin inhibitors, are disclosed as having HIV protease inhibitory activity in European Patent Publication No's 357 332 and 374 094.

Other substances having HIV protease inhibitory activity, such as pepstatin A, are disclosed in Japanese Patents Laid-Open; Hei-2-42048, Hei-2-117615, Hei-2-145515, Hei-2-152949, Hei-2-202898, Hei-2-202899, and Hei-2-209854; Proceedings of the National Academy of the United States of America 85, 6612 (1988); Biochemical and Biophysical Research Communications 159, 420 (1988); Biochemistry 29, 264 (1990); Proceedings of the National Academy of the United States of America 86 9752 (1989); Nature 343, 90 (1990); Science 246, 1149 (1989); Science 247, 454 (1990); and Science 249, 527 (1990).

We have now discovered a series of new β-amino-α-hydroxycarboxylic acid derivatives having a marked ability to inhibit the activity of HIV protease, have a good enzyme specificity and which have reduced toxicity.

The compounds of the invention are β-amino-α-hydroxycarboxylic acid derivatives, which may be represented by the general formula (I):

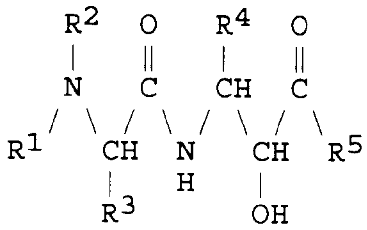

(I)

wherein:

$R^1$ represents a group of formula

$$R(Z)_xA-$$

wherein R represents

a hydrogen atom,

an alkyl group having from one to six carbon atoms,

an alkyl group having from one to six carbon atoms and being substituted by at least one of substituents (a) below,

an alkenyl group having from two to seven carbon atoms,

an alkenyl group having from two to seven carbon atoms and being substituted by at least one of substituents (a) below,

an alkynyl group having from two to seven carbon atoms,

an alkynyl group having from two to seven carbon atoms and being substituted by at least one of substituents (a) below,

a cycloalkyl group having from three to ten carbon atoms,

a cycloalkyl group having from three to ten carbon atoms and being substituted by at least one of substituents (b) below,

a carbocyclic aryl group having from six to fourteen carbon atoms,

a carbocyclic aryl group having from six to fourteen carbon atoms and being substituted by at least one of substituents (b) below, or

a heterocyclic group having from 3 to 10 ring atoms of which from 1 to 4 are nitrogen and/or oxygen and/or sulphur heteroatoms, said heterocyclic group being unsubstituted or substituted by at least one of substituents (b) below,

or R represents a group of formula $-NR^aR^b$

wherein $R^a$ and $R^b$ are independently selected from hydrogen atoms,

alkyl groups having from 1 to 4 carbon atoms, cycloalkyl groups having from three to ten carbon atoms,

cycloalkyl groups having from three to ten carbon atoms and being substituted by at least one of substituents (b) below,

heterocyclic groups having from 3 to 10 ring atoms of which from 1 to 4 are nitrogen and/or oxygen and/or sulphur heteroatoms, said heterocyclic group being unsubstituted or substituted by at least one of substituents (b) below,

carbocyclic aryl groups having from 6 to 14 carbon atoms,

carbocyclic aryl groups having from 6 to 14 carbon atoms and being substituted by at least one of substituents (b) below,

aralkenyl groups in which the aryl group has from 6 to 14 carbon atoms and the alkenyl group has from 2 to 7 carbon atoms, and

aralkyl groups in which the aryl group has from 6 to 14 carbon atoms and the alkyl group has from 1 to 3 carbon atoms;

Z represents oxygen or sulphur;

A represents a group of formula -CO-, -COCO-, -SO-, $-SO_2-$ or -CS-; and

x is the cipher 0 or the integer 1;

$R^2$ represents a hydrogen atom, an alkyl group having from one to six carbon atoms or an alkyl group having from one to six carbon atoms and being substituted with at least one of substituents (a) below;

$R^3$ represents

a hydrogen atom,

an alkyl group having from one to six carbon atoms,

an alkyl group having from one to six carbon atoms and being substituted by at least one of substituents (c) below,

a cycloalkyl group having from three to ten carbon atoms,

a cycloalkyl group having from three to ten carbon atoms and being substituted by at least one of substituents (b) below,

an alkenyl group having from two to seven carbon atoms,

an alkenyl group having from two to seven carbon atoms and being substituted by at least one of substituents (a) below,

an alkynyl group having from two to seven carbon atoms,

an alkynyl group having from two to seven carbon atoms and being substituted by at least one of substituents (a) below,

a carbocyclic aryl group having from six to fourteen carbon atoms,

a carbocyclic aryl group having from six to fourteen carbon atoms and being substituted by at least one of substituents (b) below, or

a heterocyclic group having from 3 to 10 ring atoms of which from 1 to 4 are nitrogen and/or oxygen and/or sulphur heteroatoms, said heterocyclic group being unsubstituted or substituted by at least one of substituents (b) below;

$R^4$ represents a hydrogen atom,

an alkyl group having from one to six carbon atoms,

an alkyl group having from one to six carbon atoms and being substituted by at least one of substituents (a) below,

a cycloalkyl group having from three to ten carbon atoms,

a cycloalkyl group having from three to ten carbon atoms and being substituted by at least one of substituents (b) below,

a carbocyclic aryl group having from six to fourteen carbon atoms,

a carbocyclic aryl group having from six to fourteen carbon atoms and being substituted by at least one of substituents (b) below, or

a heterocyclic group having from 3 to 10 ring atoms of which from 1 to 4 are nitrogen and/or oxygen and/or sulphur heteroatoms, said heterocyclic group being unsubstituted or substituted by at least one of substituents (b) below;

and

$R^5$ represents a group of formula $-B-(CO)-Y-R^c_y$,

wherein

B represents a heterocyclic group having from 3 to 10 ring atoms of which from 1 to 4 are nitrogen and/or oxygen and/or sulphur heteroatoms, at least one being a nitrogen heteroatom, said heterocyclic group being unsubstituted or substituted with at least one alkyl group having from one to six carbon atoms;

Y represents an oxygen atom, a nitrogen atom or a sulphur atom;

y is the integer 1 when Y represents an oxygen atom or a sulphur atom, and 2 when Y represents a nitrogen atom; and

$R^c$ represents

a hydrogen atom,

an alkyl group having from one to six carbon atoms,

an alkyl group having from one to six carbon atoms and being substituted by at least one of substituents (a) below,

a cycloalkyl group having from three to ten carbon atoms,

a cycloalkyl group having from three to ten carbon atoms and being substituted by at least one of substituents (b) below,

an alkenyl group having from two to seven carbon atoms,

an alkenyl group having from two to seven carbon atoms and being substituted by at least one of substituents (a) below,

an alkynyl group having from two to seven carbon atoms,

an alkynyl group having from two to seven carbon atoms and being substituted by at least one of substituents (a) below,

a carbocyclic aryl group having from six to fourteen carbon atoms,

a carbocyclic aryl group having from six to fourteen carbon atoms and being substituted by at least one of substituents (b) below, or

a heterocyclic group having from 3 to 10 ring atoms of which from 1 to 4 are nitrogen and/or oxygen and/or sulphur heteroatoms, said heterocyclic group being unsubstituted or substituted by at least one of substituents (b) below;

and, where there are two groups or atoms represented by $R^c$, they are the same or different; and

substituents (a):

hydroxy groups, $C_3$-$C_{10}$ cycloalkyl groups, $C_1$-$C_6$ alkoxy groups, $C_1$-$C_6$ aliphatic acyloxy groups, $C_1$-$C_6$ aliphatic acyl groups, carboxy groups, $C_2$-$C_6$ alkoxycarbonyl groups, sulpho groups, halogen atoms, amino groups, $C_2$-$C_4$ aliphatic acylamino groups, alkylamino groups in which the alkyl part is $C_1$-$C_6$ alkyl, dialkylamino groups in which each alkyl part is $C_1$-$C_3$ alkyl, carbocyclic aryl groups having from six to fourteen carbon atoms, carbocyclic aryl groups having from six to fourteen carbon atoms and being substituted by at least one of substituents (b) below, carbocyclic aryloxy groups having from six to fourteen carbon atoms, carbocyclic aryloxy groups having from six to fourteen carbon atoms and being substituted by at least one of substituents (b) below, and heterocyclic groups having from 3 to 10 ring atoms of which from 1 to 4 are nitrogen and/or oxygen and/or sulphur heteroatoms, said heterocyclic group being unsubstituted or substituted by at least one of substituents (b) below;

substituents (b):

$C_1$-$C_6$ alkyl groups having from 0 to 3 of substituents (a), $C_1$-$C_6$ haloalkyl groups, $C_1$-$C_6$ aliphatic acyl groups having from 0 to 3 of substituents (a), $C_1$-$C_6$ alkylenedioxy groups, aralkyl groups wherein the alkyl part is $C_1$-$C_6$ alkyl and the aryl part is $C_6$-$C_{14}$ carbocyclic aryl which has from 0 to 3 of substituents (b), aralkyloxycarbonyl groups wherein the alkyl part is $C_1$-$C_6$ alkyl and the aryl part is $C_6$-$C_{14}$ carbocyclic aryl which has from 0 to 3 of substituents (b), hydroxy groups, $C_1$-$C_6$ alkoxy groups, $C_6$-$C_{14}$ carbocyclic aryl groups having from 0 to 3 of substituents (b), aralkyloxy groups where the alkyl part is $C_1$-$C_6$ alkyl and the aryl part is $C_6$-$C_{14}$ carbocyclic aryl which has from 0 to 3 of substituents (b), $C_6$-$C_{14}$ carbocyclic aryloxy groups having from 0-3 of substituents (b), heterocyclic groups having from 3 to 10 ring atoms of which 1 to 4 are nitrogen and/or oxygen and/or sulphur, said heterocyclic group being unsubstituted or substituted by at least one of substituents (b), halogen atoms, nitro groups, cyano groups, carboxy groups, alkoxycarbonyl groups having a total of from 2 to 7 carbon atoms, amino groups, $C_1$-$C_6$ alkylamino groups, dialkylamino groups wherein each alkyl part is $C_1$-$C_6$ alkyl, aliphatic or carbocyclic aromatic carboxylic acylamino groups, carbamoyl groups, alkylcarbamoyl groups where the alkyl part is $C_1$-$C_6$ alkyl, dialkylcarbamoyl groups where each alkyl part is $C_1$-$C_6$ alkyl, mercapto groups, $C_1$-$C_6$ alkylthio groups, $C_6$-$C_{14}$ carbocyclic arylthio groups, $C_1$-$C_6$ alkylsulphonyl groups, $C_6$-$C_{14}$ carbocyclic arylsulphonyl groups wherein the aryl part has from 0 to 3 $C_1$-$C_6$ alkyl substituents, aminosulphonyl groups, $C_1$-$C_6$ alkylsulphinyl groups and $C_6$-$C_{14}$ carbocyclic arylsulphinyl groups wherein the aryl part has from 0 to 3 $C_1$-$C_6$ alkyl substituents;

substituents (c):

hydroxy groups, $C_1$-$C_6$ alkoxy groups, $C_6$-$C_{14}$ carbocyclic aryl groups having from 0 to 3 of substituents (b), aralkyloxy groups where the alkyl part is $C_1$-$C_6$ alkyl and the carbocyclic aryl part is $C_6$-$C_{14}$ carbocyclic aryl which has from 0 to 3 of substituents (b), $C_6$-$C_{14}$ carbocyclic aryloxy groups having from 0-3 of substituents (b), heterocyclic groups having from 3 to 10 ring atoms of which 1 to 4 are nitrogen and/or oxygen and/or sulphur, said heterocyclic group being unsubstituted or substituted by at least one of substituents (b), halogen atoms, nitro groups, cyano groups, carboxy groups, alkoxycarbonyl groups having a total of from 2 to 7 carbon atoms, amino groups, $C_1$-$C_6$ alkylamino groups, dialkylamino groups wherein each alkyl part is $C_1$-$C_6$ alkyl, aliphatic or carbocyclic aromatic carboxylic acylamino groups, carbamoyl groups, alkylcarbamoyl groups where the alkyl part is $C_1$-$C_6$ alkyl, dialkylcarbamoyl groups where each alkyl part is $C_1$-$C_6$ alkyl, mercapto groups, $C_1$-$C_6$ alkylthio groups, $C_6$-$C_{14}$ carbocyclic arylthio groups, $C_1$-$C_6$ alkylsulphonyl groups, $C_6$-$C_{14}$ carbocyclic arylsulphonyl groups wherein the aryl part has from 0 to 3 $C_1$-$C_6$ alkyl substituents, aminosulphonyl groups, $C_1$-$C_6$ alkylsulphinyl groups and $C_6$-$C_{14}$ carbocyclic arylsulphinyl groups wherein the aryl part has from 0 to 3 $C_1$-$C_6$ alkyl substituents;

provided that, where a substituent (a) is substituted by a substituent (b), then substituent (b) is not further substituted by a substituent (a); that where a substituent (b) is substituted by a substituent (a), then substituent (a) is not further substituted by a substituent (b); that, where substituent (b) is a group which is itself substituted by a further substituent (b), that further substituent (b) is not itself substituted; and that where a substituent (c) is substituted by a substituent (b), that further substituent (b) is not itself substituted.

and pharmaceutically acceptable salts and esters thereof.

The invention also provides the use of a compound of formula (I), or a pharmaceutically acceptable salt or ester thereof, for the treatment of acquired immunodeficiency syndrome in a mammal, which may be human

or non-human.

The invention still further provides a pharmaceutical composition for the treatment of acquired immunodeficiency syndrome in an animal, especially a mammal, which may be human or non-human, which comprises a therapeutically effective amount of a compound of formula (I), or a pharmaceutically acceptable salt or ester thereof, in admixture with a pharmaceutically acceptable carrier, diluent or excipient.

The invention still further provides the use of a compound of formula (I), or a pharmaceutically acceptable salt or ester thereof, in the manufacture of a medicament for the treatment of acquired immunodeficiency syndrome.

The term "non-toxic" is used herein to indicate a compound of the invention which, when used in therapy, serves, on balance, to enhance the quality of a patient's life.

The compounds of the invention may be prepared by reacting together two suitable precursor compounds, one having a terminal carboxy group or a reactive derivative thereof and the other having a terminal amino group or a reactive derivative thereof, under conditions conventional for peptide synthesis. Preferred methods of preparing the compounds are described in greater detail hereafter.

The compounds of the invention are useful in that they have the ability to inhibit protease derived from human immunodeficiency virus.

In the compounds of the present invention, where any one of R, $R^a$, $R^b$, $R^2$, $R^3$, $R^4$, $R^c$ or substituents (b) represents an alkyl group, this may be either a straight chain or a branched alkyl group having, unless otherwise specified, from one to six, preferably from one to five, carbon atoms, and examples include the methyl, ethyl, propyl, isopropyl, butyl, isobutyl, sec-butyl, t-butyl, pentyl, isopentyl, neopentyl, 2-methylbutyl, 1-ethylpropyl, 4-methylpentyl, 3-methylpentyl, 2-methylpentyl, 1-methylpentyl, 3,3-dimethylbutyl, 2,2-dimethylbutyl, 1,1-dimethylbutyl, 1,2-dimethylbutyl, 1,3-dimethylbutyl, 2,3-dimethylbutyl, 2-ethylbutyl, hexyl and isohexyl groups. Of these, we prefer those alkyl groups having from 1 to 4 carbon atoms (to which the alkyl groups for $R^a$ and $R^b$ are limited), preferably the methyl, ethyl, propyl, isopropyl, butyl, t-butyl and isobutyl groups and, most preferably, for the group represented by R, $R^a$, $R^b$, $R^2$, $R^3$, $R^4$ or substituents (b), the methyl group and for the group represented by $R^c$, a t-butyl group.

Such alkyl groups included in the definitions of R, $R^2$, $R^3$, $R^4$, $R^c$ or substituents (b) may be substituted by one or more substituents selected, in the case of R, $R^2$, $R^4$, $R^c$ and substituents (b), from substituents (a), or, in the case of $R^3$, from substituents (c), both of which are as defined above. Examples of the groups and atoms defined under substituents (a), substituents (b) and substituents (c) include:

hydroxy groups, carboxy groups, sulpho groups, amino groups, nitro groups, cyano groups, carbamoyl groups, mercapto groups, aminosulphonyl groups;

halogen atoms, such as the fluorine, chlorine, bromine and iodine atoms, preferably the fluorine, chlorine or bromine atoms, and most preferably the fluorine or chlorine atoms;

alkoxy groups, which may be straight or branched chain groups, having from 1 to 6 carbon atoms, such as the methoxy, ethoxy, propoxy, isopropoxy, butoxy, isobutoxy, sec-butoxy, t-butoxy, pentyloxy, isopentyloxy, 2-methylbutoxy, neopentyloxy, hexyloxy, 4-methylpentyloxy, 3-methylpentyloxy, 2-methylpentyloxy, 3,3-dimethylbutoxy, 2,2-dimethylbutoxy, 1,1-dimethylbutoxy, 1,2-dimethylbutoxy, 1,3-dimethylbutoxy or 2,3-dimethylbutoxy groups, of which we prefer those straight or branched chain alkoxy groups having from 1 to 4 carbon atoms and of which we most prefer, the methoxy or ethoxy group;

haloalkyl groups in which the alkyl part has from 1 to 6, preferably 1 to 4, more preferably 1 to 2, carbon atoms and is either straight chain or branched, for example the trifluoromethyl, trichloromethyl, difluoromethyl, dichloromethyl, dibromomethyl, fluoromethyl, chloromethyl, bromomethyl, iodomethyl, 2,2,2-trichloroethyl, 2,2,2-trifluoroethyl, 2-bromoethyl, 2-chloroethyl, 2-fluoroethyl or 2,2-dibromoethyl groups;

aliphatic carboxylic acyl groups having, in total, from 1 to 6 carbon atoms and either a saturated or unsaturated (referring to carbon-carbon bonds) group; it is preferably an alkylcarbonyl group, in which the alkyl part has from 1 to 6 carbon atoms, more preferably from 1 to 4 carbon atoms; examples of such groups include the acetyl, propionyl, acryloyl, methacryloyl, propioloyl, crotonoyl, butyryl, isobutyryl, valeryl, isovaleryl, 2-methylbutyryl and pivaloyl groups, of which we prefer the acetyl group;

aliphatic carboxylic acyloxy groups having from 1 to 6, preferably from 1 to 4, carbon atoms, such as the formyloxy, acetoxy, propionyloxy, butyryloxy, isobutyryloxy, valeryloxy, isovaleryloxy, pivaloyloxy, hexanoyloxy, 2-methylpentanoyloxy and 2-ethylbutyryloxy groups, and preferably such groups having 2 or 3 carbon atoms;

alkoxycarbonyl groups having a total of from 2 to 7 carbon atoms (i.e. the alkoxy part has from 1 to 6 carbon atoms), and preferably having from 2 to 5 carbon atoms, such as the methoxycarbonyl, ethoxycarbonyl, propoxycarbonyl, isopropoxycarbonyl, butoxycarbonyl, isobutoxycarbonyl, sec-butoxycarbonyl, t-butoxycarbonyl, pentyloxycarbonyl and hexyloxycarbonyl groups;

aliphatic carboxylic acylamino groups having from 2 to 4 carbon atoms; this may be a straight or branched

chain group and is preferably an alkanoylamino group; examples include the acetamido, propionamido, butyramido, isobutyramido, acrylamido, methacrylamido, propioloylamino and crotonoylamino groups, and we prefer the acetamido and propionamido groups;

carbocyclic aromatic carboxylic acylamino groups having from 6 to 14 carbon atoms, such as the benzamido and naphthamido groups, which may be substituted or unsubstituted, and, if substituted may be substituted by one or more of substituents (b), defined above and exemplified below, but are preferably unsubstituted;

alkylamino groups and dialkylamino groups in which the or each alkyl part has from 1 to 6, preferably from 1 to 4, carbon atoms, as specified, such as the methylamino,ethylamino, propylamino, isopropylamino, dimethylamino, diethylamino, N-butyl-N-methylamino, N-t-butyl-N-methylamino, N-methyl-N-Propylamino, N-ethyl-N-Propylamino, dipropylamino, diisopropylamino, butylamino, isobutylamino, dibutylamino and diisobutylamino groups;

carbocyclic aryloxy groups where the aryl part of this may be as defined and exemplified below in relation to the aryl groups which may be represented by R, $R^3$, $R^4$, $R^c$, substituents (a), substituents (b) and substituents (c), and examples of the unsubstituted groups include the phenoxy, indenyloxy, naphthyloxy (1- or 2-naphthyloxy), phenanthrenyloxy and anthryloxy groups, of which we prefer the phenoxy and naphthyloxy groups, and most prefer the phenoxy groups; examples of substituted groups include the aryloxy equivalents of the substituted aryl groups exemplified above, and examples of substituent groups are given below;

alkylenedioxy groups, in which the alkylene part has from 1 to 4 carbon atoms, preferably 1 to 2, such as methylenedioxy, ethylenedioxy, propylenedioxy, trimethylenedioxy, tetramethylenedioxy, 1-methyltrimethylenedioxy, and ethylmethylenedioxy, of which we prefer methylenedioxy and ethylenedioxy;

aralkyl groups in which the alkyl part of this group has from 1 to 6 carbon atoms, and the aryl part may be as defined and exemplified below in relation to the aryl groups which may be represented by R, $R^3$, $R^4$, $R^c$, substituents (a), substituents (b) or substituents (c), below; examples include the benzyl, α-naphthylmethyl, β-naphthylmethyl, 2-methylbenzyl, 3-methylbenzyl, 4-methylbenzyl, 2,4,6-trimethylbenzyl, 3,4,5-trimethylbenzyl, 2-methoxybenzyl, 3-methoxybenzyl, 4-methoxybenzyl, 3,4-dimethoxybenzyl, 2-nitrobenzyl, 4-nitrobenzyl, 2-chlorobenzyl, 3-chlorobenzyl, 4-chlorobenzyl, 4-bromobenzyl, 4-cyanobenzyl, naphthylmethyl, diphenylmethyl, triphenylmethyl, 2-phenethyl, 1-naphthylethyl, 2-naphthylethyl, 1-phenylpropyl, 2-phenylpropyl, 3-phenylpropyl, 1-naphthylpropyl, 2-naphthylpropyl, 3-naphthylpropyl, 1-phenylbutyl, 2-phenylbutyl, 3-phenylbutyl, 4-phenylbutyl, 1-naphthylbutyl, 2-naphthylbutyl, 3-naphthylbutyl, 4-naphthylbutyl, 1-phenylpentyl, 2-phenylpentyl, 3-phenylpentyl, 4-phenylpentyl, 5-phenylpentyl, 1-naphthylpentyl, 2-naphthylpentyl, 3-naphthylpentyl, 4-naphthylpentyl, 5-naphthylpentyl, 1-phenylhexyl, 2-phenylhexyl, 3-phenylhexyl, 4-phenylhexyl, 5-phenylhexyl, 6-phenylhexyl, 1-naphthylhexyl, 2-naphthylhexyl, 3-naphthylhexyl, 4-naphthylhexyl, 5-naphthylhexyl or 6-naphthylhexyl groups; preferably the benzyl, α-naphthylmethyl, β-naphthylmethyl, 2-methylbenzyl, 3-methylbenzyl, 4-methylbenzyl, 2,4,6-trimethylbenzyl, 3,4,5-trimethylbenzyl, 2-methoxybenzyl, 3-methoxybenzyl, 4-methoxybenzyl, 3,4-dimethoxybenzyl, 2-nitrobenzyl, 4-nitrobenzyl, 2-chlorobenzyl, 3-chlorobenzyl, 4-chlorobenzyl, 4-bromobenzyl, 4-cyanobenzyl, naphthylmethyl, diphenylmethyl, triphenylmethyl, 2-phenethyl, 1-naphthylethyl or 2-naphthylethyl groups, most preferably the benzyl, 2-methylbenzyl and 4-methylbenzyl groups; and further substituents are exemplified above in relation to the aryl groups which may be represented by R, $R^3$, $R^4$, $R^c$, substituents (a), substituents (b) or substituents (c);

aralkyloxy groups in which the aralkyl portion has from one to six carbon atoms and may be as defined and exemplified above in relation to the aralkyl groups, for example benzyloxy, α-naphthylmethyloxy, β-naphthylmethyloxy, 2-methylbenzyloxy, 3-methylbenzyloxy, 4-methylbenzyloxy, 2,4,6-trimethylbenzyloxy, 3,4,5-trimethylbenzyloxy, 2-methoxybenzyloxy, 3-methoxybenzyloxy, 4-methoxybenzyloxy, 3,4-dimethoxybenzyloxy, 2-nitrobenzyloxy, 4-nitrobenzyloxy, 2-chlorobenzyloxy, 3-chlorobenzyloxy, 4-chlorobenzyloxy, 4-bromobenzyloxy, 4-cyanobenzyloxy, 2-anthrylmethyloxy, diphenylmethyloxy, triphenylmethyloxy, 2-phenethyloxy, 1-naphthylethyloxy, 2-naphthylethyloxy, 1-phenylpropyloxy, 2-phenylpropyloxy, 3-phenylpropyloxy, 1-naphthylpropyloxy, 2-naphthylpropyloxy,3-naphthylpropyloxy, 1-phenylbutyloxy, 2-phenylbutyloxy, 3-phenylbutyloxy, 4-phenylbutyloxy, 1-naphthylbutyloxy, 2-naphthylbutyloxy, 3-naphthylbutyloxy, 4-naphthylbutyloxy, 1-phenylpentyloxy, 2-phenylpentyloxy, 3-phenylpentyloxy, 4-phenylpentyloxy, 5-phenylpentyloxy, 1-naphthylpentyloxy, 2-naphthylpentyloxy, 3-naphthylpentyloxy, 4-naphthylpentyloxy, 5-naphthylpentyloxy, 1-phenylhexyloxy, 2-phenylhexyloxy, 3-phenylhexyloxy, 4-phenylhexyloxy, 5-phenylhexyloxy, 6-phenylhexyloxy, 1-naphthylhexyloxy, 2-naphthylhexyloxy, 3-naphthylhexyloxy, 4-naphthylhexyloxy, 5-naphthylhexyloxy or 6-naphthylhexyloxy groups; preferably a benzyloxy, α-naphthylmethyloxy, β-naphthylmethyloxy, 2-methylbenzyloxy, 3-methylbenzyloxy, 4-methylbenzyloxy, 2,4,6-trimethylbenzyloxy, 3,4,5-trimethylbenzyloxy, 2-methoxybenzyloxy, 3-methoxybenzyloxy, 4-methoxybenzyloxy, 3,4-dimethoxybenzyloxy, 2-nitrobenzyloxy, 4-nitrobenzyloxy, 2-chlorobenzyloxy, 3-chlorobenzyloxy, 4-chlorobenzyloxy, 4-bromobenzyloxy, 4-cyanobenzyloxy, naphthylmethyloxy, diphenylmethyloxy, triphenylmethyloxy, 2-phenethyloxy, 1-naphthylethyloxy, 2-naphthylethyloxy; and

more preferably an unsubstituted benzyloxy or 2-phenethyloxy group;

aralkyloxycarbonyl groups in which the aralkyloxy part of the group may be as defined and exemplifed as above in relation to the aralkyloxy groups;

alkylcarbamoyl groups and dialkylcarbamoyl groups in which the or each alkyl part is independently selected from alkyl groups having from 1 to 6, preferably from 1 to 4, carbon atoms, such as the methylcarbamoyl, ethylcarbamoyl, propylcarbamoyl, isopropylcarbamoyl, dimethylcarbamoyl, diethylcarbamoyl, N-butyl-N-methylcarbamoyl, N-t-butyl-N-methylcarbamoyl, N-methyl-N-propylcarbamoyl, N-ethyl-N-propylcarbamoyl, dipropylcarbamoyl, diisopropylcarbamoyl, butylcarbamoyl, isobutylcarbamoyl, dibutylcarbamoyl and diisobutylcarbamoyl groups;

alkylthio groups having from 1 to 6 carbon atoms and being a straight or branched chain group, examples of such groups including the methylthio, ethylthio, propylthio, isopropylthio, butylthio, isobutylthio, sec-butylthio, t-butylthio, pentylthio, isopentylthio, 2-methylbutylthio, neopentylthio, 1-ethylpropylthio, hexylthio, 4-methylpentylthio, 3-methylpentylthio, 2-methylpentylthio, 1-methylpentylthio, 3,3-dimethylbutylthio, 2,2-dimethylbutylthio, 1,1-dimethylbutylthio, 1,2-dimethylbutylthio, 1,3-dimethylbutylthio, 2,3-dimethylbutylthio and 2-ethylbutylthio groups, of which we prefer the methylthio and ethylthio groups;

arylthio groups wherein the aryl part of the group may be as defined and exemplified below for the aryl group represented by R $R^3$ $R^4$ $R^c$ substituents (a), substituents (b) and substituents (c);

alkylsulphonyl groups having from 1 to 6 carbon atoms and being a straight or branched chain group, examples of such groups including the methylsulphonyl, ethylsulphonyl, propylsulphonyl, isopropylsulphonyl, butylsulphonyl, isobutylsulphonyl, sec-butylsulphonyl, t-butylsulphonyl, pentylsulphonyl, isopentylsulphonyl, 2-methylbutylsulphonyl, neopentylsulphonyl, 1-ethylpropylsulphonyl, hexylsulphonyl, 4-methylpentylsulphonyl, 3-methylpentylsulphonyl, 2-methylpentylsulphonyl, 1-methylpentylsulphonyl, 3,3-dimethylbutylsulphonyl, 2,2-dimethylbutylsulphonyl, 1,1-dimethylbutylsulphonyl, 1,2-dimethylbutylsulphonyl, 1,3-dimethylbutylsulphonyl, 2,3-dimethylbutylsulphonyl and 2-ethylbutylsulphonyl groups, of which we prefer the methylsulphonyl and ethylsulphonyl groups;

arylsulphonyl groups wherein the aryl part is as defined below for R, $R^3$, $R^4$, $R^c$, substituents (a), substituents (b) and substituents (c), the aryl portion optionally being further substituted by 0 to 3 $C_1$-$C_6$ alkyl substituents, wherein the alkyl substituents may be as exemplified above,and examples include phenylsulphonyl, 4-methylphenylsulphonyl, 3,4-dimethylphenylsulphonyl, 2,3-dimethylphenylsulphonyl, 3,5-dimethylphenylsulphonyl, 2,4-dimethylphenylsulphonyl, 4-ethylphenylsulphonyl, 4-propylphenylsulphonyl and naphthylsulphonyl groups, and preferably the phenylsulphonyl or naphthylsulphonyl groups;

alkylsulphinyl groups having from 1 to 6 carbon atoms and being a straight or branched chain group, examples of such groups including the methylsulphinyl, ethylsulphinyl, propylsulphinyl, isopropylsulphinyl, butylsulphinyl, isobutylsulphinyl, sec-butylsulphinyl, t-butylsulphinyl, pentylsulphinyl, isopentylsulphinyl, 2-methylbutylsulphinyl, neopentylsulphinyl, 1-ethylpropylsulphinyl, hexylsulphinyl, 4-methylpentylsulphinyl, 3-methylpentylsulphinyl, 2-methylpentylsulphinyl, 1-methylpentylsulphinyl, 3,3-dimethylbutylsulphinyl, 2,2-dimethylbutylsulphinyl, 1,1-dimethylbutylsulphinyl, 1,2-dimethylbutylsulphinyl, 1,3-dimethylbutylsulphinyl, 2,3-dimethylbutylsulphinyl and 2-ethylbutylsulphinyl groups, preferably the methylsulphinyl and ethylsulphinyl groups

arylsulphinyl groups wherein the aryl part is as defined below for R, $R^3$, $R^4$, $R^c$, substituents (a), substituents (b) and substituents (c), the aryl portion optionally being further substituted by 0 to 3 $C_1$-$C_6$ alkyl substituents, wherein the alkyl substituents may be as exemplified above, and examples include phenylsulphinyl, 4-methylphenylsulphinyl, 3,4-dimethylphenylsulphinyl, 2,3-dimethylphenylsulphinyl, 3,5-dimethylphenylsulphinyl, 2,4-dimethylphenylsulphinyl, 4-ethylphenylsulphinyl, 4-propylphenylsulphinyl and naphthylsulphinyl groups, and preferably the phenylsulphinyl and naphthylsulphinyl groups.

In the compounds of the present invention, $R^3$ is preferably an alkyl group having from one to six carbon atoms or an alkyl group having from one to six carbon atoms and being substituted by at least one substituent selected from: cyano groups, hydroxy groups, carboxy groups, carbamoyl groups, $C_1$-$C_6$, mono- or dialkylcarbamoyl groups, $C_1$-$C_6$ alkylthio groups, $C_1$-$C_6$ alkylsulphonyl groups and aminosulphonyl groups, for example the carbamoylmethyl, 2-carbamoylethyl, dimethylcarbamoylmethyl, hydroxymethyl, 2-hydroxyethyl, cyanomethyl, 2-cyanoethyl, carboxymethyl, 2-carboxyethyl, methylthiomethyl, 2-methylthioethyl, methanesulphonylmethyl, 2-methanesulphonylethyl, sulphamoylmethyl and 2-sulphamoylethyl groups, preferably the carbamoylmethyl, 2-carbamoylethyl, dimethylcarbamoylmethyl, hydroxymethyl, cyanomethyl, carboxymethyl, methylthiomethyl, methanesulphonylmethyl and sulphamoylmethyl groups.

The cycloalkyl groups, which are groups within substituents (a), and the carbocyclic aryl groups and heterocyclic groups, which are all groups within substituents (a), substituents (b) and substituents (c), are all as exemplified hereafter in relation to the groups which may be represented by R, $R^3$, $R^4$ and $R^c$, for example.

In the compounds of the present invention, where any one of R, $R^a$, $R^b$, $R^3$, $R^4$, $R^c$ or substituents (a) represents a cycloalkyl group, this is a saturated cyclic hydrocarbon group with from 3 to 10, preferably 5 to 10, ring carbon atoms which may be a single ring or a bridged ring (including certain cyclic terpenyl groups), or it may be formed from at least two fused rings, for example the cyclopropyl, cyclobutyl, cyclopentyl, cyclohexyl, cycloheptyl, norbornyl and adamantyl groups, preferably the cyclopentyl and cyclohexyl groups.

Such cycloalkyl groups included in the definitions of R, $R^a$, $R^b$, $R^3$, $R^4$ or $R^c$ may be substituted by one or more substituents selected from substituents (b), which are as defined above. Examples of such substituents are given above. Where the group is substituted, the preferred substituents are alkyl groups, especially the methyl group; however, the unsubstituted groups are preferred.

In the compounds of the present invention, where any one of R, $R^3$, $R^4$, $R^a$, $R^b$, $R^c$, substituents (a), substituents (b) or substituents (c) represents a carbocyclic aryl group, this is a single ring, for example phenyl, or a fused ring system with a total of from 6 to 14, preferably 6 to 10, most preferably 6 or 10, ring carbon atoms. The fused ring system is either formed from fusion of one aryl ring with another aryl ring, for example pentalenyl, naphthyl, anthryl, phenanthrenyl, azulenyl, heptalenyl, as-indacenyl, s-indacenyl and acenaphthylenyl, preferably naphthyl, phenanthrenyl or anthryl, or from fusion of at least one aryl ring with at least one cycloalkyl or heterocyclic ring, for example indenyl, indanyl, fluorenyl or phenalenyl, preferably 2-indanyl. However, the phenyl and naphthyl (α- or β- naphthyl) groups are preferred.

Such carbocyclic aryl groups included in the definitions of R, $R^3$, $R^4$, $R^a$, $R^b$, $R^c$, substituents (a), substituents (b) or substituents (c) may be substituted, in the case of R, $R^3$, $R^4$, $R^a$, $R^b$, $R^c$, substituents (a) and substituents (c), by one or more of substituents (b), and in the case of substituents (b), by one or more of substituents (a), all of which are as defined above. Examples of such substituents are given above in relation to the substituents on the alkyl groups. Specific such substituents for the carbocyclic aryl groups include: substituted and unsubstituted aralkyl groups, for example the benzyl, α-naphthylmethyl, β-naphthylmethyl, 2-methylbenzyl, 3-methylbenzyl, 4-methylbenzyl, 2,4,6-trimethylbenzyl, 3,4,5-trimethylbenzyl, 2-methoxybenzyl, 3-methoxybenzyl, 4-methoxybenzyl, 3,4-dimethoxybenzyl, 2-nitrobenzyl, 4-nitrobenzyl, 2-chlorobenzyl, 3-chlorobenzyl, 4-chlorobenzyl, 4-bromobenzyl, 4-cyanobenzyl, naphthylmethyl, diphenylmethyl, triphenylmethyl, 2-phenethyl, 1-naphthylethyl, 2-naphthylethyl, 1-phenylpropyl, 2-phenylpropyl, 3-phenylpropyl, 1-naphthylpropyl, 2-naphthylpropyl, 3-naphthylpropyl, 1-phenylbutyl, 2-phenylbutyl, 3-phenylbutyl, 4-phenylbutyl, 1-naphthylbutyl, 2-naphthylbutyl, 3-naphthylbutyl, 4-naphthylbutyl, 1-phenylpentyl, 2-phenylpentyl, 3-phenylpentyl, 4-phenylpentyl, 5-phenylpentyl, 1-naphthylpentyl, 2-naphthylpentyl, 3-naphthylpentyl, 4-naphthylpentyl, 5-naphthylpentyl, 1-phenylhexyl, 2-phenylhexyl, 3-phenylhexyl, 4-phenylhexyl, 5-phenylhexyl, 6-phenylhexyl, 1-naphthylhexyl, 2-naphthylhexyl, 3-naphthylhexyl, 4-naphthylhexyl, 5-naphthylhexyl and 6-naphthylhexyl groups; alkylcarbamoyl groups, for example the methylcarbamoyl, ethylcarbamoyl, propylcarbamoyl, isopropylcarbamoyl, butylcarbamoyl, isobutylcarbamoyl, sec-butylcarbamoyl, t-butylcarbamoyl, pentylcarbamoyl, isopentylcarbamoyl, 2-methylbutylcarbamoyl, neopentylcarbamoyl, hexylcarbamoyl, 4-methylpentylcarbamoyl, 3-methylpentylcarbamoyl and 2-methylpentylcarbamoyl groups; dialkylcarbamoyl groups, for example the 3,3-dimethylbutylcarbamoyl, 2,2-dimethylbutylcarbamoyl, 1,1-dimethylbutylcarbamoyl, 1,2-dimethylbutylcarbamoyl, 1,3-dimethylbutylcarbamoyl, 2,3-dimethylbutylcarbamoyl, dimethylcarbamoyl, diethylcarbamoyl, dipropylcarbamoyl, diisopropylcarbamoyl, dibutylcarbamoyl, diisobutylcarbamoyl, di-sec-butylcarbamoyl and di-t-butylcarbamoyl groups; halogen atoms; $C_1$-$C_6$ alkyl groups, as hereinbefore exemplifed; $C_1$-$C_6$ alkoxy groups, as hereinbefore exemplified; aralkyloxy groups, as hereinafter exemplified; haloalkyl groups having from 1 to 6, preferably from 1 to 4, and more preferably 1 or 2, carbon atoms and preferably from 1 to 3 halogen atoms, for example the trifluoromethyl, trichloromethyl, difluoromethyl, dichloromethyl, dibromomethyl, fluoromethyl, 2,2,2-trichloroethyl, 2,2,2-trifluoroethyl, 2-bromoethyl, 2-chloroethyl, 2-fluoroethyl and 2,2-dibromoethyl groups; aliphatic acyl groups, as hereinbefore exemplified, for example the formyl, acetyl, propionyl, butyryl, isobutyryl, pivaloyl, valeryl and isovaleryl groups; halogenated alkylcarbonyl groups, for example the chloroacetyl, dichloroacetyl, trichloroacetyl and trifluoroacetyl groups; alkoxyalkylcarbonyl groups, for example methoxyacetyl; alkenylcarbonyl groups, for example the (E)-2-methyl-2-butenoyl, acryloyl, methacryloyl and crotonoyl groups; or $C_1$-$C_4$ alkylenedioxy groups, for example the methylenedioxy, ethylenedioxy, propylenedioxy, ethylidenedioxy and propylidenedioxy groups. Preferred such substituents are $C_1$-$C_6$ alkyl groups, $C_1$-$C_6$ alkoxy gruops or halogen atoms.

Examples of aryl groups which are represented by R, $R^3$, $R^4$, $R^c$, substituents (a), substituents (b) or substituents (c) include: aryl groups such as phenyl or naphthyl; aryl groups substituted by at least one halogen atom, such as 2-fluorophenyl, 3-fluorophenyl, 4-fluorophenyl, 2-chlorophenyl, 3-chlorophenyl, 4-chlorophenyl, 2-bromophenyl, 3-bromophenyl, 4-bromophenyl, 3,5-difluorophenyl, 2,5-difluorophenyl, 2,6-difluorophenyl, 2,4-difluorophenyl, 3,5-dibromophenyl, 2,5-dibromophenyl, 2,6-dichlorophenyl, 2,4-dichlorophenyl, 2,3,6-trifluorophenyl, 2,3,4-trifluorophenyl, 3,4,5-trifluorophenyl, 2,5,6-trifluorophenyl, 2,4,6-trifluorophenyl, 2,3,6-tribromophenyl, 2,3,4-tribromophenyl, 3,4,5-tribromophenyl, 2,5,6-trichlorophenyl, 2,4,6-trichlorophenyl, 1-fluoro-2-naphthyl, 2-fluoro-1-naphthyl, 3-fluoro-1-naphthyl, 1-chloro-2-naphthyl, 2-chloro-1-naphthyl, 3-bromo-1-naphthyl, 3,8-difluoro-1-naphthyl, 2,3-difluoro-1-naphthyl, 4,8-difluoro-1-naphthyl, 5,6-difluoro-1-naphthyl, 3,8-dichloro-1-naphthyl, 2,3-dichloro-1-naphthyl, 4,8-dibromo-1-naphthyl, 5,6-dibromo-1-naphthyl, 2,3,6-trifluoro-1-naphthyl, 2,3,4-trifluoro-1-naphthyl, 3,4,5-trifluoro-1-naphthyl, 4,5,6-trifluoro-1-naphthyl or 2,4,8-tri-fluoro-1-naphthyl; aryl groups substituted by a $C_1$-$C_6$ haloalkyl group, such as 2-trifluoromethylphenyl, 3-trifluoromethylphenyl, 4-trifluoromethylphenyl, 2-trichloromethylphenyl, 3-dichloromethylphenyl, 4-trichloromethylphenyl, 2-tribromomethylphenyl, 3-dibromomethylphenyl, 4-dibromomethylphenyl, 3,5-bis-trifluoromethylphenyl, 2,5-bis-tri-fluoromethylphenyl, 2,6-bis-trifluoromethylphenyl, 2,4-bis-trifluoromethylphenyl, 3,5-bis-tribromomethylphenyl, 2,5-bis-dibromomethylphenyl, 2,6-bis-dichloromethylphenyl, 2,4-bis-dichloromethylphenyl, 2,3,6-tris-trifluoromethylphenyl, 2,3,4-tris-trifluoromethylphenyl, 3,4,5-tris-trifluoromethylphenyl, 2,5,6-tris-trifluoromethylphenyl, 2,4,6-tris-trifluoromethylphenyl, 2,3,6-tris-tribromomethylphenyl, 2,3,4-tris-dibromomethylphenyl, 3,4,5-tris-tribromomethylphenyl, 2,5,6-tris-dichloromethylphenyl, 2,4,6-tris-dichloromethylphenyl, 1-trifluoromethyl-2-naphthyl, 2-trifluoromethyl-1-naphthyl, 3-trifluoromethyl-1-naphthyl, 3,8-bis-trifluoromethyl-1-naphthyl, 2,3-bis-trifluoromethyl-1-naphthyl, 4,8-bis-trifluoromethyl-1-naphthyl, 5,6-bis-trifluoromethyl-1-naphthyl, 3,8-bis-trichloromethyl-1-naphthyl, 2,3-bis-dichloromethyl-1-naphthyl, 4,8-bis-dibromomethyl-1-naphthyl, 5,6-bis-tribromomethyl-1-naphthyl, 2,3,6-tris-trifluoromethyl-1-naphthyl, 2,3,4-tris-trifluoromethyl-1-naphthyl, 3,4, 5-tris-trifluoromethyl-1-naphthyl, 4,5,6-tris-trifluoromethyl-1-naphthyl or 2,4,8-tris-trifluoromethyl-1-naphthyl; aryl groups substituted by at least one $C_1$-$C_6$ alkyl group, such as 2-methylphenyl, 3-methylphenyl, 4-methylphenyl, 2-ethylphenyl, 3-propylphenyl, 4-ethylphenyl, 2-butylphenyl, 3-pentylphenyl, 4-pentylphenyl, 3,5-dimethylphenyl, 2,5-dimethylphenyl, 2,6-dimethylphenyl, 2,4-dimethylphenyl, 3,5-dibutylphenyl, 2,5-dipentylphenyl, 2,6-dipropylmethylphenyl, 2,4-dipropylphenyl, 2,3,6-trimethylphenyl, 2,3,4-trimethylphenyl, 3,4,5-trimethylphenyl, 2,5,6-trimethylphenyl, 2,4,6-trimethylphenyl, 2,3,6-tributylphenyl, 2,3,4-tripentylphenyl, 3,4,5-tributylphenyl, 2,5,6-tripropylmethylphenyl, 2,4,6-tripropylphenyl, 1-methyl-2-naphthyl, 2-methyl-1-naphthyl, 3-methyl-1-naphthyl, 1-ethyl-2-naphthyl, 2-propyl-1-naphthyl, 3-butyl-1-naphthyl, 3,8-dimethyl-1-naphthyl, 2,3-dimethyl-1-naphthyl, 4,8-dimethyl-1-naphthyl, 5,6-dimethyl-1-naphthyl, 3,8-diethyl-1-naphthyl, 2,3-dipropyl-1-naphthyl, 4,8-dipentyl-1-naphthyl, 5,6-dibutyl-1-naphthyl, 2,3,6-trimethyl-1-naphthyl, 2,3,4-trimethyl-1-naphthyl, 3,4,5-trimethyl-1-naphthyl, 4,5,6-trimethyl-1-naphthyl or 2,4,8-trimethyl-1-naphthyl; aryl groups substituted by at least one $C_1$-$C_6$ alkoxy group, such as 2-methoxyphenyl, 3-methoxyphenyl, 4-methoxyphenyl, 2-ethoxyphenyl, 3-propoxyphenyl, 4-ethoxyphenyl, 2-butoxyphenyl, 3-pentyloxyphenyl, 4-pentyloxyphenyl, 3,5-dimethoxyphenyl, 2,5-dimethoxyphenyl, 2,6-dimethoxyphenyl, 2,4-dimethoxyphenyl, 3,5-dibutoxyphenyl, 2,5-dipentyloxyphenyl, 2,6-dipropoxymethoxyphenyl, 2,4-dipropoxyphenyl, 2,3,6-trimethoxyphenyl, 2,3,4-trimethoxyphenyl, 3,4,5-trimethoxyphenyl, 2,5,6-trimethoxyphenyl, 2,4,6-trimethoxyphenyl, 2,3,6-tributoxyphenyl, 2,3,4-tripentyloxyphenyl, 3,4,5-tributoxyphenyl, 2,5,6-tripropoxyphenyl, 2,4,6-tripropoxyphenyl, 1-methoxy-2-naphthyl, 2-methoxy-1-naphthyl, 3-methoxy-1-naphthyl,1-ethoxy-2-naphthyl, 2-propoxy-1-naphthyl, 3-butoxy-1-naphthyl, 3,8-dimethoxy-1-naphthyl, 2,3-dimethoxy-1-naphthyl, 4,8-dimethoxy-1-naphthyl, 5,6-dimethoxy-1-naphthyl, 3,8-diethoxy-1-naphthyl, 2,3-dipropoxy-1-naphthyl, 4,8-dipentyloxy-1-naphthyl, 5,6-dibutoxy-1-naphthyl, 2,3,6-trimethoxy-1-naphthyl, 2,3,4-trimethoxy-1-naphthyl, 3,4,5-trimethoxy-1-naphthyl, 4,5,6-trimethoxy-1-naphthyl or 2,4,8-trimethoxy-1-naphthyl; aryl groups substituted by at least one amino group, such as 2-aminophenyl, 3-aminophenyl, 4-aminophenyl, 3,5-diaminophenyl, 2,5-diaminophenyl, 2,6-diaminophenyl, 2,4-diaminophenyl, 2,3,6-triaminophenyl, 2,3,4-triaminophenyl, 3,4,5-triaminophenyl, 2,5,6-triaminophenyl, 2,4,6-triaminophenyl, 1-amino-2-naphthyl, 2-amino-1-naphthyl, 3-amino-1-naphthyl, 3,8-diamino-1-naphthyl, 2,3-diamino-1-naphthyl, 4,8-diamino-1-naphthyl, 5,6-diamino-1-naphthyl, 2,3,6-triamino-1-naphthyl, 2,3,4-triamino-1-naphthyl, 3,4,5-triamino-1-naphthyl, 4,5,6-triamino-1-naphthyl or 2,4,8-triamino-1-naphthyl; aryl groups substituted by at least one hydroxy group, such as 2-hydroxyphenyl, 3-hydroxyphenyl, 4-hydroxyphenyl, 3,5-dihydroxyphenyl, 2,5-dihydroxyphenyl, 2,6-dihydroxyphenyl, 2,4-dihydroxyphenyl, 2,3,6-trihydroxyphenyl, 2,3,4-trihydroxyphenyl, 3,4,5-trihydroxyphenyl, 2,5,6-trihydroxyphenyl, 2,4,6-trihydroxyphenyl, 1-hydroxy-2-naphthyl, 2-hydroxy-1-naphthyl, 3-hydroxy-1-naphthyl, 3,8-dihydroxy-1-naphthyl, 2,3-dihydroxy-1-naphthyl, 4,8-dihydroxy-1-napthyl, 5,6-dihydroxy-1-naphthyl, 2,3,6-trihydroxy-1-naphthyl, 2,3,4-trihydroxy-1-naphthyl, 3,4,5-trihydroxy-1-naphthyl, 4,5,6-trihydroxy-1-naphthyl or 2,4,8-trihydroxy-1-naphthyl; aryl groups substituted by at least one cyano group, such as 2-cyanophenyl, 3-cyanophenyl, 4-cyanophenyl, 3,5-dicyanophenyl, 2,5-dicyanophenyl, 2,6-dicyanophenyl, 2,4-dicyanophenyl, 2,3,6-tricyanophenyl, 2,3,4-tricyanophenyl, 3,4,5-tricyanophenyl, 2,5,6-tricyanophenyl, 2,4,6-tricyanophenyl, 1-cyano-2-naphthyl, 2-cyano-1-naphthyl, 3-cyano-1-naphthyl, 3,8-dicyano-1-naphthyl, 2,3-dicyano-1-naphthyl, 4,8-dicyano-1-naphthyl, 5,6-dicyano-1-naphthyl, 2,3,6-tricyano-1-naphthyl, 2,3,4-tricyano-1-naphthyl, 3,4,5-tricyano-1-naphthyl, 4,5,6-tricyano-1-naphthyl or 2,4,8-tricyano-1-naphthyl; aryl groups substituted by at least one aliphatic acyl group, such as 2-acetylphenyl, 3-acetylphenyl, 4-acetylphenyl, 3,5-diacetylphenyl, 2,5-diacetylphenyl, 2,6-diacetylphenyl, 2,4-diacetylphenyl, 2,3,6-tripropionylphenyl, 2,3,4-tripropionylphenyl, 3,4,5-tripropionylphenyl, 2,5,6-tributyrylphenyl, 2,4,6-tributyrylphenyl, 1-acetyl-2-naphthyl, 2-acetyl-1-naphthyl, 3-acetyl-1-naphthyl, 3,8-diacetyl-1-naphthyl, 2,3-dipropionyl-1-naphthyl, 4,8-dibutyryl-1-naphthyl, 5,6-dibutyryl-1-naphthyl, 2,3,6-triacetyl-1-naphthyl, 2,3,4-triacetyl-1-naphthyl, 3,4,5-tripropionyl-1-naphthyl, 4,5,6-tributyryl-1-naphthyl or 2,4,8-tributyryl-1-naphthyl; aryl groups substituted by at least one carboxyl group, such as 2-carboxyphenyl, 3-carboxyphenyl, 4-carboxyphenyl, 3,5-dicarboxyphenyl, 2,5-dicarboxyphenyl, 2,6-dicarboxyphenyl or 2,4-dicarboxyphenyl; aryl groups substituted by at least one carbamoyl group, such as 2-carbamoylphenyl, 3-carbamoylphenyl, 4-carbamoylphenyl, 3,5-dicarbamoylphenyl, 2,5-di-carbamoylphenyl, 2,6-dicarbamoylphenyl or 2,4-dicarbamoylphenyl; or aryl groups substituted by at least one an alkylenedioxy group, such as 3,4-methylenedioxyphenyl.

Where, in the compounds of the present invention, B represents a heterocyclic group, this heterocyclic group has from 3 to 10 ring atoms, of which from 1 to 4 are nitrogen and/or oxygen and/or sulphur heteroatoms, and at least one is a nitrogen heteroatom. The group may be unsubstituted or it may be substituted by at least one $C_1$-$C_6$ alkyl group, for example as hereinbefore exemplified. Where there are 4 heteroatoms, we prefer that all 4 should be nitrogen atoms. Where there are 3 heteroatoms, we prefer that at least one (more preferably 2) should be a nitrogen atom and one or two should be nitrogen, oxygen or sulphur atoms (and, where there are two, they may be the same or different). Where there are two heteroatoms, these may be the same or different and they are selected from nitrogen, oxygen and sulphur atoms; however, more preferably one is a nitrogen atom or an oxygen atom and the other is a nitrogen, oxygen or sulphur atom. Where there is one heteroatom, it may be any of the nitrogen, oxygen and sulphur atoms. Examples of such groups include the azetidinyl, morpholinyl, thiomorpholinyl, pyrrolidinyl, pyrrolinyl, imidazolidinyl, imidazolinyl, pyrazolidinyl, pyrazolinyl, thiazolidinyl, oxazolidinyl, piperidyl, piperazinyl, quinolyl isoquinolyl, 1H,2H,3H,4H-tetrahydroquinolyl, decahydroquinolyl, 1H,2H,3H,4H-tetrahydroisoquinolyl, decahydroisoquinolyl, 4-methylazetidinyl, 4-methylmorpholinyl, 4-methylthiomorpholinyl, 2-methylpyrrolidinyl, 2-methylpyrrolinyl, 4-methylimidazolidinyl, 4-methylimidazolinyl, 4-methylpyrazolidinyl, 4-methylpyrazolinyl, 4-methylthiazolidinyl, 4-methyloxazolidinyl, 4-methylpiperidyl, 4-methylpiperazinyl and 5,5-dimethylthiazolidinyl groups. We prefer that this heterocyclic group has from 4 to 6 ring atoms, and that this group is preferably a pyrrolidinyl, thiazolidinyl or oxazolidinyl group.

In the compounds of the present invention, where any one of R, $R^3$, $R^4$, $R^a$, $R^b$, $R^c$, substituents (a), substituents (b) or substituents (c) represents a heterocyclic group, this is a saturated or unsaturated heterocyclic group having from 3 to 10 ring atoms of which from 1 to 4 are nitrogen and/or oxygen and/or sulphur heteroatoms. More preferably the group has from 0 to 3 such nitrogen atoms, 0, 1 or 2 such oxygen atoms and 0, 1 or 2 such sulphur atoms, provided that the total number of heteroatoms is not less than 1 and does not exceed 4. Where the group is unsaturated, it may be non-aromatic or aromatic in character. The group may be monocyclic or it may be fused to one or two aryl, heterocyclic or cycloalkyl rings, to produce a bicyclic or tricyclic group, in which the heterocyclic part may be aromatic or non-aromatic in character. Examples of such groups include the furyl, thienyl, pyranyl, pyrrolyl, azepinyl, imidazolyl, oxazolyl, pyrazolyl, thiazolyl, isothiazolyl, 1,2,3-oxadiazolyl, triazolyl, tetrazolyl, isoxazolyl, pyridyl, pyrazinyl, pyrimidinyl, pyridazinyl, pyrrolidinyl, imidazolidinyl, pyrazolidinyl, pyrazolinyl, piperidyl, piperazinyl or morpholinyl; preferably pyrrolyl, azepinyl, pyrazolyl, imidazolyl, oxazolyl, isoxazolyl, thiazolyl, isothiazolyl, 1,2,3-oxadiazolyl, triazolyl, tetrazolyl, thiadiazolyl, pyridyl, pyridazinyl, pyrimidinyl, pyrazinyl, benzofuranyl, benzimidazolyl, chromenyl, xanthenyl, phenoxathienyl, indolizinyl, indolyl, indazolyl, isoindolyl, quinolidinyl, naphthylidinyl, quinolyl, isoquinolyl, phthalazinyl, quinoxalinyl, quinazolinyl, cinnolinyl, chromanyl, 1,2,3,4-tetrahydroisoquinolyl, decahydroisoquinolyl, 1,2,3,4-tetrahydroquinolyl, decahydroquinolyl, isoindolinyl, indolinyl, purinyl or naphthyridinyl groups. More preferred are pyridyl, benzofuranyl, benzimidazolyl, indolyl, quinolyl, isoquinolyl and quinoxalinyl, of which benzofuranyl, indolyl, quinolyl and quinoxalinyl are most preferred.

Such heterocyclic groups included in the definitions of R, $R^3$, $R^4$, $R^c$, substituents (a), substituents (b) or substituents (c) may be substituted, in the case of R, $R^3$, $R^4$, $R^c$, substituents (a) and substituents (c), by one or more of substituents (b), and, in the case of substituents (b), by one or more of substituents (a), all of which are as defined above. Examples of such substituents are given above in relation to the substituents on the alkyl groups.

In the compounds of the present invention, when any of R, $R^3$ or $R^c$ represents an alkenyl group, this is either straight chain or branched and has from two to seven, preferably from three to six, carbon atoms, for example the 2-propenyl, 1-methyl-2-propenyl, 2-methyl-2-propenyl, 2-ethyl-2-propenyl, 2-butenyl, 1-methyl-2-butenyl, 2-methyl-2-butenyl, 1-ethyl-2-butenyl, 3-butenyl, 1-methyl-3-butenyl, 2-methyl-3-butenyl, 1-ethyl-3-butenyl, 2-pentenyl, 1-methyl-2-pentenyl, 2-methyl-2-pentenyl, 3-pentenyl, 1-methyl-3-pentenyl, 2-methyl-3-pentenyl, 4-pentenyl, 1-methyl-4-pentenyl, 2-methyl-4-pentenyl, 2-hexenyl, 3-hexenyl, 4-hexenyl, 5-hexenyl, 3-heptenyl, 1-methyl-3-hexenyl, 2-methyl-3-hexenyl, 1-ethyl-3-pentenyl, 2-heptenyl, 1-methyl-2-hexenyl, 2-methyl-2-hexenyl, 4-heptenyl, 1-methyl-3-hexenyl, 2-methyl-3-pentenyl, 5-heptenyl, 1-methyl-4-heptenyl, 2-methyl-4-heptenyl and 6-heptenyl groups.

Such alkenyl groups included within the definitions of R, $R^3$ and $R^c$ can be substituted by one or more of

substituents (a), which are defined above. Examples of such substituents (a) are provided above in relation to the alkyl groups.

In the compounds of the present invention, when either one of R, $R^3$ or $R^c$ represents an alkynyl group, this may be straight chained or branched and has from two to seven, preferably three to six, carbon atoms, and examples include the ethynyl, propargyl (2-propynyl), 1-propynyl, 1-butynyl, 2-butynyl, 3-butynyl, 1-pentynyl, 2-pentynyl, 3-pentynyl and 4-pentynyl groups.

Such alkynyl groups included within the definitions of R, $R^3$ or $R^c$ may be unsubstituted or substituted by one or more of substituents (a), which are defined above. Examples of such substituents (a) are provided above in relation to the alkyl groups.

In the compounds of the present invention, when $R^a$ or $R^b$ represents an aralkyl group, then this may be as exemplified above for aralkyl in relation to substituents (b).

In the compounds of the present invention, when $R^a$ or $R^b$ represents an aralkenyl group, the alkenyl part of this group has from 2 to 7 carbon atoms, and the aryl part may be as defined and exemplified above. Examples include the 2-phenylethenyl, 1-naphthylethenyl, 2-naphthylethenyl, 1-phenylpropenyl, 2-phenylpropenyl, 3-phenylpropenyl, 1-naphthylpropenyl, 2-naphthylpropenyl, 3-naphthylpropenyl, 1-phenylbutenyl, 2-phenylbutenyl, 3-phenylbutenyl, 4-phenylbutenyl, 1-naphthylbutenyl, 2-naphthylbutenyl, 3-naphthylbutenyl, 4-naphthylbutenyl, 1-phenylpentenyl, 2-phenylpentenyl, 3-phenylpentenyl, 4-phenylpentenyl, 5-phenylpentenyl, 1-naphthylpentenyl, 2-naphthylpentenyl, 3-naphthylpentenyl, 4-naphthylpentenyl, 5-naphthylpentenyl, 1-phenylhexenyl, 2-phenylhexenyl, 3-phenylhexenyl, 4-phenylhexenyl, 5-phenylhexenyl, 6-phenylhexenyl, 1-naphthylhexenyl, 2-naphthylhexenyl, 3-naphthylhexenyl, 4-naphthylhexenyl, 5-naphthylhexyl, 6-naphthylhexyl, 1-phenylheptenyl, 2-phenylheptenyl, 3-phenylheptenyl, 4-phenylheptenyl, 5-phenylheptenyl, 6-phenylheptenyl, 7-phenylheptenyl, 1-naphthylheptenyl, 2-naphthylheptenyl, 3-naphthylheptenyl, 4-naphthylheptenyl, 5-naphthylheptyl or 6-naphthylheptyl and 7-naphthylheptyl groups.

Preferred classes of compounds of the present invention include those compounds of formula (I) in which A represents a group of formula -CO- or $-SO_2-$, especially -CO-.

Other preferred classes of compounds of the present invention are as follows:

I) Compounds wherein $R^1$ represents a group of formula

$$R(Z)_xA-$$

wherein R represents

a hydrogen atom,

an alkyl group having from one to six carbon atoms,

an alkyl group having from one to six carbon atoms and being substituted by at least one of substituents (a′) below,

a carbocyclic aryl group having from six to fourteen carbon atoms,

a carbocyclic aryl group having from six to fourteen carbon atoms and being substituted by at least one of substituents (b′) below,

or

a heterocyclic group having from 3 to 10 ring atoms of which from 1 to 4 are nitrogen and/or oxygen and/or sulphur atoms, said heterocyclic group being unsubstituted or substituted by at least one of substituents (b′) below;

Z represents oxygen;

A represents a group of formula -CO- or $-SO_2-$; and

x is the cipher 0 or the integer 1;

substituents (a′):

hydroxy groups, $C_3$-$C_{10}$ cycloalkyl groups, $C_1$-$C_6$ alkoxy groups, $C_1$-$C_6$ aliphatic acyloxy groups, $C_1$-$C_6$ aliphatic acyl groups, carboxy groups, $C_2$-$C_6$ alkoxycarbonyl groups, sulpho groups, halogen atoms, amino groups, $C_2$-$C_4$ aliphatic acylamino groups, alkylamino groups in which the alkyl part is $C_1$-$C_3$ alkyl, dialkylamino groups in which each alkyl part is $C_1$-$C_3$ alkyl, carbocyclic aryl groups having from six to fourteen carbon atoms, carbocyclic aryl groups having from six to fourteen carbon atoms and being substituted by at least one of substituents (b′) below, carbocyclic aryloxy groups having from six to fourteen carbon atoms, carbocyclic aryloxy groups having from six to fourteen carbon atoms and being substituted by at least one of substituents (b′) below, and heterocyclic groups having from 3 to 10 ring atoms of which from 1 to 4 are nitrogen and/or oxygen and/or sulphur heteroatoms, said heterocyclic group being unsubstituted or substituted by at least one of substituents (b′) below;

substituents (b′):

$C_1$-$C_6$ alkyl groups having from 0 to 3 of substituents (a′), $C_1$-$C_2$ haloalkyl groups, $C_1$-$C_6$ aliphatic carboxylic acyl groups having from 0 to 3 of substituents (a′), hydroxy groups, $C_1$-$C_6$ alkoxy groups, $C_6$-$C_{14}$ carbocyclic aryl groups having from 0 to 3 of substituents (b′), halogen atoms, nitro groups, cyano groups, carboxy groups, amino groups, carbamoyl groups, mercapto groups, $C_1$-$C_6$ alkylthio groups, $C_1$-$C_6$ alkylsulphonyl groups and aminosulphonyl groups;
provided that, where a substituent (a′) is substituted by a substituent (b′), then substituent (b′) is not further substituted by a substituent (a′); that where a substituent (b′) is substituted by a substituent (a′), then substituent (a′) is not further substituted by a substituent (b′); and that, where substituent (b′) is a group which is itself substituted by a further substituent (b′), that further substituent (b′) is not itself substituted.

II. Compounds wherein $R^1$ represents a group of formula

$$R(Z)_xA-$$

wherein R represents

an alkyl group having from one to six carbon atoms and being substituted by at least one of substituents (a′) above,

a carbocyclic aryl group having from six to fourteen carbon atoms,

a carbocyclic aryl group having from six to fourteen carbon atoms and being substituted by at least one substituent selected from $C_{1-6}$ alkyl groups, $C_{1-6}$ alkoxy groups and hydroxy groups,

a heterocyclic group having from 3 to 10 ring atoms of which from 1 to 4 are nitrogen and/or oxygen and/or sulphur heteroatoms, said heterocyclic group being unsubstituted or substituted by at least one substituent selected from $C_1$-$C_6$ alkyl groups, $C_1$-$C_6$ alkoxy groups and hydroxy groups,

Z represents oxygen;

A represents a group of formula -CO- or $-SO_2-$; and

x is the cipher 0 or the integer 1

III. Compounds wherein $R^1$ represents a group of formula

$$R(Z)_xA-$$

wherein R represents a group of formula $-NR^aR^b$ wherein $R^a$ and $R^b$ are independently selected from

heterocyclic groups having from 3 to 10 ring atoms of which from 1 to 4 are nitrogen and/or oxygen and/or sulphur heteroatoms, said heterocyclic group being unsubstituted or substituted by at least one $C_1$-$C_6$ alkyl group, $C_1$-$C_6$ alkoxy group or hydroxy group;

carbocyclic aryl groups having from 6 to 14 carbon atoms; and

aralkyl groups in which the aryl part has from 6 to 14 carbon atoms and the alkyl part has from 1 to 3 carbon atoms;

A represents a group of formula -CO-; and

x is the cipher 0.

IV. Compounds wherein $R^1$ represents a group of formula

$$R(Z)_xA-$$

wherein R represents

an alkyl group having from one to six carbon atoms and being substituted by at least one of phenyl, naphthyl, phenoxy and naphthoxy, or phenyl, naphthyl, phenoxy and naphthoxy substituted by at least one substituent selected from methyl, hydroxy, methoxy and phenoxy groups,

a carbocyclic aryl group having from six to fourteen carbon atoms,

a heterocyclic group having from 3 to 10 ring atoms of which from 1 to 4 are nitrogen and/or oxygen and/or sulphur heteroatoms, said heterocyclic group being unsubstituted or substituted by at least one substituent selected from $C_1$-$C_6$ alkyl groups, $C_1$-$C_6$ alkoxy groups and hydroxy groups,

A represents a group of formula -CO-;

Z represents an oxygen atom; and

x is the cipher 0 or the integer 1.

V. Compounds wherein $R^1$ represents a group of formula

$$R(Z)_xA-$$

wherein R represents

an alkyl group having from one to six carbon atoms and being substituted by at least one phenyl, phenoxy, 4-methoxyphenyl, 4-methoxyphenoxy, 3-phenylphenoxy, naphthyl or naphthoxy group,

a carbocyclic aryl group having from six to fourteen carbon atoms,

a benzofuranyl, indolyl, quinolyl or quinoxalinyl group which is unsubstituted or substituted by at least one substituent selected from $C_1$-$C_6$ alkyl groups, $C_1$-$C_6$ alkoxy groups and hydroxy groups,

A represents a group of formula -CO-;

Z represents an oxygen atom; and

x is the cipher 0 or the integer 1.

VI. Compounds wherein $R^2$ represents a hydrogen atom, an alkyl group having from one to four carbon atoms or an alkyl group having from one to four carbon atoms and being substituted with at least one of substituents (a′) above. We especially prefer those compounds wherein $R^2$ represents a hydrogen atom.

VII. Compounds wherein $R^3$ represents

an alkyl group having from one to six carbon atoms;

or

an alkyl group having from one to six carbon atoms and being substituted by at least one of substituents (c′) below;

substituents (c′):

hydroxy groups, $C_1$-$C_6$ alkoxy groups, $C_6$-$C_{14}$ carbocyclic aryl groups having from 0 to 3 of substituents (b′), heterocyclic groups having from 3 to 10 ring atoms of which 1 to 4 are nitrogen and/or oxygen and/or sulphur, said heterocyclic group being unsubstituted or substituted by at least one of substituents (b′), halogen atoms, cyano groups, carboxy groups, amino groups, $C_1$-$C_6$ alkylamino groups, dialkylamino groups wherein each alkyl part is $C_1$-$C_6$ alkyl, carbamoyl groups, alkylcarbamoyl groups where the alkyl part is $C_1$-$C_6$ alkyl, dialkylcarbamoyl groups where each alkyl part is $C_1$-$C_6$ alkyl, $C_1$-$C_6$ alkylthio groups, $C_6$-$C_{14}$ carbocyclic arylthio groups, $C_1$-$C_6$ alkylsulphonyl groups, $C_6$-$C_{14}$ carbocyclic arylsulphonyl groups wherein the aryl part has from 0 to 3 $C_1$-$C_6$ alkyl substituents, aminosulphonyl groups, $C_1$-$C_6$ alkylsulphinyl groups and $C_6$-$C_{14}$ carbocyclic arylsulphinyl groups wherein the aryl part has from 0 to 3 $C_1$-$C_6$ alkyl substituents;

provided that where a substituent (c′) is substituted by a substituent (b′), that further substituent (b′) is not itself substituted.

VIII. Compounds wherein $R^3$ represents

an alkyl group having from one to six carbon atoms;

or

an alkyl group having from one to six carbon atoms and being substituted by at least one of a cyano group, a carbamoyl group, a cycloalkyl group having from three to ten carbon atoms, a carbocyclic aryl group having from six to fourteen carbon atoms, a heterocyclic group having from 3 to 10 ring atoms, of which from 1 to 4 are nitrogen and/or oxygen and/or sulphur heteroatoms, a hydroxy group, a halogen atom, an amino group, a $C_1$-$C_6$ alkylthio group, a $C_1$-$C_6$ alkylsulphonyl group, an aminosulphonyl group and a carboxy group.

Especially preferred are those compounds wherein $R^3$ represents an alkyl group having from one to six carbon atoms and being substituted by at least one of a cyano group, a hydroxy group, a carbamoyl group, a $C_1$-$C_6$ mono- or di- alkylcarbamoyl group, a $C_1$-$C_6$ alkylthio group, a $C_1$-$C_6$ alkylsulphonyl group, an aminosulphonyl group and a carboxy group.

IX. Compounds wherein $R^4$ represents

an alkyl group having from one to six carbon atoms;

or

an alkyl group having from one to six carbon atoms and being substituted by at least one of substituents (a′) above.

X. Compounds wherein $R^4$ represents an alkyl group having from one to six carbon atoms and being substituted by at least one substituent selected from

cycloalkyl groups having from three to ten carbon atoms,

carbocyclic aryl groups having from six to fourteen carbon atoms,

heterocyclic groups having from 3 to 10 ring atoms of which from 1 to 4 are nitrogen and/or oxygen and/or sulphur heteroatoms,

hydroxy groups,

halogen atoms, and

amino groups.

XI. Compounds wherein $R^4$ represents an alkyl group having from one to six carbon atoms and being. substituted by at least one substituent selected from

cycloalkyl groups having from three to ten carbon atoms,

carbocyclic aryl groups having from six to fourteen carbon atoms, and

heterocyclic groups having from 3 to 10 ring atoms of which from 1 to 4 are nitrogen and/or oxygen and/or sulphur heteroatoms.

XII. Compounds wherein $R^4$ represents an alkyl group having from one to six carbon atoms and being substituted by at least one of a cycloalkyl group having from three to ten carbon atoms, and a carbocyclic aryl group

having from six to fourteen carbon atoms, especially those compounds wherein $R^4$ represents an alkyl group having from one to six carbon atoms and being substituted by a carbocyclic aryl group having from six to fourteen carbon atoms, and particularly those compounds wherein $R^4$ represents a benzyl or cyclohexylmethyl group.

XIII. Compounds wherein $R^5$ represents a group of formula $-B-(CO)-Y-R^c_y$ , wherein

B represents a heterocyclic group having from 3 to 10 ring atoms of which from 1 to 4 are nitrogen and/or oxygen and/or sulphur heteroatoms, at least one of which is a nitrogen heteroatom, said heterocyclic group being unsubstituted or substituted with at least one alkyl group having from one to six carbon atoms;

Y represents an oxygen atom or a nitrogen atom;

y is the integer 1 when Y represents an oxygen atom and 2 when Y represents a nitrogen atom; and

$R^c$ represents

a hydrogen atom,

an alkyl group having from one to six carbon atoms,

an alkyl group having from one to six carbon atoms and being substituted by at least one of substituents (a′) above;

and, where there are two groups or atoms represented by $R^c$, they are the same or different.

XIV. Compounds wherein $R^5$ represents a group of formula $-B-(CO)-Y-R^c$, wherein

B represents a heterocyclic group having from 3 to 10 ring atoms of which from 1 to 4 are nitrogen and/or oxygen and/or sulphur heteroatoms, at least one of which is a nitrogen heteroatom, said heterocyclic group being unsubstituted or substituted with at least one alkyl group having from one to six carbon atoms;

Y represents an oxygen atom; and

$R^c$ represents

an alkyl group having from one to six carbon atoms, or

an alkyl group having from one to six carbon atoms and being substituted by at least one substituent selected from:

cycloalkyl groups having from three to ten carbon atoms,

carbocyclic aryl groups having from six to fourteen carbon atoms,

heterocyclic groups having from 3 to 10 ring atoms of which from 1 to 4 are nitrogen and/or oxygen and/or sulphur heteroatoms, and hydroxy groups.

XV. Compounds wherein $R^5$ represents a group of formula $-B-(CO)-Y-R^c_y$ , wherein

B represents a heterocyclic group having from 3 to 10 ring atoms of which from 1 to 4 are nitrogen and/or oxygen and/or sulphur heteroatoms, at least one of which is a nitrogen heteroatom, said heterocyclic group being unsubstituted or substituted with at least one alkyl group having from one to six carbon atoms;

Y represents a nitrogen atom;

y is the integer 2; and

$R^c$ represents

an alkyl group having from one to six carbon atoms, or

an alkyl group having from one to six carbon atoms and being substituted by at least one substituent selected from:

cycloalkyl groups having from three to ten carbon atoms,

carbocyclic aryl groups having from six to fourteen carbon atoms,

heterocyclic groups having from 3 to 10 ring atoms of which from 1 to 4 are nitrogen and/or oxygen and/or sulphur heteroatoms, and hydroxy groups;

and, where there are two groups or atoms represented by $R^c$, they are the same or different.

XVI. Compounds wherein $R^1$ represents a group of formula

$$R (Z)_x A-$$

wherein R represents

a hydrogen atom,

an alkyl group having from one to six carbon atoms,

an alkyl group having from one to six carbon atoms and being substituted by at least one of substituents (a′) above,

a carbocyclic aryl group having from six to fourteen carbon atoms,

a carbocyclic aryl group having from six to fourteen carbon atoms and being substituted by at least one of substituents (b′) above,

a heterocyclic group having from 3 to 10 ring atoms of which 1 to 4 are nitrogen and/or oxygen and/or sulphur atoms, said heterocyclic group being unsubstituted or substituted by at least one of substituents (b′), above,

A represents the group -CO-,
Z represents an oxygen atom; and
x is the cipher 0 or the integer 1;
$R^2$ represents a hydrogen atom;
$R^3$ represents
an alkyl group having from one to six carbon atoms,
an alkyl group having from one to six carbon atoms and being substituted by at least one of substituents (c'), above;
$R^4$ represents
an alkyl group having from one to six carbon atoms and being substituted by at least one substituent selected from carbocyclic aryl groups having from six to fourteen carbon atoms and carbocyclic aryl groups having from six to fourteen carbon atoms and substituted with at least one $C_1$-$C_6$ alkyl group;
and
$R^5$ represents a group of formula $-B-(CO)-Y-R^c_y$ , wherein
B represents a heterocyclic group having from 3 to 10 ring atoms of which from 1 to 4 are nitrogen and/or oxygen and/or sulphur heteroatoms, at least one of which is a nitrogen heteroatom, said heterocyclic group being unsubstituted or substituted with at least one alkyl group having from one to six carbon atoms;
Y represents an oxygen atom or a nitrogen atom;
y is the integer 1 when Y represents an oxygen atom and 2 when Y represents a nitrogen atom; and
$R^c$ represents
an alkyl group having from one to six carbon atoms,
an alkyl group having from one to six carbon atoms and being substituted by at least one substituent selected from:
cycloalkyl groups having from three to ten carbon atoms,
carbocyclic aryl groups having from six to fourteen carbon atoms,
heterocyclic groups having from 3 to 10 ring atoms of which from 1 to 4 are nitrogen and/or oxygen and/or sulphur heteroatoms,
hydroxy groups,
halogen atoms, and
amino groups;
and, where there are two groups or atoms represented by $R^c$, they are the same or different.
and pharmaceutically acceptable salts and esters thereof, are particularly preferred.

The compounds of the present invention necessarily contain several asymmetric carbon atoms in their molecules, and can thus form optical isomers. Although these are all represented herein by a single molecular formula, the present invention includes both the individual, isolated isomers and mixtures, including racemates thereof. Where stereospecific synthesis techniques are employed or optically active compounds are employed as starting materials, individual isomers may be prepared directly; on the other hand, if a mixture of isomers is prepared, the individual isomers may be obtained by conventional resolution techniques.

The compounds of the present invention can form salts. There is no particular restriction on the nature of these salts, provided that, where they are intended for therapeutic use, they are pharmaceutically acceptable. Where they are intended for non-therapeutic uses, e.g. as intermediates in the preparation of other, and possibly more active, compounds, even this restriction does not apply. When the compounds of the present invention contain an acidic group, they can form salts with bases. Examples of such salts include: salts with an alkali metal, such as sodium, potassium or lithium; salts with an alkaline earth metal, such as barium or calcium; salts with another metal, such as magnesium and aluminum; organic base salts, such as a salt with dicyclohexylamine; and salts with a basic amino acid, such as lysine or arginine. Also, where the compound of the present invention contains a basic group in its molecule, it can form acid addition salts. Examples of such acid addition salts include: salts with mineral acids, especially hydrohalic acids (such as hydrofluoric acid, hydrobromic acid, hydroiodic acid or hydrochloric acid), nitric acid, carbonic acid, sulphuric acid or phosphoric acid; salts with lower alkylsulphonic acids, such as methanesulphonic acid, trifluoromethanesulphonic acid or ethanesulphonic acid; salts with arylsulphonic acids, such as benzenesulphonic acid or p-toluenesulphonic acid; salts with organic carboxylic acids, such as acetic acid, fumaric acid, tartaric acid, oxalic acid, maleic acid, malic acid, succinic acid or citric acid; and salts with amino acids, such as glutamic acid or aspartic acid.

Specific examples of individual compounds of the present invention are shown in the following formula:

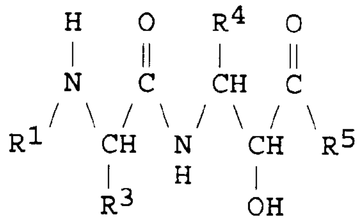

(IA)

In the above formula, the meanings of the various substituent groups are as given in the following Table. In the Table, the following abbreviations are used:

| | |
|---|---|
| Ac | acetyl |
| Azt | azetidinyl |
| tBoc | t-butoxycarbonyl |
| Boz | benzoyl |
| Bu | butyl |
| iBu | isobutyl |
| tBu | t-butyl |
| Bfur | benzofuranyl |
| Bz | benzyl |
| Bzc | benzyloxycarbonyl |
| Bzhy | benzhydryl |
| Bzim | benzimidazolyl |
| Car | carbamoyl |
| Et | ethyl |
| cHx | cyclohexyl |
| Dhiq | decahydroisoquinolyl |
| Imid | imidazolyl |
| Ind | indolyl |
| Indi | indolinyl |
| Me | methyl |
| Mes | methanesulphonyl |
| Mor | morpholino |
| Mph | p-methoxyphenyl |
| Np | naphthyl |
| Npo | naphthoyl |
| Ph | phenyl |
| Pip | piperidyl |
| Pr | propyl |
| iPr | isopropyl |
| Prc | propoxycarbonyl |
| Pyr | pyridyl |
| Pyrd | pyrrolidinyl |
| Pyz | pyrazinyl |
| Quix | quinoxalinyl |
| Sam | sulphamoyl |
| Sfo | sulpho |
| Thi | thienyl |
| Thiz | thiazolyl |
| Thiq | 1H,2H,3H,4H-tetrahydroisoquinolyl |
| Thz | Thiazolidinyl |

TABLE 1

| Compd. No. | $R^1$ | $R^3$ | $R^4$ | $R^5$ |
|---|---|---|---|---|
| 1 | Bzc | $CarCH_2$- | Bz | 2-tBoc-1-Pyrd |
| 2 | 2-Pyr.CO- | $CarCH_2$- | Bz | 2-tBoc-1-Pyrd |
| 3 | 2-Quin.CO- | $CarCH_2$- | Bz | 2-tBoc-1-Pyrd |
| 4 | 3-Quin.CO- | $CarCH_2$- | Bz | 2-tBoc-1-Pyrd |
| 5 | 4-Quin.CO- | $CarCH_2$- | Bz | 2-tBoc-1-Pyrd |
| 6 | 4-MeO-2-Quin.CO- | $CarCH_2$- | Bz | 2-tBoc-1-Pyrd |
| 7 | 2-Np.CO- | $CarCH_2$- | Bz | 2-tBoc-1-Pyrd |
| 8 | 2-Bfur.CO- | $CarCH_2$- | Bz | 2-tBoc-1-Pyrd |
| 9 | 2-Ind.CO- | $CarCH_2$- | Bz | 2-tBoc-1-Pyrd |
| 10 | 2-Quix.CO- | $CarCH_2$- | Bz | 2-tBoc-1-Pyrd |
| 11 | 5-Bu-2-Pyr.CO- | $CarCH_2$- | Bz | 2-tBoc-1-Pyrd |
| 12 | 2-Pyz.CO- | $CarCH_2$- | Bz | 2-tBoc-1-Pyrd |
| 13 | 4-OH-2-Quin.CO- | $CarCH_2$- | Bz | 2-tBoc-1-Pyrd |
| 14 | 3-OH-2-Quix.CO- | $CarCH_2$- | Bz | 2-tBoc-1-Pyrd |
| 15 | 2-Np-Sfo- | $CarCH_2$- | Bz | 2-tBoc-1-Pyrd |
| 16 | Boz | $CarCH_2$- | Bz | 2-tBoc-1-Pyrd |
| 17 | Bz-NHCO- | $CarCH_2$- | Bz | 2-tBoc-1-Pyrd |
| 18 | Bz-NHCS- | $CarCH_2$- | Bz | 2-tBoc-1-Pyrd |
| 19 | Bzc | $CarCH_2$- | Bz | 2-tBuNHCO-1-Pyrd |
| 20 | 2-Quin.CO- | $CarCH_2$- | Bz | 2-tBuNHCO-1-Pyrd |
| 21 | 3-Quin.CO- | $CarCH_2$- | Bz | 2-tBuNHCO-1-Pyrd |
| 22 | 2-Np.CO- | $CarCH_2$- | Bz | 2-tBuNHCO-1-Pyrd |
| 23 | 2-Quix.CO- | $CarCH_2$- | Bz | 2-tBuNHCO-1-Pyrd |
| 24 | 2-Bfur.CO- | $CarCH_2$- | Bz | 2-tBuNHCO-1-Pyrd |
| 25 | 2-Ind.CO- | $CarCH_2$- | Bz | 2-tBuNHCO-1-Pyrd |
| 26 | Bzc | 2-Car-1-Et- | Bz | 2-tBoc-1-Pyrd |
| 27 | 2-Quin.CO- | 2-Car-1-Et- | Bz | 2-tBoc-1-Pyrd |

TABLE 1 (cont.)

| Compd. No. | $R^1$ | $R^3$ | $R^4$ | $R^5$ |
|---|---|---|---|---|
| 28 | 3-Quin.CO- | 2-Car-1-Et- | Bz | 2-tBoc-1-Pyrd |
| 29 | 2-Bfur.CO- | 2-Car-1-Et- | Bz | 2-tBoc-1-Pyrd |
| 30 | 2-Np.CO- | 2-Car-1-Et- | Bz | 2-tBoc-1-Pyrd |
| 31 | 2-Np-Sfo | 2-Car-1-Et- | Bz | 2-tBoc-1-Pyrd |
| 32 | 2-Ind.CO- | 2-Car-1-Et- | Bz | 2-tBoc-1-Pyrd |
| 33 | 2-Quix.CO- | 2-Car-1-Et- | Bz | 2-tBoc-1-Pyrd |
| 34 | Bzc | $CNCH_2$- | Bz | 2-tBoc-1-Pyrd |
| 35 | 2-Quin.CO- | $CNCH_2$- | Bz | 2-tBoc-1-Pyrd |
| 36 | 3-Quin.CO- | $CNCH_2$- | Bz | 2-tBoc-1-Pyrd |
| 37 | 2-Bfur.CO- | $CNCH_2$- | Bz | 2-tBoc-1-Pyrd |
| 38 | 2-Np.CO- | $CNCH_2$- | Bz | 2-tBoc-1-Pyrd |
| 39 | 2-Ind.CO- | $CNCH_2$- | Bz | 2-tBoc-1-Pyrd |
| 40 | 2-Quix.CO- | $CNCH_2$- | Bz | 2-tBoc-1-Pyrd |
| 41 | Bzc | $CarCH_2$- | 4-BrBz | 2-tBoc-1-Pyrd |
| 42 | 2-Quin.CO- | $CarCH_2$- | 4-MeOBz | 2-tBoc-1-Pyrd |
| 43 | 2-Quin.CO- | $CarCH_2$- | 4-MeBz | 2-tBoc-1-Pyrd |
| 44 | Bzc | $CarCH_2$- | Bz | 2-iPrc-1-Pyrd |
| 45 | 2-Quix.CO- | $CarCH_2$- | Bz | 2-iPrc-1-Pyrd |
| 46 | 2-Bfur.CO- | $CarCH_2$- | Bz | 2-iPrc-1-Pyrd |
| 47 | Bzc | $CarCH_2$- | Bz | 2-$cHxCH_2OCO$-1-Pyrd |
| 48 | 2-Quin.CO- | $CarCH_2$- | Bz | 2-$cHxCH_2OCO$-1-Pyrd |
| 49 | Bzc | $CarCH_2$- | Bz | 2-(2-MeBu)OCO-1-Pyrd |
| 50 | 2-Quix.CO- | $CarCH_2$- | Bz | 2-(2-MeBu)OCO-1-Pyrd |
| 51 | Bzc | $CarCH_2$- | Bz | 2-(2-MeBu)NHCO-1-Pyrd |
| 52 | 2-Quin.CO- | $CarCH_2$- | Bz | 2-(2-MeBu)NHCO-1-Pyrd |
| 53 | 2-Bfur.CO- | $CarCH_2$- | Bz | 2-(2-MeBu)NHCO-1-Pyrd |
| 54 | Bzc | $CarCH_2$- | Bz | 2-$cHxCH_2NHCO$-1-Pyrd |

TABLE 1 (cont.)

| Compd. No. | $R^1$ | $R^3$ | $R^4$ | $R^5$ |
|---|---|---|---|---|
| 55 | 2-Quix.CO- | $CarCH_2$- | Bz | 2-$cHxCH_2$NHCO-1-Pyrd |
| 56 | 2-Quix.CO- | $CarCH_2$- | Bz | 2-BzNHCO-1-Pyrd |
| 57 | 2-Quix.CO- | $CarCH_2$- | Bz | 2-tBoc-1-Pip |
| 58 | 2-Quin.CO- | $CarCH_2$- | Bz | 2-tBoc-1-Pip |
| 59 | 2-Bfur.CO- | $CarCH_2$- | Bz | 2-tBoc-1-Pip |
| 60 | Bzc | $CarCH_2$- | Bz | 3-tBuNHCO-2-Dhiq |
| 61 | 2-Quin.CO- | $CarCH_2$- | Bz | 3-iBuNHCO-2-Thiq |
| 62 | 2-Quin.CO- | $CarCH_2$- | Bz | 3-tBoc-2-Dhiq |
| 63 | 2-Quix.CO- | $CarCH_2$- | Bz | 3-tBoc-2-Dhiq |
| 64 | 2-Bfur.CO- | $CarCH_2$- | Bz | 3-tBoc-2-Dhiq |
| 65 | 2-Bfur.CO- | $CarCH_2$- | Bz | 3-tBuNHCO-2-Dhiq |
| 66 | 2-Quix.CO- | $CarCH_2$- | Bz | 3-tBuNHCO-2-Dhiq |
| 67 | 2-Quix.CO- | $CarCH_2$- | Bz | 2-tBoc-1-Azt |
| 68 | 2-Quin.CO- | $CarCH_2$- | Bz | 2-tBoc-1-Azt |
| 69 | 2-Quin.CO- | iPr | Bz | 2'-MorEtNHCO-1-Pyrd |
| 70 | Bzc | iPr | Bz | 2'-MorEtNHCO-1-Pyrd |
| 71 | Bzc | tBu | Bz | 2-(2-MeBu)NHCO-1-Pyrd |
| 72 | 2-Quin.CO- | tBu | Bz | 2-(2-MeBu)NHCO-1-Pyrd |
| 73 | 2-Quin.CO- | $DiMeCarCH_2$- | Bz | 2-tBoc-1-Pyrd |
| 74 | Bzc | $DiMeCarCH_2$- | Bz | 2-tBoc-1-Pyrd |
| 75 | Bzc | $MorCOCH_2$- | Bz | 2-tBoc-1-Pyrd |
| 76 | Bzc | $PipCOCH_2$- | Bz | 2-tBoc-1-Pyrd |
| 77 | 2-Quin.CO- | $CarCH_2$- | Bz | 2-tBoc-1-Indi |
| 78 | 2-Quix.CO- | $CarCH_2$- | Bz | 2-tBoc-1-Indi |
| 79 | 2-Quix.CO- | $CarCH_2$- | Bz | 2-BuNHCO-1-Pyrd |
| 80 | 2-Bfur.CO- | $CarCH_2$- | Bz | 2-BuNHCO-1-Pyrd |
| 81 | Bzc | $CarCH_2$- | Bz | 2-BuNHCO-1-Pyrd |

TABLE 1 (cont.)

| Compd. No. | $R^1$ | $R^3$ | $R^4$ | $R^5$ |
|---|---|---|---|---|
| 82 | Bzc | $CarCH_2$- | Bz | 2-(2-Pyr)$CH_2$NHCO-1-Pyrd |
| 83 | 2-Quin.CO- | $CarCH_2$- | Bz | 2-(2-Pyr)$CH_2$NHCO-1-Pyrd |
| 84 | Bzc | $CarCH_2$- | Bz | 2-(2-Pyr)$CH_2$OCO-1-Pyrd |
| 85 | 2-Quin.CO- | $CarCH_2$- | Bz | 2-(2-Pyr)$CH_2$OCO-1-Pyrd |
| 86 | Bzc | $CarCH_2$- | $cHxCH_2$- | 2-tBuNHCO-1-Pyrd |
| 87 | 2-Quin.CO- | $CarCH_2$- | $cHxCH_2$- | 2-tBuNHCO-1-Pyrd |
| 88 | tBoc | $CarCH_2$- | Bz | 2-tBuNHCO-1-Pyrd |
| 89 | 2-Pyrd.CO- | $CarCH_2$- | Bz | 2-tBoc-1-Pyrd |
| 90 | 2-Pip.CO- | $CarCH_2$- | Bz | 2-tBoc-1-Pyrd |
| 91 | 2-Pyr.CO- | $CarCH_2$- | Bz | 2-tBoc-1-Pyrd |
| 92 | 3-Pyr.CO- | $CarCH_2$- | Bz | 2-tBoc-1-Pyrd |
| 93 | 2-Thi.CO- | $CarCH_2$- | Bz | 2-tBoc-1-Pyrd |
| 94 | Bzc | 2-$H_2$NEt- | Bz | 2-tBoc-1-Pyrd |
| 95 | Bzc | 3-$H_2$NPr- | Bz | 2-tBoc-1-Pyrd |
| 96 | Bzc | $MeCarCH_2$- | Bz | 2-tBoc-1-Pyrd |
| 97 | Bzc | $EtCarCH_2$- | Bz | 2-tBoc-1-Pyrd |
| 98 | Bzc | H | Bz | 2-tBoc-1-Pyrd |
| 99 | Bzc | Bz | Bz | 2-tBoc-1-Pyrd |
| 100 | Bzc | $HOCH_2$- | Bz | 2-tBoc-1-Pyrd |
| 101 | Bzc | 4-$ThizCH_2$- | Bz | 2-tBoc-1-Pyrd |
| 102 | Bzc | 4-$ImidCH_2$- | Bz | 2-tBoc-1-Pyrd |
| 103 | Bzc | $CarCH_2$- | Bz | 2-(1-HOMe-2-MeBuNHCO)-1-Pyrd |
| 104 | 2-Quin.CO- | $CarCH_2$- | Bz | 2-(1-HOMe-2-MeBuNHCO)-1-Pyrd |
| 105 | Bzc | $CarCH_2$- | Bz | 2-DiMeCar-1-Pyrd |
| 106 | Bzc | $CarCH_2$- | Bz | 2-BzNHCO-1-Pyrd |

TABLE 1 (cont.)

| Compd. No. | $R^1$ | $R^3$ | $R^4$ | $R^5$ |
|---|---|---|---|---|
| 107 | 2-Quix.CO- | $CarCH_2$- | Bz | 2-BzNHCO-1-Pyrd |
| 108 | 2-Indi.CO- | $CarCH_2$- | Bz | 2-tBoc-1-Pyrd |
| 109 | Bzc | $CarCH_2$- | Bz | 2-BzOCO-1-Pyrd |
| 110 | Bzc | $CarCH_2$- | Bz | 2-Car-1-Pyrd |
| 111 | Bzc | $CarCH_2$- | Bz | 2-COOH-1-Pyrd |
| 112 | Bzc | $CarCH_2$- | Bz | 2-MeOCO-1-Pyrd |
| 113 | MeOCOCO- | $CarCH_2$- | Bz | 2-tBoc-1-Pyrd |
| 114 | Bzc | $CarCH_2$- | Bz | 2-EtOCO-1-Pyrd |
| 115 | PhOAc | $CarCH_2$- | Bz | 2-tBoc-1-Pyrd |
| 116 | MphOAc | $CarCH_2$- | Bz | 2-tBuNHCO-1-Pyrd |
| 117 | 5-MeO-2-Ind.CO- | $CarCH_2$- | Bz | 2-tBuNHCO-1-Pyrd |
| 118 | 5-HO-2-Ind.CO- | $CarCH_2$- | Bz | 2-tBuNHCO-1-Pyrd |
| 119 | 5-AcO-2-Ind.CO- | $CarCH_2$- | Bz | 2-tBuNHCO-1-Pyrd |
| 120 | 5-$H_2$NAcO- -2-Ind.CO- | $CarCH_2$- | Bz | 2-tBuNHCO-1-Pyrd |
| 121 | Bzc | $CarCH_2$- | Bz | 2-(2-PhEt)NHCO-1-Pyrd |
| 122 | Bzc | $CarCH_2$- | Bz | 2-BzhyNHCO-1-Pyrd |
| 123 | Bzc | $CarCH_2$- | Bz | 2-PhCH(Me)NHCO-1-Pyrd |
| 124 | Bzc | $CarCH_2$- | Bz | 2-[N(Me)BuCO]-1-Pyrd |
| 125 | Bzc | $CarCH_2$- | Bz | 2-(3-Pyr)$CH_2$NHCO- -1-Pyrd |
| 126 | Bzc | $CarCH_2$- | Bz | 2-(4-Pyr)$CH_2$NHCO- -1-Pyrd |
| 127 | Bzc | $CarCH_2$- | Bz | 2-(1-Et-2-Pyrd)- -$CH_2$NHCO-1-Pyrd |
| 128 | Bzc | $CarCH_2$- | Bz | 2-(3-HOPr)NHCO-1-Pyrd |
| 129 | Bzc | $HOOCCH_2$- | Bz | 2-tBoc-1-Pyrd |

TABLE 1 (cont.)

| Compd. No. | $R^1$ | $R^3$ | $R^4$ | $R^5$ |
|---|---|---|---|---|
| 130 | Bzc | 2-HOOCEt- | Bz | 2-tBoc-1-Pyrd |
| 131 | 5-Bzim.CO- | $CarCH_2$- | Bz | 2-tBoc-1-Pyrd |
| 132 | Bzc | $CarCH_2$- | Bz | 2-(1,1-DiMe-2-HOEt)--NHCO-1-Pyrd |
| 133 | Bzc | $CarCH_2$- | Bz | 2-(1-Me-2-HOEt)NHCO-1-Pyrd |
| 134 | Bzc | $CarCH_2$- | Bz | 2-(1,1-DiHOMe-2-HOEt)NHCO-1-Pyrd |
| 135 | 2-Quix.CO- | $CarCH_2$- | Bz | 2-(1,1-DiMe-2-HOEt)--NHCO-1-Pyrd |
| 136 | (2-NpO)Ac | $CarCH_2$- | Bz | 2-tBuNHCO-1-Pyrd |
| 137 | 2-Quin.CO- | $CarCH_2$- | Bz | 4-tBuNHCO-1-Thz |
| 138 | 3-Quin.CO- | $CarCH_2$- | Bz | 4-tBuNHCO-1-Thz |
| 139 | 2-Quix.CO- | $CarCH_2$- | Bz | 4-tBuNHCO-1-Thz |
| 140 | 2-Npo | $CarCH_2$- | Bz | 4-tBuNHCO-1-Thz |
| 141 | 2-Bfur.CO- | $CarCH_2$- | Bz | 4-tBuNHCO-1-Thz |
| 142 | 2-Ind.CO- | $CarCH_2$- | Bz | 4-tBuNHCO-1-Thz |
| 143 | (2-NpO)Ac | $CarCH_2$- | Bz | 4-tBuNHCO-1-Thz |
| 144 | MphOAc | $CarCH_2$- | Bz | 4-tBuNHCO-1-Thz |
| 145 | 2-Quin.CO- | $CarCH_2$- | Bz | 5,5-DiMe-4-tBuNHCO-1-Thz |
| 146 | 3-Quin.CO- | $CarCH_2$- | Bz | 5,5-DiMe-4-tBuNHCO-1-Thz |
| 147 | 2-Quix.CO- | $CarCH_2$- | Bz | 5,5-DiMe-4-tBuNHCO-1-Thz |
| 148 | 2-Npo | $CarCH_2$- | Bz | 5,5-DiMe-4-tBuNHCO-1-Thz |
| 149 | 2-Bfur.CO- | $CarCH_2$- | Bz | 5,5-DiMe-4-tBuNHCO-1-Thz |
| 150 | 2-Ind.CO- | $CarCH_2$- | Bz | 5,5-DiMe-4-tBuNHCO-1-Thz |
| 151 | (2-NpO)Ac | $CarCH_2$- | Bz | 5,5-DiMe-4-tBuNHCO-1-Thz |
| 152 | MphOAc | $CarCH_2$- | Bz | 5,5-DiMe-4-tBuNHCO-1-Thz |

TABLE 1 (cont.)

| Compd. No. | $R^1$ | $R^3$ | $R^4$ | $R^5$ |
|---|---|---|---|---|
| 153 | (3-PhPhO)Ac | $CarCH_2$- | Bz | 2-tBuNHCO-1-Pyrd |
| 154 | (3-PhPhO)Ac | $CarCH_2$- | Bz | 4-tBuNHCO-1-Thz |
| 155 | Bzc | $MeSCH_2$- | Bz | 2-tBuNHCO-1-Pyrd |
| 156 | 2-Quin.CO- | $MeSCH_2$- | Bz | 2-tBuNHCO-1-Pyrd |
| 157 | 2-Quin.CO- | $MeSCH_2$- | Bz | 4-tBuNHCO-1-Thz |
| 158 | 2-Quin.CO- | $MeSCH_2$- | Bz | 5,5-DiMe-4-tBuNHCO-1-Thz |
| 159 | 3-Quin.CO- | $MeSCH_2$- | Bz | 5,5-DiMe-4-tBuNHCO-1-Thz |
| 160 | 3-Quin.CO- | $MeSCH_2$- | Bz | 4-tBuNHCO-1-Thz |
| 161 | 3-Quin.CO- | $MeSCH_2$- | Bz | 2-tBuNHCO-1-Pyrd |
| 162 | 2-Quix.CO- | $MeSCH_2$- | Bz | 2-tBuNHCO-1-Pyrd |
| 163 | 2-Quix.CO- | $MeSCH_2$- | Bz | 5,5-DiMe-4-tBuNHCO-1-Thz |
| 164 | 2-Quix.CO- | $MeSCH_2$- | Bz | 4-tBuNHCO-1-Thz |
| 165 | 2-Ind.CO- | $MeSCH_2$- | Bz | 4-tBuNHCO-1-Thz |
| 166 | 2-Ind.CO- | $MeSCH_2$- | Bz | 5,5-DiMe-4-tBuNHCO-1-Thz |
| 167 | (1-NpO)Ac | $MeSCH_2$- | Bz | 2-tBuNHCO-1-Pyrd |
| 168 | MphOAc | $MeSCH_2$- | Bz | 2-tBuNHCO-1-Pyrd |
| 169 | MphOAc | $MeSCH_2$- | Bz | 4-tBuNHCO-1-Thz |
| 170 | MphOAc | $MeSCH_2$- | Bz | 2-tBuNHCO-1-Pyrd |
| 171 | MphOAc | $MesCH_2$- | Bz | 2-tBuNHCO-1-Pyrd |
| 172 | MphOAc | $MesCH_2$- | Bz | 4-tBuNHCO-1-Thz |
| 173 | MphOAc | $MesCH_2$- | Bz | 5,5-DiMe-4-tBuNHCO-1-Thz |
| 174 | (1-NpO)Ac | $MesCH_2$- | Bz | 4-tBuNHCO-1-Thz |
| 175 | (1-NpO)Ac | $MesCH_2$- | Bz | 2-tBuNHCO-1-Pyrd |
| 176 | 2-Ind.CO- | $MesCH_2$- | Bz | 2-tBuNHCO-1-Pyrd |
| 177 | 2-Ind.CO- | $MesCH_2$- | Bz | 4-tBuNHCO-1-Thz |
| 178 | 2-Ind.CO- | $MesCH_2$- | Bz | 5,5-DiMe-4-tBuNHCO-1-Thz |
| 179 | 2-Quix.CO- | $MesCH_2$- | Bz | 5,5-DiMe-4-tBuNHCO-1-Thz |

TABLE 1 (cont.)

| Compd. No. | $R^1$ | $R^3$ | $R^4$ | $R^5$ |
|---|---|---|---|---|
| 180 | 2-Quix.CO- | $MesCH_2$- | Bz | 4-tBuNHCO-1-Thz |
| 181 | 2-Quix.CO- | $MesCH_2$- | Bz | 2-tBuNHCO-1-Pyrd |
| 182 | 2-Quin.CO- | $MesCH_2$- | Bz | 2-tBuNHCO-1-Pyrd |
| 183 | 2-Quin.CO- | $MesCH_2$- | Bz | 4-tBuNHCO-1-Thz |
| 184 | 2-Quin.CO- | $MesCH_2$- | Bz | 5,5-DiMe-4-tBuNHCO-1-Thz |
| 185 | 3-Quin.CO- | $MesCH_2$- | Bz | 5,5-DiMe-4-tBuNHCO-1-Thz |
| 186 | 3-Quin.CO- | $MesCH_2$- | Bz | 4-tBuNHCO-1-Thz |
| 187 | 3-Quin.CO- | $MesCH_2$- | Bz | 2-tBuNHCO-1-Pyrd |
| 188 | (3-PhPhO)Ac | $MesCH_2$- | Bz | 2-tBuNHCO-1-Pyrd |
| 189 | (3-PhPhO)Ac | $MesCH_2$- | Bz | 4-tBuNHCO-1-Thz |
| 190 | (3-PhPhO)Ac | $SamCH_2$- | Bz | 5,5-DiMe-4-tBuNHCO-1-Thz |
| 191 | (3-PhPhO)Ac | $SamCH_2$- | Bz | 4-tBuNHCO-1-Thz |
| 192 | (3-PhPhO)Ac | $SamCH_2$- | Bz | 2-tBuNHCO-1-Pyrd |
| 193 | MphOAc | $SamCH_2$- | Bz | 2-tBuNHCO-1-Pyrd |
| 194 | MphOAc | $SamCH_2$- | Bz | 4-tBuNHCO-1-Thz |
| 195 | MphOAc | $SamCH_2$- | Bz | 5,5-DiMe-4-tBuNHCO-1-Thz |
| 196 | (1-NpO)Ac | $SamCH_2$- | Bz | 5,5-DiMe-4-tBuNHCO-1-Thz |
| 197 | (1-NpO)Ac | $SamCH_2$- | Bz | 4-tBuNHCO-1-Thz |
| 198 | (1-NpO)Ac | $SamCH_2$- | Bz | 2-tBuNHCO-1-Pyrd |
| 199 | 2-Quix.CO- | $SamCH_2$- | Bz | 2-tBuNHCO-1-Pyrd |
| 200 | 2-Quix.CO- | $SamCH_2$- | Bz | 4-tBuNHCO-1-Thz |
| 201 | 2-Quix.CO- | $SamCH_2$- | Bz | 5,5-DiMe-4-tBuNHCO-1-Thz |
| 202 | MphOAc | $CarCH_2$- | Bz | 2-(1,1-DiMe-2-HOEt)NHCO-1-Pyrd |
| 203 | (1-NpO)Ac | $CarCH_2$- | Bz | 2-(1,1-DiMe-2-HOEt)NHCO-1-Pyrd |
| 204 | (1-NpO)Ac | $MeSCH_2$- | Bz | 2-(1,1-DiMe-2-HOEt)NHCO-1-Pyrd |
| 205 | (1-NpO)Ac | $MesCH_2$- | Bz | 2-(1,1-DiMe-2-HOEt)NHCO-1-Pyrd |
| 206 | MphOAc | $MesCH_2$- | Bz | 2-(1,1-DiMe-2-HOEt)NHCO-1-Pyrd |

TABLE 1 (cont.)

| Compd. No. | $R^1$ | $R^3$ | $R^4$ | $R^5$ |
|---|---|---|---|---|
| 207 | MphOAc | $MeSCH_2$- | Bz | 2-(1,1-DiMe-2-HOEt)NHCO-1-Pyrd |
| 208 | 2-Quix.CO- | $HOOCCH_2$- | Bz | 2-tBuNHCO-1-Pyrd |
| 209 | Bzc | $CarCH_2$- | Bz | 3-iBuNHCO-2-Thiq |
| 210 | 2-Ind.CO- | $CarCH_2$- | Bz | 2-tBuSCO-1-Pyrd |
| 211 | 2-Quix.CO- | $CarCH_2$- | Bz | 2-tBuSCO-1-Pyrd |
| 212 | MphOAc | $CarCH_2$- | Bz | 2-tBuSCO-1-Pyrd |
| 213 | 2-Quix.CO- | $CarCH_2$- | Bz | 4-tBuSCO-1-Thz |
| 214 | MphOAc | $MesCH_2$- | Bz | 2-tBuSCO-1-Pyrd |
| 215 | 2-Quix.CO- | $MesCH_2$- | Bz | 5,5-DiMe-4-tBuNHCO-1-Thz |

Of the compounds listed above, preferred are compounds no.: 1, 3, 4, 6, 7, 8, 9, 10, 11, 19, 20, 21, 22, 23, 24, 25, 27, 28, 29, 30, 32, 33, 43, 44, 45, 46, 49, 50, 51, 52, 53, 79, 80, 81, 114, 115, 116, 117, 118, 128, 129, 130, 132, 133, 135, and 139 to 215 inclusive;

More preferred are compounds no.: 1, 3, 4, 7, 8, 9, 10, 19, 20, 21, 22, 23, 24, 25, 44, 45, 46, 50, 52, 53, 79, 81, 114, 116, 128, 129, 130, 132, 135, and 139 to 215 inclusive;

Still more preferred are compounds no.: 1, 3, 4, 7, 8, 9, 10, 19, 20, 21, 22, 23, 24, 25, 116, 132, 135, 142, 147, 150, 155, 156, 157, 165, 166, 167, 170, 171, 172, 173, 174, 178, 181, 182, 183, 184, 191, 192, 196, 202, 205 and 207;

The most preferred compounds are:

3. [3-(N-2′-Quinolinecarbonyl-L-asparaginyl)amino-2-hydroxy-4-phenylbutyryl]-L-proline t-butyl ester,

4. [3-(N-3′-Quinolinecarbonyl-L-asparaginyl)amino-2-hydroxy-4-phenylbutyryl]-L-proline t-butyl ester,

8. [3-(N-2′-Benzofurancarbonyl-L-asparaginyl)amino-2-hydroxy-4-phenylbutyryl]-L-proline t-butyl ester,

9. [3-(N-2′-Indolecarbonyl-L-asparaginyl)amino-2-hydroxy-4-phenylbutyryl]-L-proline t-butyl ester,

20. N-{[3-(N-2′-Quinolinecarbonyl-L-asparaginyl)amino-2-hydroxy-4-phenylbutyryl]-L-prolyl}-N-t-butylamine,

21. N-{[3-(N-3′-Quinolinecarbonyl-L-asparaginyl)amino-2-hydroxy-4-phenylbutyryl]-L-prolyl}-N-t-butylamine,

23. N-{[3-(N-2′-Quinoxalinecarbonyl-L-asparaginyl)amino-2-hydroxy-4-phenylbutyryl]-L-prolyl}-N-t-butylamine,

116. N-{[3-(N-p-Methoxyphenoxyacetyl-L-asparaginyl)amino-2-hydroxy-4-phenylbutyryl]-L-prolyl}-N-t-butylamine,

135. N-{[3-(N-Quinoxaline-2′-carbonyl-L-asparaginyl)amino-2-hydroxy-4-phenylbutyryl]-L-prolyl}-2-methylalaninol,

142. 4-t-Butylaminocarbonyl-1-[3-(N-2′-indolecarbonyl-L-asparaginyl)amino-2-hydroxy-4-phenylbutyryl]thiazolidine,

147. 4-t-Butylaminocarbonyl-5,5-dimethyl-1-[3-(N-quinoxaline-2′-carbonyl-L-asparaginyl)amino-2-hydroxy-4-phenylbutyryl]thiazolidine,

150. 4-t-Butylaminocarbonyl-5,5-dimethyl-1-[3-(N-2′-indolecarbonyl-L-asparaginyl)amino-2-hydroxy-4-phenylbutyryl]thiazolidine,

165. 4-t-Butylaminocarbonyl-1-[3-(N-2′-indolecarbonyl-L-3-methylthioalanyl)amino-2-hydroxy-4-phenylbutyryl]-thiazolidine,

171. N-{[2-Hydroxy-3-(N-p-methylphenoxyacetyl-L-3-methanesulphonylalanyl)amino-4-phenylbutyryl]-L-prolyl}-N-t-butylamine,

182. N-{[2-Hydroxy-4-phenylbutyryl-3-(N-2′-quinolinecarbonyl-L-3-methanesulphonylalanyl)amino]-L-prolyl}-N-t-butylamine,

183. 4-t-Butylaminocarbonyl-1-[2-hydroxy-4-phenylbutyryl-3-(N-2′-quinolinecarbonyl-L-3-methanesulphonylalanyl)amino]thiazolidine,

and

196. 4-t-Butylaminocarbonyl-5,5-dimethyl-1-{3-[N-(1-naphthyloxy)acetyl-L-3-sulphamoylalanyl]amino-2-hydroxy-4-phenylbutyryl}-thiazolidine.

The compounds of the present invention can be prepared by a variety of methods, some of which may be known in the art for the preparation of compounds of this type. For example, in general terms, the compounds may be prepared by either of the following Methods A and B, which are novel methods and themselves form part of the present invention.

Method A:

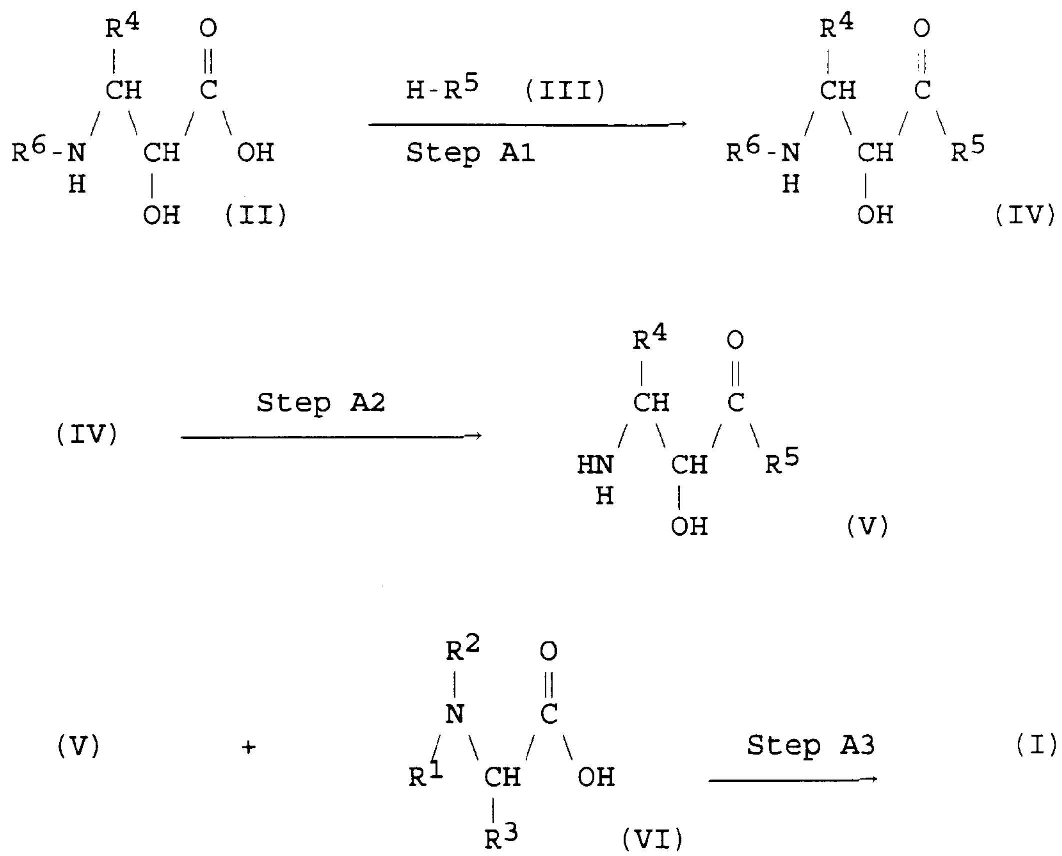


In the above formulae, $R^1$, $R^2$, $R^3$, $R^4$ and $R^5$ are as defined above and $R^6$ represents an amino protecting group. Because this protecting group does not remain in the final product, its nature is not critical, and it can be chosen, solely on the basis of its functionality in the reaction, from the wide range of such protecting groups which are known. Examples of such groups include:

aliphatic acyl groups, preferably: alkanoyl groups having from 1 to 25 carbon atoms, more preferably from 1 to 20 carbon atoms, and most preferably from 1 to 6 carbon atoms (such as the formyl, acetyl, propionyl, butyryl, isobutyryl, pivaloyl, valeryl, isovaleryl, hexanoyl, heptanoyl, octanoyl, lauroyl, myristoyl, tridecanoyl, palmitoyl and stearoyl groups); halogenated alkanoyl groups having from 2 to 6 carbon atoms, especially halogenated acetyl groups (such as the chloroacetyl, dichloroacetyl, trichloroacetyl and trifluoroacetyl groups); lower alkoxyalkanoyl groups in which the alkoxy part preferably has from 1 to 3 carbon atoms and the alkanoyl part has from 2 to 6 carbon atoms and is preferably an acetyl group (such as the methoxyacetyl group); and unsaturated analogs of such groups, especially alkenoyl or alkynoyl groups having from 3 to 6 carbon atoms [such as the acryloyl, methacryloyl, propioloyl, crotonoyl, isocrotonoyl and (E)-2-methyl-2-butenoyl groups];

aromatic acyl groups, preferably arylcarbonyl groups, in which the aryl part has from 6 to 14, more preferably from 6 to 10, and most preferably 6 or 10, ring carbon atoms and is a carbocyclic group, which is unsubstituted or has from 1 to 5, preferably from 1 to 3 substituents, preferably: unsubstituted groups (such as the benzoyl, α-naphthoyl and β-naphthoyl groups); halogenated arylcarbonyl groups (such as the 2-bromobenzoyl and 4-chlorobenzoyl groups); lower alkyl-substituted arylcarbonyl groups, in which the or each alkyl substituent preferably has from 1 to 4 carbon atoms (such as the 2,4,6-trimethylbenzoyl and 4-toluoyl groups); lower alkoxy-substituted arylcarbonyl groups, in which the or each alkoxy substituent preferably has from 1 to 4 carbon atoms (such as the 4-anisoyl group); nitro-substituted arylcarbonyl groups (such as the 4-nitrobenzoyl and 2-nitrobenzoyl groups); lower alkoxycarbonyl-substituted arylcarbonyl groups, in which the or each alkoxycarbonyl substituent preferably has from 2 to 5 carbon atoms [such as the 2-(methoxycarbonyl)benzoyl group]; and aryl-substituted arylcarbonyl groups, in which the aryl substituent is as defined above, except that, if it is substituted by a further aryl group, that aryl group is not itself substituted by an aryl group (such as the 4-phenylbenzoyl group);

alkoxycarbonyl groups, especially such groups having from 2 to 7, more preferably 2 to 5, carbon atoms and which may be unsubstituted (such as the methoxycarbonyl, ethoxycarbonyl, t-butoxycarbonyl and isobutoxycarbonyl groups) or substituted with a halogen atom or a tri-substituted silyl group, for example a tri(lower alkylsilyl) group (such as the 2,2,2-trichloroethoxycarbonyl and 2-trimethylsilylethoxycarbonyl groups);

alkenyloxycarbonyl groups in which the alkenyl part has from 2 to 6, preferably from 2 to 4, carbon atoms (such as the vinyloxycarbonyl and allyloxycarbonyl groups);

aralkyloxycarbonyl groups, in which the aralkyl part is as defined and exemplified above, and in which the aryl ring, if substituted, preferably has one or two lower alkoxy or nitro substituents (such as the benzyloxycarbonyl, 4-methoxybenzyloxycarbonyl, 3,4-dimethoxybenzyloxycarbonyl, 2-nitrobenzyloxycarbonyl and 4-nitrobenzyloxycarbonyl groups);

tri-substituted silyl groups, in which all three or two or one of the substituents are alkyl groups having from 1 to 4 carbon atoms, and none, one or two of the substituents are aryl groups, as defined above, but preferably phenyl or substituted phenyl groups, preferably: tri(lower alkyl)silyl groups (such as the trimethylsilyl, triethylsilyl, isopropyldimethylsilyl, t-butyldimethylsilyl, methyldiisopropylsilyl, methyldi-t-butylsilyl and triisopropylsilyl groups); and tri(lower alkyl)silyl groups in which one or two of the alkyl groups have been replaced by aryl groups (such as the diphenylmethylsilyl, diphenylbutylsilyl, diphenyl-t-butylsilyl, diphenylisopropylsilyl and phenyldiisopropylsilyl groups);

aralkyl groups, preferably alkyl groups having from 1 to 4, more preferably from 1 to 3 and most preferably 1 or 2, carbon atoms and which are substituted with from 1 to 3 aryl groups, as defined and exemplified above, which may be unsubstituted (such as the benzyl, α-naphthylmethyl, β-naphthylmethyl, diphenylmethyl, triphenylmethyl, α-naphthyldiphenylmethyl and 9-anthrylmethyl groups) or substituted on the aryl part with a lower alkyl group, a lower alkoxy group, a nitro group, a halogen atom, a cyano group, or an alkylenedioxy group having from 1 to 3 carbon atoms, in which the alkyl and alkoxy groups may be as defined and exemplified above and the alkylenedioxy group is preferably a methylenedioxy group, [such as the 4-methylbenzyl, 2,4,6-trimethylbenzyl, 3,4,5-trimethylbenzyl, 2-methoxybenzyl, 3-methoxybenzyl, 4-methoxybenzyl, 3,4-dimethoxybenzyl, 4-methoxyphenyl-diphenylmethyl, 2-nitrobenzyl, 4-nitrobenzyl, 2-chlorobenzyl, 3-chlorobenzyl, 4-chlorobenzyl, 4-bromobenzyl, 4-cyanobenzyl, 2-phenethyl, 1-naphthylethyl, 2-naphthylethyl, 1-phenylpropyl, 2-phenylpropyl, 3-phenylpropyl, 1-naphthylpropyl, 2-naphthylpropyl, 3-naphthylpropyl, 1-phenylbutyl, 2-phenylbutyl, 3-phenylbutyl, 4-phenylbutyl, 1-naphthylbutyl, 2-naphthylbutyl, 3-naphthylbutyl, 4-naphthylbutyl, 1-phenylpentyl, 2-phenylpentyl, 3-phenylpentyl, 4-phenylpentyl, 5-phenylpentyl, 1-naphthylpentyl, 2-naphthylpentyl, 3-naphthylpentyl, 4-naphthylpentyl, 5-naphthylpentyl, 1-phenylhexyl, 2-phenylhexyl, 3-phenylhexyl, 4-phenylhexyl, 5-phenylhexyl, 5-phenylhexyl, 1-naphthylhexyl, 2-naphthylhexyl, 3-naphthylhexyl, 4-naphthylhexyl, 5-naphthylhexyl or 6-naphthylhexyl 4-cyanobenzyldiphenylmethyl, bis(2-nitrophenyl)methyl and piperonyl groups]; and

a substituted methylene group capable of forming the corresponding Schiff base [such as N,N-dimethylaminomethylene, benzylidene, 4-methoxybenzylidene, 4-nitrobenzylidene, salicylidene, 5-chlorosalicylidene, diphenylmethylene or (5-chloro-2-hydroxyphenyl)phenylmethylene].

Of these, we prefer the alkoxycarbonyl group or the aralkyloxycarbonyl group, most preferably t-butoxycarbonyl, benzyloxycarbonyl or 4-methoxybenzyloxycarbonyl.

In step A1 of this Method, the compound (IV) is produced as a result of the formation of a peptide bond between the compounds of formula (II) and (III). The reaction involved is a standard condensation reaction of the type conventionally used in peptide synthesis and it may be carried out according to any of the well known techniques conventionally employed in peptide synthesis, for example by the azide method, the active ester method, the mixed acid anhydride method or the condensation method. The reactive derivatives employed in these reactions are those reactive derivatives conventionally employed in such methods. Certain of these methods are described in more detail below.

Azide Method

First, the carboxylic acid of formula (II) as such, or, more usually, in the form of its corresponding alkyl ester, is treated with hydrazine in an inert solvent, to give the corresponding acid hydrazide. The nature of the solvent employed in this reaction is not critical and any solvent commonly employed in this type of reaction may equally be employed here, provided that it has no adverse effect on the reaction; however, we generally find it convenient to use a polar solvent, especially a fatty acid amide, such as dimethylformamide. Also, the reaction temperature is not critical and the reaction will take place over a wide range of temperatures; we generally find it convenient to carry out the reaction at a temperature of from 0°C to about ambient temperature.

The resulting hydrazide is then reacted with a nitrite, to convert it into an azide, after which the azide is reacted with the amine of formula (III).

Examples of nitrites which may be employed include: alkali metal nitrites, such as sodium nitrite; and alkyl nitrites, such as isoamyl nitrite.

The reaction of the acid hydrazide with the nitrite and the subsequent reaction of the resulting azide with the amine of formula (III) are commonly carried out in the same reaction solution, without intermediate isolation of the azide. Both reactions are preferably carried out in the presence of an inert solvent. The nature of the solvent is not critical, provided that it does not interfere with the reaction. Suitable solvents for these reactions include, for example: amides, especially fatty acid amides, such as N,N-dimethylformamide or N,N-dimethylacetamide; sulphoxides, such as dimethyl sulphoxide; and pyrrolidones, such as N-methylpyrrolidone. Although there is no criticality as to the reaction temperature, the reaction with the nitrite is preferably effected at a relatively low temperature, for example from -50°C to 0°C, whilst the reaction of the azide with the amine is preferably effected at a temperature of from -10°C to +10°C. The time required for each of these reactions will vary, depending upon the nature of the reagents and the reaction temperature but, under the preferred conditions outlined above, a period of from 5 minutes to 1 hour and a period of from 10 hours to 5 days will normally suffice for the reaction with the nitrite and the reaction of the azide with the amine, respectively.

Active Ester Method

In this method, the carboxylic acid of formula (II) is first converted to an active ester by reacting it with a suitable reagent for producing active esters, after which this active ester is reacted with the amine of formula (III).

Formation of the active ester is preferably effected by reacting the carboxylic acid of formula (II) with, for example, an N-hydroxyimide compound, such as N-hydroxysuccinimide, 1-hydroxybenzotriazole, N-hydroxy-5-norbornene-2,3-dicarboximide, 1,1′-oxazolyldiimidazole; 2,2′-dipyridyl disulphide; N,N′-disuccinimidyl carbonate; N,N′-bis(2-oxo-3-oxazolidinyl)phosphonic chloride; N,N′-carbonyldiimidazole; N,N′-disuccinimidyl oxalate; N,N′-diphthalimide oxalate; N,N′-bis(norbornenylsuccinimidyl) oxalate; 1,1′-bis(benzotriazolyl) oxalate; 1,1′-bis(6-chlorobenzotriazolyl) oxalate; or 1,1′-bis(6-trifluoromethylbenzotriazolyl) oxalate. The reaction to form the active ester is preferably effected in the presence of a condensing agent, such as dicyclohexylcarbodiimide or carbonyldiimidazole.

The reaction to form the active ester is also preferably carried out in the presence of an inert solvent, the nature of which is not critical, provided that it has no adverse effect upon the reaction. Suitable solvents include, for example: halogenated hydrocarbons, preferably halogenated aliphatic hydrocarbons, such as methylene chloride or chloroform; ethers, such as diethyl ether or tetrahydrofuran; and amides, such as N,N-dimethylformamide or N,N-dimethylacetamide.

The reaction temperature is not critical and may vary over a wide range, for example from -10°C to +25°C. The time required for the reaction may also vary widely, depending upon many factors, such as the nature of the reagents and the reaction temperature, but a period of from 30 minutes to 10 hours will normally suffice.

Reaction of this active ester with the amine of formula (III) may be carried out with or without intermediate isolation of the active ester. Reaction of the active ester with the amine is preferably effected in the presence of an inert solvent, examples of which are as given for the preparation of the active ester itself. The temperature required for the reaction is not particularly critical and, for this reason, we normally prefer to carry out the reaction at about ambient temperature, although other reaction temperatures may also be employed with equal success. The time required for the reaction will vary widely, depending on many factors, but a period of from 30 minutes to 10 hours will generally be sufficient.

Mixed Acid Anhydride Method

In this method, the carboxylic acid of formula (II) is first converted to a mixed acid anhydride, and this is

then reacted with the amine of formula (III).

Preparation of the mixed acid anhydride is effected by reacting the acid of formula (II) with a suitable reagent, preferably in the presence of an inert solvent. Suitable reagents include: lower alkyl haloformates, such as ethyl chloroformate or isobutyl chloroformate; and di(lower alkyl)cyanophosphonates, such as diethyl cyanophosphonate. Examples of suitable inert solvents include the amides and ethers referred to in relation to the active ester method.

This reaction is preferably effected in the presence of an organic amine, such as triethylamine or N-methylmorpholine. The reaction temperature is not critical and may vary over a wide range, for example from -10°C to 25°C. The period required for the reaction may also vary widely, depending upon such factors as the nature of the reagents and the reaction temperature, but a period of from 30 minutes to 5 hours will normally suffice.

Reaction of the resulting mixed acid anhydride with the amine of formula (III) is preferably effected in the presence of an inert solvent, the nature of which is not critical, provided that it does not interfere with the reaction. Suitable solvents include the amides and ethers hereinbefore exemplified in relation to the active ester method. The reaction will take place over a wide range of temperatures, but we generally find it convenient to carry out the reaction at a temperature of from 0°C to about ambient temperature. The time required for the reaction may vary widely, depending upon many factors, such as the nature of the reagents and the reaction temperature, but a period of from 1 hour to 24 hours will normally suffice.

Condensation Method

In this method, the carboxylic acid of formula (II) is directly reacted with the amine of formula (III). Such a reaction is preferably effected in the presence of a condensing agent, such as dicyclohexylcarbodiimide or carbonyldiimidazole. Otherwise, the reaction conditions and solvents are similar to those already described in relation to the active ester method.

In step A2 of Method A, the compound of formula (V) is prepared by removal of the amino-protecting group from the compound of formula (IV). The removal of the protecting group may be effected by conventional means and the particular removal reaction chosen is not critical to the present invention and will depend upon the nature of the protecting group.

For example, where the amino-protecting group is a silyl group, this group may be removed by treatment with a compound capable of forming a fluorine anion (for example tetrabutylammonium fluoride). Such a reaction is preferably effected in an inert solvent. The nature of the solvent is not critical, provided that is has no adverse effect on the reaction, and examples of suitable solvents include ethers, such as dioxane and tetrahydrofuran. The reaction will take place over a wide range of temperatures, and the precise temperature chosen is not critical to the invention; we generally find it convenient to carry out the reaction at, for example, room temperature. The time required for the reaction may vary widely, depending upon many factors, notably the nature of the reagents and the reaction temperature. However, under the preferred conditions outlined above, a period of from 10 hours to 18 hours will normally suffice.

When the amino-protecting group is an alkoxycarbonyl group, this group may be removed by treatment with an acid (for example a mineral acid, such as hydrochloric acid or hydrofluoric acid, an organic acid, such as trifluoroacetic acid or a Lewis acid, such as boron trifluoride, preferably in the form of a complex, for example the diethyl etherate). Such a reaction is preferably carried out in an inert solvent. The nature of the solvent is not critical, provided that it has no adverse effect on the reaction. Examples of suitable solvents include: amides, such as N,N-dimethylformamide or N,N-dimethylacetamide; ethers, such as diethyl ether, diisopropyl ether, tetrahydrofuran, dioxane, dimethoxyethane or diethylene glycol dimethyl ether; or lower alcohols, such as methanol, ethanol, propanol, isopropanol, butanol, isobutanol, t-butanol, isoamylalcohol, diethylene glycol, glycerin, octanol, cyclohexanol or methyl cellosolve. The reaction will take place over a wide range of temperatures, and the precise temperature chosen is not critical to the invention. We generally find it convenient to carry out the reaction at, for example a temperature of from 0°C to 30°C. The time required for the reaction may vary widely, depending upon the reaction conditions, but a period of from 20 minutes to 1 hour will normally suffice.

When the amino-protecting group is an aliphatic acyl group, aromatic acyl group, alkenyloxycarbonyl group or a substituted methylene group capable of forming a Schiff base, this group can be removed by treatment with an acid or a base in the presence of an aqueous solvent. Acids which can be used in the reaction are inorganic acids, such as hydrochloric acid, sulphuric acid, phosphoric acid or hydrobromic acid. Any base may be used to effect the reaction, so long as the base chosen has no adverse effect on the reaction. Isomerisation may occur when a base is used for the hydrolysis. Examples of bases include metal alkoxides, such as sodium methoxide; alkali metal carbonates, such as sodium carbonate or potassium carbonate; alkali metal hydroxides, such as sodium hydroxide or potassium hydroxide; or an ammonia compound, such as aqueous ammonia or concentrated ammonia-methanol. The nature of the solvent is not critical, provided that it has no adverse effect

on the reaction, and examples of suitable solvents include: water; organic solvents, for example alcohols, such as methanol, ethanol or propanol; or ethers, such as tetrahydrofuran or dioxane; or mixtures thereof with water. We generally find it convenient to carry out the reaction at temperatures of from 0°C to 150°C, although this is not critical. The time required for the reaction may vary widely, depending upon the reaction conditions, but a period of from 1 to 10 hours will normally suffice.

When the amino-protecting group is allyloxycarbonyl, it is preferred that this group is removed by treatment similar to that described in the preceding paragraph, but employing, in addition, palladium, triphenylphosphine or nickel tetracarbonyl to avoid any unwanted side reactions.

When the amino group is protected by an aralkyloxycarbonyl group, the protecting group can be removed by catalytic reduction of the protected compound in the presence of hydrogen (for example under a hydrogen pressure of from atmospheric to 10 atmospheres) and in the presence of a suitable hydrogenation catalyst, for example palladium-on-carbon, palladium black or Raney nickel. The reaction is preferably effected in the presence of an inert solvent, the nature of which is not critical, provided that it has no adverse effect on the reaction, and examples of suitable solvents include: lower alcohols, such as methanol, ethanol, isopropanol, butanol, isobutanol, t-butanol, isoamylalcohol, diethylene glycol, glycerin, octanol, cyclohexanol or methyl cellosolve; and ethers, such as diethyl ether, diisopropyl ether, dioxane, dimethoxyethane, diethylene glycol dimethyl ether or tetrahydrofuran. We generally find it convenient to carry out the reaction at about ambient temperature, although this is not critical. The time required for the reaction may vary widely, depending upon the reaction conditions, but a period of from 1 to 8 hours will normally suffice.

When the amino group is protected by an aralkyl group, the protecting group can be removed by catalytic reduction of the protected compound (for example under a pressure of from atmospheric to 10 atmospheres) and in the presence of a suitable hydrogenation catalyst, for example palladium black, palladium-on-carbon, platinum oxide, platinum black, rhodium-aluminum oxide, triphenylphosphine-rhodium chloride, palladium-barium sulphate or Raney nickel. The reaction is preferably effected in the presence of an inert solvent, the nature of which is not critical, provided that it has no adverse effect on the reaction, and examples of suitable solvents include: lower alcohols, such as methanol, ethanol, isopropanol; ethers, such as diethyl ether, dioxane, or tetrahydrofuran; aromatic hydrocarbons, such as hexane or cyclohexane; esters, such as ethyl acetate or propyl acetate; fatty acids, such as formic acid, acetic acid; a mixture thereof with water, or a mixture of a fatty acid and an alcohol. We generally find it convenient to carry out the reaction at from 0°C to 100°C, although this is not critical. The time required for the reaction may vary widely, depending upon the reaction conditions, but a period of from 5 minutes to 24 hours will normally suffice.

If the amino-protecting group is an aralkyl group, this may otherwise be removed by treatment with an oxidizing agent (for example potassium persulphate, sodium persulphate, cerium ammonium nitrate or 2,3-dichloro-5,6-dicyano-p-benzoquinone). The reaction is preferably effected in an inert solvent, the nature of which is not critical, provided that it does not have an adverse effect on the reaction. Suitable solvents include: ketones, such as acetone; halohydrocarbons, such as methylene chloride, chloroform or carbon tetrachloride; ethers, such as diethyl ether, tetrahydrofuran or dioxane; amides, such as N,N-dimethylformamide, N,N-dimethylacetamide or hexamethylphosphoric triamide; or sulphoxides, such as dimethyl sulphoxide. The reaction will take place over a wide range of temperatures, and the precise temperature chosen is not critical to the invention. We generally find it convenient to carry out the reaction at, for example, a temperature of from 0°C to 150°C. The time required for the reaction may vary widely, depending upon many factors, notably the nature of the reagents and the reaction temperature; however, under the preferred conditions outlined above, a period of from 10 minutes to 24 hours will normally suffice.

In step A3 of Method A, the compound of formula (V), resulting from the de-protection step above, is reacted with a compound of formula (VI) to form the compound of formula (I). This reaction is also a standard condensation reaction of the type conventionally used in peptide synthesis and it may be performed by any one of the methods outlined for step A1 of Method A above, using similar reagents and conditions.

Method B

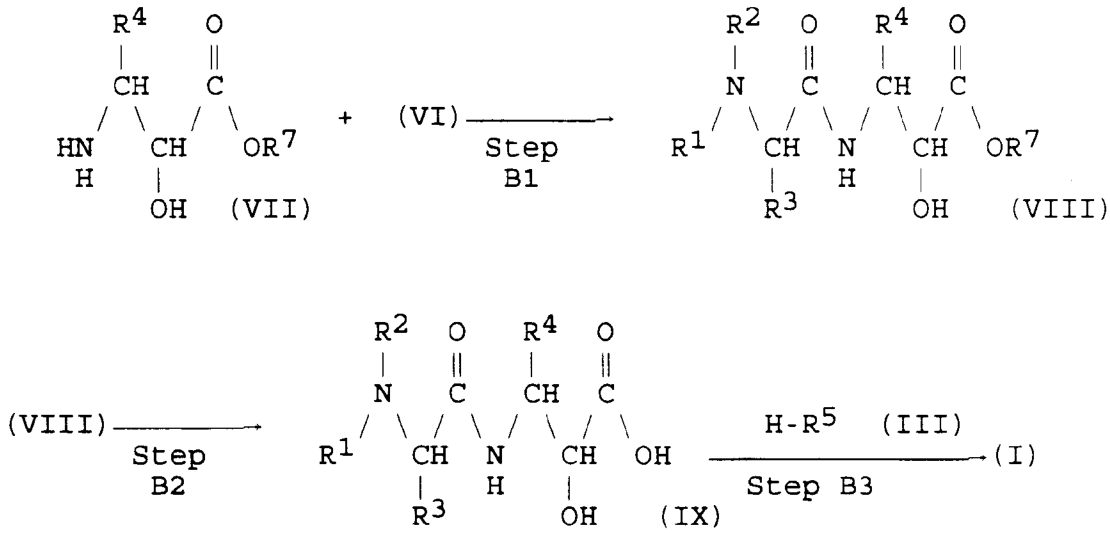


In the above formulae, $R^1$, $R^2$, $R^3$, $R^4$ and $R^5$ are as defined above and $R^7$ is a hydrogen atom or a carboxy protecting group. Because this protecting group does not remain in the final product, its nature is not critical, and it can be chosen, solely on the basis of its functionality in the reaction, from the wide range of such protecting groups which are known. Examples of such groups include:

lower alkyl groups, preferably: straight chain or branched alkyl groups having from 1 to 6 carbon atoms, for example methyl, ethyl, propyl, isopropyl, butyl, isobutyl, sec-butyl, t-butyl, pentyl, isopentyl, 2-methylbutyl, neopentyl, 1-ethylpropyl, hexyl, 4-methylpentyl, 3-methylpentyl, 2-methylpentyl, 1-methylpentyl, 3,3-dimethylbutyl, 2,2-dimethylbutyl, 1,1-dimethylbutyl, 1,2-dimethylbutyl, 1,3-dimethylbutyl, 2,3-dimethylbutyl or 2-ethylbutyl; preferably a straight or branched chain alkyl group having from 1 to 4 carbon atoms;

haloalkyl groups, preferably in which the alkyl part of the group is straight chain or branched and has from 1 to 6 carbon atoms, for example trifluoromethyl, trichloromethyl, tribromomethyl, difluoromethyl, dichloromethyl, dibromomethyl, fluoromethyl, 2,2,2-trichloroethyl, 2,2,2-trifluoroethyl, 2-bromoethyl, 2-chloroethyl, 2-fluoroethyl or 2,2-dibromoethyl; and

aralkyl groups, preferably alkyl groups having from 1 to 4, more preferably from 1 to 3 and most preferably 1 or 2, carbon atoms which are substituted with from 1 to 3 aryl groups, as defined and exemplified above, which may be unsubstituted (such as the benzyl, α-naphthylmethyl, β-naphthylmethyl, diphenylmethyl, triphenylmethyl, α-naphthyldiphenylmethyl and 9-anthrylmethyl groups) or substituted on the aryl part with a lower alkyl group, a lower alkoxy group, a nitro group, a halogen atom, a cyano group, or an alkylenedioxy group having from 1 to 3 carbon atoms, in which the alkyl and alkoxy groups may be as defined and exemplified above and the alkylenedioxy group is preferably a methylenedioxy group [such as the 4-methylbenzyl, 2,4,6-trimethylbenzyl, 3,4,5-trimethylbenzyl, 2-methoxybenzyl, 3-methoxybenzyl, 4-methoxybenzyl, 3,4-dimethoxybenzyl, 4-methoxyphenyl-diphenylmethyl, 2-nitrobenzyl, 4-nitrobenzyl, 2-chlorobenzyl, 3-chlorobenzyl, 4-chlorobenzyl, 4-bromobenzyl, 4-cyanobenzyl, 2-phenethyl, 1-naphthylethyl, 2-naphthylethyl, 1-phenylpropyl, 2-phenylpropyl, 3-phenylpropyl, 1-naphthylpropyl, 2-naphthylpropyl, 3-naphthylpropyl, 1-phenylbutyl, 2-phenylbutyl, 3-phenylbutyl, 4-phenylbutyl, 1-naphthylbutyl, 2-naphthylbutyl, 3-naphthylbutyl, 4-naphthylbutyl, 1-phenylpentyl, 2-phenylpentyl, 3-phenylpentyl, 4-phenylpentyl, 5-phenylpentyl, 1-naphthylpentyl, 2-naphthylpentyl, 3-naphthylpentyl, 4-naphthylpentyl, 5-naphthylpentyl, 1-phenylhexyl, 2-phenylhexyl, 3-phenylhexyl, 4-phenylhexyl, 5-phenylhexyl, 5-phenylhexyl, 1-naphthylhexyl, 2-naphthylhexyl, 3-naphthylhexyl, 4-naphthylhexyl, 5-naphthylhexyl or 6-naphthylhexyl 4-cyanobenzyldiphenylmethyl, bis(2-nitrophenyl)methyl and piperonyl groups].

Of these we prefer the lower alkyl group or the aralkyl group.

In step B1 of this Method, a compound of formula (VII) is allowed to react with a compound of formula (VI) to form the compound of formula (VIII). This reaction is also a standard condensation reaction of the type conventionally used in peptide synthesis and it may be performed by any one of the methods outlined for step A1 of Method A above, using similar reagents and conditions.

In step B2 of Method B, when $R^7$ is a carboxy-protecting group, this protecting group is removed from compound of formula (VIII). The removal of the protecting group may be effected by conventional means and the

particular removal reaction chosen will depend upon the nature of the protecting group and is not critical to the present invention.

When the carboxy-protecting group is a lower alkyl group, this may be removed by treatment with an acid (for example an organic acid, such as hydrochloric acid, sulphuric acid, phosphoric acid or hydrobromic acid) or with a base (for example an alkali metal carbonate, such as sodium carbonate or potassium carbonate; an alkali metal hydroxide, such as sodium hydroxide or potassium hydroxide; or concentrated ammonia-methanol solution). The reaction is normally effected in the presence of an inert solvent. The nature of the solvent is not critical, provided that it does not have an adverse effect on the reaction. Suitable solvents include water or an organic solvent, such as methanol, ethanol or propanol; or an ether, such as tetrahydrofuran or dioxane, mixed with water. Although there is no criticality as to the reaction temperature, the reaction is preferably carried out at temperatures from 0°C to 150°C in order to avoid unwanted side reactions. The time required for the reaction will vary, depending upon the nature of the reagents and the reaction conditions. Under the preferred conditions outlined above, a period of from 1 to 10 hours will generally be sufficient.

When the carboxy-protecting group is an aralkyl group, such as diphenylmethyl, this can be removed by treatment with an acid (for example a fluorinated organic acid, such as trifluoroacetic acid) in the presence of a solvent. The nature of the solvent is not critical, provided that it does not have an adverse effect on the reaction. Solvents which are suitable include aromatic hydrocarbons, such as anisole. The temperature at which the reaction is carried out is not critical and the reaction is suitably carried out at room temperature. The time required for the reaction will vary, depending upon the reagents and conditions used. Generally, however, from 30 minutes to 10 hours will suffice.

When the group protecting the carboxy group is an aralkyl or haloalkyl group, this group can be removed by catalytic reduction in the presence of a solvent. Catalysts suitable for use when the carboxy-protecting group is haloalkyl include zinc-acetic acid. When the carboxy-protecting group is an aralkyl group, suitable catalysts include palladium-on-carbon or platinum, or an alkali metal sulphide such as potassium sulphide or sodium sulphide. The nature of the solvent is not critical, provided that it does not have an adverse effect on the reaction. Suitable solvents include alcohols, such as methanol or ethanol; ethers, such as tetrahydrofuran or dioxane; fatty acids, such as acetic acid; or a mixture of such solvents in water. The reaction temperature will vary widely and is not critical to the invention; the reaction is suitably carried out at temperatures from 0°C to room temperature. The time required for the reaction will also vary, and is dependent upon the nature of the reagents. Under the preferred conditions outlined above, a period of from 5 minutes to 12 hours will normally suffice.

The step B3 of Method B involves the reaction of the compound of formula (IX) with a compound of formula (III), as defined in Method A. This reaction is the formation of a peptide bond between the two compounds, and it can be effected using procedures conventional in peptide synthesis. The reaction may be performed by any one of the methods outlined for step A1 of Method A above, using similar reagents and conditions.

Amino groups comprising any of the substituents $R^1$, $R^3$, $R^4$ or $R^5$ may be protected through the reactions. The amino-protecting groups mentioned above are suitable to provide protection. When any of these amino groups is protected in, for example, the compound of formula (VI), the protecting group can be removed from the final compound of formula (I) after completion of reaction steps A3 and B3.

The compounds of formula (I) can, if desired, be converted into pharmaceutically acceptable salts thereof or into pharmaceutically acceptable esters thereof by conventional means. For example, for the formation of a pharmaceutically acceptable salt, the compound of formula (I) may be dissolved in an organic solvent, such as ethyl acetate or methylene chloride, and an equimolar amount or an excess of an acid, such as hydrogen chloride - dioxane, may be added. The solvent may then be distilled off, and the resulting compound of formula (I) may be obtained in the form of a salt by crystallization or solidification in an organic solvent such as diethyl ether or diisopropyl ether.

After completion of any of the above reactions or of the final such reaction, the desired compound may be isolated from the reaction mixture by conventional means. For example, one suitable recovery procedure comprises: if necessary, neutralizing the reaction mixture; removing the insoluble residue, if any, by filtration; and then distilling off the solvent to give the desired compound. If necessary, the resulting compound may be further purified by such conventional means as recrystallization, reprecipitation or the various chromatography techniques, such as column chromatography or preparative thin layer chromatography.

Preparation of Starting Materials

The compounds of formulae (II), (III), (VI) and (VII) are known, or can be prepared without any difficulty according to known methods [for example, as described in Synthesis, 703-706, (1989) and Tetrahedron Letters, 29, 3295-3298 (1988)].

The compounds of the present invention have shown excellent activity in the inhibition of protease derived

from human immunodeficiency virus, and they also demonstrate good specificity for the enzyme. They are, accordingly, useful as agents for the treatment of acquired immunodeficiency syndrome (AIDS).

The compounds of the invention may be administered orally or parenterally as required and may, if desired, be formulated into appropriate pharmaceutical formulations, depending upon the desired route of administration. For example, for oral administration, the compounds may be formulated as tablets, capsules, granules, powders or syrups. For parenteral administration, they may be formulated as injectible solutions or suspensions or as suppositories. These pharmaceutical preparations can be produced by conventional means using adjuvants generally known in this field, such as excipients, diluents, dispersants, binders, disintegrators, lubricants, stabilizers, corrigents and the like.

The dosage and frequency of administration may vary depending upon the symptoms, age and body weight of the patient, as well as upon the route of administration, but, in general, the compounds of the invention may be administered orally in a daily dose of from 0.1 to 100 mg for an adult, which may be administered either as single dose or as divided doses.

The present invention will be further illustrated in the following Examples. These examples do not limit the scope of the present invention in any way. Test Examples 1 and 2 illustrate the activity of the compounds of the invention in various assays.

EXAMPLE 1

(2S,3S)-[3-(N-Benzyloxycarbonyl-L-asparaginyl)-amino-2-hydroxy-4-phenylbutyryl]-L-proline t-butyl ester (Compound No. 1)

(1a) (2S,3S)-(3-Benzyloxycarbonylamino-2-hydroxy-4-phenylbutyryl)-L-proline t-butyl ester

105 mg (0.61mmol) of proline t-butyl ester and 183 mg (0.56 mmol) of (2S,3S)-3-benzyloxycarbonylamino-2-hydroxy-4-phenylbutyric acid [prepared according to the method of R. Herranz, J. Castro-Pichel & T. Garcia-Lopez Synthesis (1989) 703-706] were dissolved in 5 ml of N,N-dimethylformamide and cooled on an ice bath. 107 mg (0.61 mmol) of diethyl cyanophosphonate were added, and subsequently 62 mg (0.61 mmol) of triethylamine were added dropwise. The mixture was then stirred for 3 hours. At the end of this time, the reaction mixture was condensed by evaporation under reduced pressure. 1N aqueous sulphuric acid was added to the residue, and the oily substance which separated out was extracted with ethyl acetate. The organic layer was washed with water, with a 5% w/v aqueous solution of sodium hydrogencarbonate and with a saturated aqueous solution of sodium chloride, and then dried over anhydrous sodium sulphate. The solvent was distilled off under reduced pressure to give 260 mg of the title compound as a colourless syrupy substance.
Mass spectrum: 483 ($M^+$).

(1b) (2S,3S)-[3-(N-benzyloxycarbonyl-L-asparaginyl)-amino-2-hydroxy-4-phenylbutyryl]-L-proline t-butyl ester

260 mg (0.54 mmol) of (2S,3S)-(3-benzyloxycarbonylamino-2-hydroxy-4-phenylbutyryl)-L-proline t-butyl ester, as formed in step (1a), were dissolved in 20 ml of ethanol. 0.6 ml of 1N aqueous hydrochloric acid and 60 mg of 10% w/w palladium-on-carbon were added to the mixture and then hydrogen gas was bubbled through the mixture. After 5 hours, the catalyst was removed by filtration and the filtrate was condensed to dryness by evaporation under reduced pressure. The whole of the resulting t-butyl ester of (2S,3S)-(3-amino-2-hydroxy4-phenylbutyryl)-L-proline hydrochloride and 217 mg (0.56 mmol) of N-benzyloxycarbonyl-L-asparagine p-nitrophenyl ester were dissolved in 5 ml of N,N-dimethylformamide. 63 mg (0.62 mmol) of triethylamine were added to the reaction mixture on an ice bath, and the resulting mixture was stirred for 5 hours at room temperature and subsequently allowed to stand overnight at the same temperature. The reaction solution was then condensed by evaporation under reduced pressure. 1N aqueous sulphuric acid was added to the residue and the oily substance which separated out was extracted with ethyl acetate. The organic extract was washed with a 10% w/v aqueous solution of sodium carbonate and with a saturated aqueous solution of sodium chloride, in that order, and then dried over anhydrous sodium sulphate. The solvent was distilled off under reduced pressure. Diethyl ether was added to the residue, and 215 mg of the title compound were obtained as colourless powdery crystals, melting at 109-112°C.

Elemental analysis:

Calculated for $C_{31}H_{40}N_4O_8.1/2H_2O$

C, 61.47%; H, 6.82%; N, 9.25%.

Found: C, 61.25%; H, 6.62%; N, 9.32%.

Molecular Weight 605.7.

EXAMPLE 2

(2R,3S)-[3-(N-Benzyloxycarbonyl-L-asparaginyl)-amino-2-hydroxy-4-phenylbutyryl]-L-proline t-butyl ester (Compound No. 1)

Following the procedure described in step (1b) of Example 1, (2R,3S)-(3-benzyloxycarbonylamino-2-hydroxy-4-phenylbutyryl)-L-proline t-butyl ester was obtained from proline t-butyl ester and (2S,3S)-3-benzyloxycarbonylamino-2-hydroxy-4-phenylbutyric acid. Subsequently, the procedure of step (b) of Example 1 was followed, but using 171 mg (0.52 mmol) of (2R,3S)-(3-benzyloxycarbonylamino-2-hydroxy-4-phenylbutyryl)-L-proline t-butyl ester as the starting material, to obtain 50 mg of the title compound as colourless powdery crystals, melting at 188-193°C.

Elemental analysis:

Calculated for $C_{31}H_{40}N_4O_8.1/2H_2O$

C, 61.47%; H, 6.82%; N, 9.25%.

Found: C, 61.74%; H, 6.56%; N, 9.46%.

Molecular Weight 605.7.

EXAMPLE 3

(2S,3S)-[3-(N-2'-Quinolinecarbonyl-L-asparaginyl)-amino-2-hydroxy-4-phenylbutyryl]-L-proline t-butyl ester (Compound No. 3)

(3a) t-Butyl ester of (2S,3S)-(3-L-asparaginylamino-2-hydroxy-4-phenylbutyryl)-L-proline hydrochloride

215 mg (0.35 mmol) of (2S,3S)-[3-(N-benzyloxycarbonyl-L-asparaginyl)amino-2-hydroxy-4-phenylbutyryl]-L-proline t-butyl ester, prepared as described in Example 1, were dissolved in 10 ml ethanol. 0.4 ml of IN aqueous hydrochloric acid and 50 mg of 10% w/w palladium-on-carbon were added to the solution. Hydrogen gas was then bubbled through the solution for 3 hours to remove the benzyloxycarbonyl group. After this time, the catalyst was removed by filtration. The filtrate was then condensed to dryness by evaporation under reduced pressure, to obtain 179 mg of the t-butyl ester of (2S,3S)-(3-L-asparaginylamino-2-hydroxy-4-phenylbutyryl)-L-proline hydrochloride as a solid residue.

(3b) (2S,3S)-[3-(N-2'-Quinolinecarbonyl-L-asparaginyl)-amino-2-hydroxy-4-phenylbutyryl]-L-proline t-butyl ester

90 mg (0.18 mmol) of the t-butyl ester of (2S,3S)-(3-L-asparaginylamino-2-hydroxy-4-phenylbutyryl)-L-proline hydrochloride, prepared as described in step (a) above, and 33 mg (0.19 mmol) of quinaldic acid were dissolved in 3 ml of N,N-dimethylformamide. The mixture was placed on an ice bath and 35 mg (0.2 mmol) of diethyl cyanophosphonate and 38 mg (0.38 mmol) of triethylamine were added to the mixture. The mixture was then stirred for 3 hours. At the end of this time, the reaction mixture was condensed by evaporation under reduced pressure. A 10% w/v aqueous solution of sodium carbonate was added to the residue, and the yellow precipitate which separated out was extracted with ethyl acetate. The organic layer was washed with water and with a saturated sodium chloride solution before drying over anhydrous sodium sulphate, and then the solvent was distilled off under reduced pressure. The yellow residue was purified by preparative thin layer chromatography, using a 10:1 by volume mixture of methylene chloride and methanol as the developing solvent, to obtain 98

mg of the title compound as a colourless powder, melting at 109-112°C.

Elemental analysis:

Calculated for $C_{33}H_{39}N_5O_7.H_2O$

C, 62.35%; H, 6.50%; N, 11.02%.

Found: C, 62.29%; H, 6.50%; N, 10.60%.

Molecular Weight 635.72.
Mass spectrum: 617 ($M^+$).

EXAMPLE 4

(2S,3S)-[3-(N-2'-Nahthoyl-L-asparaginyl)-amino-2-hydroxy-4-phenylbutyryl]-L-proline t-butyl ester (Compound No. 7)

Following the procedure of step (b) of Example 3, but using 89 mg (0.18 mmol) of (2S,3S)-(3-L-asparaginylamino-2-hydroxy-4-phenylbutyryl)-L-proline t-butyl ester hydrochloride [as prepared in step (a) of Example 3] and 33 mg (0.19 mmol) of 2-naphthoic acid, 90 mg of the title compound were obtained as a colourless powder, melting at 134-140°C.

Elemental analysis:

Calculated for $C_{34}H_{40}N_4O_7.H_2O$

C, 64.34%; H, 6.67%; N, 8.83%.

Found: C, 64.53%; H, 6.47%; N, 8.84%.

Molecular Weight 634.74.
Mass spectrum: 616 ($M^+$).

EXAMPLE 5

(2S,3S)-[3-(N-2'-Quinolinecarbonyl-L-asparaginyl)-amino-2-hydroxy-4-phenylbutyryl]-L-pipecolic acid t-butyl ester (Compound No. 58)

(5a) (2S,3S)-(3-Benzyloxycarbonylamino-2-hydroxy-4-phenylbutyryl)-L-pipecolic acid t-butyl ester

329 mg (1 mmol) of (2S,3S)-3-benzyloxycarbonylamino-2-hydroxy-4-phenylbutyric acid and 222 mg (1 mmol) of the t-butyl ester of L-pipecolic acid hydrochloride were dissolved in 10 ml of N,N-dimethylformamide. The mixture was placed on an ice bath, and 210 mg (1.2 mmol) of diethyl cyanophosphonate and 223 mg (2.2 mmol) of triethylamine were added to the mixture, which was then stirred for 3 hours. The procedure described in step (a) of Example 1 was then followed, and 230 mg of the title compound were obtained as a colourless syrup.
Mass spectrum: 497 ($M^+$).

(5b) (2S,3S)-[3-(N-Benzyloxycarbonyl-L-asparaginyl)-amino-2-hydroxy-4-phenylbutyryl]-L-pipecolic acid t-butyl ester

All of the (2S,3S)-(3-benzyloxycarbonylamino-2-hydroxy-4-phenylbutyryl)-L-pipecolic acid t-butyl ester, obtained as described in step (a) above, was dissolved in 10 ml ethanol. 0.46 ml of 1N aqueous hydrochloric acid and 50 mg of 10% w/w palladium-on-carbon were added to the mixture, and then hydrogen gas was bubbled through it. After 5 hours the catalyst was removed by filtration. The filtrate was condensed by evaporation under reduced pressure. The t-butyl ester of (2S,3S)-(3-amino-2-hydroxy-4-phenylbutyryl)-L-pipecolic acid hydrochloride thus obtained, and 267 mg (0.69 mmol) of N-benzyloxycarbonyl-L-asparagine p-nitrophenyl ester were dissolved in 5 ml of N,N-dimethylformamide. The mixture was placed on an ice bath, and 50 mg (0.5 mmol) of triethylamine were added. The procedure described in step (b) of Example 1 was then followed to give 60

mg of the title compound as a colourless powder.

(5c) (2S,3S)-[3-(N-2′-Quinolinecarbonyl-L-asparaginyl)-amino-2-hydroxy-4-phenylbutyryl]-L-pipecolic acid t-butyl ester

50 mg (0.08 mmol) of (2S,3S)-[3-(N-benzyloxycarbonyl-L-asparaginyl)amino-2-hydroxy-4-phenylbutyryl]-L-pipecolic acid t-butyl ester, prepared as described in step (b) above, were dissolved in 5 ml ethanol. 0.08 ml of 1N aqueous hydrochloric acid and 10 mg of 10% w/w palladium-on-carbon were added to the mixture, and then hydrogen gas was bubbled through the mixture for 5 hours to remove the benzyloxycarbonyl group. The catalyst was removed by filtration and the filtrate condensed by evaporation under reduced pressure. The solid residue thus obtained and 20 mg (0.12 mmol) of quinaldic acid were dissolved in 3 ml of N,N-dimethylformamide. The mixture was placed on an ice bath, and 20 mg (0.12 mmol) of diethyl cyanophosphonate and 40 mg (0.40 mmol) of triethylamine were added to the mixture, which was then stirred for 3 hours. After this time, the procedure described in step (b) of Example 3, above, was followed and 29 mg of the title compound were obtained as a colourless powder, melting at 86-88°C.

Elemental analysis:

Calculated for $C_{34}H_{41}N_5O_7.3\ 1/2H_2O$
C, 58.77%; H, 6.96%; N, 10.08%.

Found: C, 58.83%; H, 6.62%; N, 8.95%.

Molecular Weight 694.764.

EXAMPLE 6

N-{(2S,3S)-[3-(N-2′-Quinolinecarbonyl-L-valyl)-amino-2-hydroxy-4-phenylbutyryl]-L-prolyl}-N-(2-morpholinoethyl)amine (Compound No. 69)

(6a) N-[(2S,3S)-(3-Benzyloxycarbonylamino-2-hydroxy-4-phenylbutyryl)-L-prolyl]-N-(2-morpholinoethyl)amine

Following the procedure described in step (a) of Example 1, but using 100 mg (0.30 mmol) of (2S,3S)-3-benzyloxycarbonylamino-2-hydroxy-4-phenylbutyric acid and 99 mg (0.33 mmol) of L-prolyl-N-(2-morpholinoethyl) amine hydrochloride as starting materials, the title compound was obtained in a yield of 100 mg. Mass spectrum: 538 ($M^+$).

(6b) N-{(2S,3S)-[3-(N-t-Butoxycarbonyl-L-valyl)amino-2-hydroxy-4-phenylbutyryl]-L-prolyl}-(2-morpholinoethyl)amine

Following the procedure described in step (b) of Example 1 for removal of the protecting group, 100 mg (0.186 mmol) of N-[(2S,3S)-(3-benzyloxycarbonylamino-2-hydroxy-4-phenylbutyryl)-L-prolyl]-(2-morpholinoethyl)-amine, prepared as described in step (a) above, were converted into N-[(2S,3S)-(3-amino-2-hydroxy-4-phenylbutyryl)-L-prolyl]-(2-morpholinoethyl)amine hydrochloride. This compound and 48 mg (0.22 mmol) of N-t-butoxycarbonyl-L-valine were dissolved in 5 ml of methylene chloride. The mixture was placed on an ice bath, and 36 mg (0.22 mmol) of diethyl cyanophosphonate were added to the mixture, followed by 111 mg (1.1 mmol) of triethylamine. The mixture was then stirred for 5 hours at room temperature. After this time, the reaction mixture was washed with a 10% w/v aqueous solution of citric acid, with a 10% w/v aqueous solution of sodium hydrogencarbonate and with a saturated solution of sodium chloride and then dried over anhydrous sodium sulphate. The solvent was distilled off under reduced pressure. The residue obtained was purified by preparative thin layer chromatography, using a 10:1 by volume mixture of methylene chloride and methanol as developing solvent, to give 80 mg of the title compound as a colourless powder, melting at 202-204°C.

Elemental analysis:

Calculated for $C_{31}H_{49}N_5O_7.1/2H_2O$

C, 60.76%; H, 8.22%; N, 11.43%.

Found: C, 60.70%; H, 8.08%; N, 11.41%.

Molecular Weight 612.78.
Mass spectrum: 603 ($M^+$).

(6c) N-{(2S,3S)-[3-(N-2′-Quinolinecarbonyl-L-valyl)-amino-2-hydroxy-4-phenylbutyryl]-L-prolyl}-N-(2-morpholinoethyl)amine

40 mg (0.066 mmol) of N-{(2S,3S)-[3-(N-butoxycarbonyl-L-valy)amino-2-hydroxy-4-phenylbutyryl]-L-prolyl}-(2-morpholinoethyl)amine, prepared as described in step (b) above, were dissolved in 5 ml of a 4N solution of hydrogen chloride in dioxane, and the solution was stirred for 30 minutes. The reaction solution was condensed by evaporation under reduced pressure and then 14 mg (0.080 mmol) of quinaldic acid were added to the solution. 5 ml of methylene chloride were then added to the solution to form a suspension. The mixture was then placed on an ice bath, and 13 mg (0.080 mmol) of diethyl cyanophosphonate and 40 mg (0.40 mmol) of triethylamine were added to the mixture. The resulting mixture was stirred for 18 hours at room temperature, after which time the mixture was washed with a 10% w/v aqueous solution of citric acid, with a 10% w/v aqueous solution of sodium hydrogencarbonate and with a saturated solution of sodium chloride; and dried over anhydrous sodium sulphate. The solvent was condensed by evaporation under reduced pressure. After purification by preparative thin layer chromatography using a 10:1 by volume mixture of methylene chloride and methanol as developing solvent, 25 mg of the title compound were obtained as a colourless powder, melting at 98-103°C.

Elemental analysis:

Calculated for $C_{36}H_{46}N_6O_6.H_2O$

C, 63.89%; H, 7.15%; N, 12.42%.

Found: C, 64.15%; H, 6.90%; N, 12.10%.

Molecular Weight 676.83.
Mass spectrum: 658 ($M^+$).

## EXAMPLE 7

N-{(2S,3S)-[3-(N-2′-Quinolinecarbonyl-L-t-leucyl)-amino-2-hydroxy-4-phenylbutyryl]-L-prolyl}-N-[(2S)-2-methylbutyl]amine (Compound No. 72)

(7a) N-{(2S,3S)-(3-Benzyloxycarbonylamino-2-hydroxy-4-phenylbutyryl)-L-prolyl}-N-[(2S)-2-methylbutyl]amine

Following the procedure described in step (a) of Example 1 above, but using 100 mg of (2S,3S)-3-benzyloxycarbonylamino-2-hydroxy-4-phenylbutyric acid and 72.6 mg (0.33 mmol) of L-prolyl-N-(2-morpholinoethyl)amine hydrochloride as the starting materials, 100 mg of the title compound were obtained.
Mass spectrum: 495 ($M^+$).

(7b) N-{(2S,3S)-[3-(N-t-Butoxycarbonyl-L-t-leucyl)-amino-2-hydroxy-4-phenylbutyryl]-L-prolyl}-N-[(2S)-2-methylbutyl]amine

Following the procedure described in step (b) of Example 1 above, the protecting group was removed from 100 mg (0.20 mmol) of the N-{(2S,3S)-(3-benzyloxycarbonylamino-2-hydroxy-4-phenylbutyryl)-L-prolyl}-N-[(2S)-2-methylbutyl]amine, obtained in step (a) above, to give N-{(2S,3S)-[3-(N-t-butoxycarbonyl-L-t-leucyl)amino-2-hydroxy-4-phenylbutyryl]-L-prolyl}-N-[(2S)-2-methylbutyl]amine. This was then treated as described in step (b) of Example 6 with 55 mg (0.238 mmol) of N-t-butoxycarbonyl-L-t-leucine, to give 100 mg of the title compound, melting at 240-242°C.

Elemental analysis:

Calculated for $C_{31}H_{50}N_4O_6$

C, 64.78%; H, 8.77%; N, 9.75%.

Found: C, 64.92%; H, 8.87%; N, 9.61%.

Molecular Weight 574.77.
Mass spectrum: 575 $(M + H)^+$.

(7c) N-{(2S,3S)-[3-(N-2′-Quinolinecarbonyl-L-t-leucyl)amino-2-hydroxy-4-phenylbutyryl]-L-prolyl}-[(2S)-2-methylbutyl]amine

Following the procedure described in step (c) of Example 6, but using 50 mg (0.087 mmol) of N-{(2S,3S)-[3-(N-t-butoxycarbonyl-L-t-leucyl)-amino-2-hydroxy-4-phenylbutyryl]-L-prolyl}-N-[(2S)-2-methylbutyl]amine, prepared as described in step (b) above, and 18 mg (0.10 mmol) of quinaldic acid, 53 mg of the title compound were obtained as a colourless powder, melting at 106-109°C.

Elemental analysis:

Calculated for $C_{36}H_{47}N_5O_5.H_2O$

C, 66.75%; H, 7.62%; N, 10.81%.

Found: C, 66.58%; H, 7.34%; N, 10.53%.

Molecular Weight 647.83.
Mass spectrum: 629 $(M^+)$.

EXAMPLE 8

(2S,3S)-[3-(N-2′-Benzofurancarbonyl-L-asparaginyl)-amino-2-hydroxy-4-phenylbutyryl]-L-proline t-butyl ester (Compound No. 8)

Following the procedure of step (b) of Example 3, but using 60 mg (0.12 mmol) of the t-butyl ester of (2S,3S)-(3-L-asparaginyl-amino-2-hydroxy-4-phenylbutyryl)-L-proline hydrochloride [obtained as described in step (a) of Example 3] and 20 mg (0.14 mmol) of coumaric acid as starting materials, the title compound was obtained in a yield of 65 mg as a colourless powder, melting at 114-116°C.

Elemental analysis:

Calculated for $C_{32}H_{38}N_4O_8.H_2O$

C, 61.52%; H, 6.45%; N, 8.97%.

Found: C, 61.53%; H, 6.15%; N, 8.85%.

Molecular Weight 624.67.

EXAMPLE 9

(2S,3S)-[3-(N-2′-Indolecarbonyl-L-asparaginyl)-amino-2-hydroxy-4-phenylbutyryl]-L-proline t-butyl ester (Compound No. 9)

Following the procedure described in step (b) of Example 3, but using 60 mg (0.12 mmol) of the t-butyl ester of (2S,3S)-(3-L-asparaginylamino-2-hydroxy-4-phenylbutyryl)-L-proline hydrochloride [prepared as described in step (a) of Example 3] and 20 mg (0.14 mmol) of indole-2-carboxylic acid, 13 mg of the title compound were obtained as a colourless powder, melting at 140-143°C.

Elemental analysis:

Calculated for $C_{32}H_{39}N_5O_7$.3 1/2 $H_2O$

C, 57.47%; H, 6.93%; N, 10.47%.

Found: C, 57.23%; H, 6.27%; N, 10.06%

Molecular Weight 668.728.

EXAMPLE 10

N-{(2S,3S)-[3-(N-Benzyloxycarbonyl-L-asparaginyl)-amino-4-cyclohexyl-2-hydroxybutyryl]-L-prolyl}-N-t-butylamine (Compound No. 86)

(10a) N-[(2S,3S)-(3-t-Butoxycarbonylamino-4-cyclohexyl-2-hydroxybutyryl)-L-prolyl]-N-t-butylamine

200 mg (1.00 mmol) of (2S,3S)-3-amino-4-cyclohexyl-2-hydroxybutyric acid, described in the literature [Harada et al., Chem. Pharm. Bull. 37, 2570 (1989)], and 84 mg (1.00 mmol) of sodium hydrogencarbonate were dissolved in 10 ml of 1:1 v/v mixture of dioxane and water, and the solution placed on an ice bath for 0.5 hours. After this time, 218 mg (1.00 mmol) of di-t-butyl dicarbonate were added to the solution, and the resulting mixture was stirred at room temperature for 14 hours. At the end of this time, the reaction mixture was condensed under reduced pressure, and a 5% w/v aqueous solution of citric acid was added to the residue. The oily substance which separated out was extracted with ethyl acetate. The resulting organic layer was washed with a saturated aqueous solution of sodium chloride, and then dried over anhydrous sodium sulphate. The solvent was distilled off under reduced pressure to give 270 mg of (2S,3S)-3-t-butoxycarbonylamino-4-cyclohexyl-2-hydroxybutyric acid as a colourless amorphous substance.

160 mg (0.53 mmol) of (2S,3S)-3-t-butoxycarbonylamino-4-cyclohexyl-2-hydroxybutyric acid and 143 mg (0.69 mmol) of N-prolyl-N-t-butylamine hydrochloride were dissolved in 2 ml of N,N-dimethylformamide. The mixture was placed on an ice bath, and 102 mg (0.58 mmol) of diethyl cyanophosphonate were added to the mixture. 129 mg (1.27 mmol) of triethylamine were then added dropwise, and the mixture was stirred for 7 hours. After this time, the reaction mixture was condensed by evaporation under reduced pressure. Ethyl acetate was then added to the residue. The organic layer was washed with a 5% w/v aqueous solution of citric acid, with a 5% w/v aqueous solution of sodium hydrogencarbonate and with a saturated aqueous solution of sodium chloride solution, and dried over anhydrous sodium sulphate. The solvent was distilled off under reduced pressure. The compound obtained was purified by silica gel column chromatography using a 30:1 by volume mixture of methylene chloride and methanol as the eluent, to give 170 mg of the title compound as a colourless powder, melting at 69-71°C.

Elemental analysis:

Calculated for $C_{24}H_{43}N_3O_5$.1/4$H_2O$

C, 62.92%; H, 9.57%; N, 9.17%.

Found: C, 62.76%; H, 9.49%; N, 9.04%.

Molecular Weight 458.11.

(10b) N-{(2S,3S)-[3-(N-Benzyloxycarbonyl-L-asparaginyl) amino-4-cyclohexyl-2-hydroxybutyryl]-L-prolyl}-N-t-butylamine

120 mg (0.27 mmol) of N-[(2S,3S)-(3-t-butoxycarbonylamino-4-cyclohexyl-2-hydroxybutyryl)-L-prolyl]-N-t-butylamine, obtained as described in step (a) above, were dissolved in 3 ml of a 4N solution of hydrogen chloride in dioxane. The solution was allowed to stand for 20 minutes at room temperature, and then condensed by evaporation under reduced pressure. The whole of the compound obtained, N-{(2S,3S)-(3-amino-4-cyclohexyl-2-hydroxybutyryl)-L-prolyl}-N-t-butylamine hydrochloride, and 154 mg (0.40 mmol) of N-benzyloxycarbonyl-L-asparagine p-nitrophenyl ester were dissolved in 2 ml of N,N-dimethylformamide. The resulting mixture was placed on an ice bath, 67 mg (0.66 mmol) of triethylamine were added, and the mixture was stirred for 15 hours

at room temperature. After this time, the mixture was condensed by evaporation under reduced pressure. Ethyl acetate was added to the residue. The organic layer was washed with a 5% w/v aqueous solution of sodium hydrogencarbonate, with 1N aqueous hydrochloric acid and with a saturated solution of sodium chloride, in that order, and dried over anhydrous sodium sulphate. The solvent was distilled off under reduced pressure. Addition of diethyl ether to the residue afforded 116 mg of the title compound as a colourless powder, melting at 112-114°C.

Elemental analysis:

Calculated for $C_{31}H_{47}N_5O_7 \cdot 1\ 1/2H_2O$

C, 59.21%; H, 8.02%; N, 11.14%.

Found: C, 59.18%; H, 7.85%; N, 11.04%.

Molecular Weight 628.75.

EXAMPLE 11

N-{(2S,3S)-[3-(N-2'-Quinolinecarbonyl-L-asparaginyl)-amino-4-cyclohexyl-2-hydroxybutyryl]-L-prolyl}-N-t-butylamine (Compound No. 87)

The benzyloxycarbonyl group was removed from 60 mg (0.10 mmol) of N-{(2S,3S)-[3-(N-benzyloxycarbonyl-L-asparaginyl)amino-4-cyclohexyl-2-hydroxybutyryl]-L-prolyl}-N-t-butylamine [obtained according to the procedure described in step (b) of Example 10] following the procedure described in step (a) of Example 3. The resulting N-[(2S,3S)-(3-L-asparaginylamino-4-cyclohexyl-2-hydroxybutyryl)-L-prolyl]-N-t-butylamine hydrochloride was then reacted with 23 mg (0.13 mmol) of quinaldic acid following the procedure described in step (b) of Example 3, to give 31 mg of the title compound as a colourless powder, melting at 135-137°C.

Elemental analysis:

Calculated for $C_{33}H_{46}N_6O_6 \cdot H_2O$

C, 61.85%; H, 7.55%; N, 13.12%.

Found: C, 61.68%; H, 7.33%; N, 12.77%.

Molecular Weight 640.76.

EXAMPLE 12

N-{(2S,3S)-[3-(N-t-Butoxycarbonyl-L-asparaginyl)-amino-2-hydroxy-4-phenylbutyryl]-L-prolyl}-N-t-butylamine (Compound No. 88)

(12a) N-[(2S,3S)-(3-Benzyloxycarbonylamino-2-hydroxy-4-phenylbutyryl)-L-prolyl]-N-t-butylamine

Following the procedure described in step (1a) of Example 1, but using 329 mg (1 mmol) of (2S,3S)-3-benzyloxycarbonylamino-2-hydroxy-4-phenylbutyric acid and 204 mg (1 mmol) of N-proline-N-t-butylamine hydrochloride as starting materials, the title compound was obtained in a yield of 410 mg as a colourless syrupy substance.

Mass spectrum: 479($M^+$).

(12b) N-{(2S,3S)-[3-(N-t-Butoxycarbonyl-L-asparaginyl)-amino-2-hydroxy-4-phenylbutyryl]-L-prolyl}-N-t-butylamine

Following the procedure described in step (b) of Example 1, the benzyloxycarbonyl group was removed from 410 mg (0.86 mmol) of N-[(2S,3S)-(3-benzyloxycarbonylamino-2-hydroxy-4-phenylbutyryl)-L-prolyl]-N-t-butylamine [prepared in step (a) above]. The whole of the resulting N-[(2S,3S)-(3-amino-2-hydroxy-4-phenylbutyryl)-L-prolyl]-N-t-butylamine hydrochloride and 0.36 mg (1.03 mmol) of N-benzyloxycarbonyl-L-asparagine p-nitrophenyl ester were reacted as described in step (b) of Example 1, to produce 0.14 g of the title compound

as a colourless powder, melting at 108-110°C.

Elemental analysis:

Calculated for $C_{28}H_{43}N_5O_7.1/2H_2O$

C, 58.93%; H, 7.77%; N, 12.27%.

Found: C, 58.74%; H, 7.84%; N, 11.90%.

Molecular Weight 570.7.

EXAMPLE 13

N-{(2S,3S)-[3-(N-2′-Quinolinecarbonyl-L-asparaginyl)-amino-2-hydroxy-4-phenylbutyryl]-L-prolyl}-N-t-butylamine (Compound No. 20)

64 mg (0.11 mmol) of N-{(2S,3S)-[3-(N-t-butoxycarbonyl-L-asparaginyl)amino-2-hydroxy-4-phenylbutyryl] L-prolyl}-N-t-butylamine [as prepared in step (b) of Example 12 above], were treated with 2 ml of a 4N solution of hydrogen chloride in dioxane to remove the t-butoxycarbonyl group. Following the procedure described in step (b) of Example 3, the resulting N-[(2S,3S)-(3-L-asparaginylamino-2-hydroxy-4-phenylbutyryl)-L-prolyl]-N-t-butylamine hydrochloride was treated with 21 mg (0.12 mmol) of quinaldic acid to produce 46 mg of the title compound as a colourless powder, melting at 133-135°C.

Elemental analysis:

Calculated for $C_{33}H_{40}N_6O_6.H_2O$

C, 62.44%; H, 6.67%; N, 13.24%.

Found: C, 62.16%; H, 6.49%; N, 13.15%

Molecular Weight 634.7.

EXAMPLE 14

N-{(2S,3S)-[3-(N-2′-Quinoxalinecarbonyl-L-asparaginyl)amino-2-hydroxy-4-phenylbutyryl]-L-prolyl}-N-t-butylamine (Compound No. 23)

Following the procedure described in Example 13, 64 mg (0.11 mmol) of N-{(2S,3S)-[3-(N-t-butoxycarbonyl-L-asparaginyl)amino-2-hydroxy-4-phenylbutyryl]-L-prolyl}-N-t-butylamine [as prepared in step (b) of Example 12 above] were treated with 21 mg (0.11 mmol) of 2-quinoxalinecarboxylic acid, to yield 56 mg of the title compound as a colourless powder, melting at 129-131°C.

Elemental analysis:

Calculated for $C_{32}H_{39}N_7O_6.H_2O$

C, 60.46%; H, 6.50%; N, 15.42%.

Found: C, 60.70%; H, 6.26%; N, 15.43%.

Molecular Weight 635.7.

EXAMPLE 15

(2S,3S)-[3-(N-3′-Quinolinecarbonyl-L-asparaginyl)-amino-2-hydroxy-4-phenylbutyryl]-L-proline t-butyl ester (Compound No. 4)

Following the procedure described in step (b) of Example 3, 60 mg (0.12 mmol) of the t-butyl ester of (2S,3S)-(3-L-asparaginylamino-2-hydroxy-4-phenylbutyryl)-L-proline hydrochloride [obtained according to the

procedure described in step (a) of Example 3] were treated with 20 mg (0.14 mmol) of quinoline-3-carboxylic acid to produce the title compound in a yield of 15 mg and melting at 122-124°C.

Elemental analysis:

Calculated for $C_{33}H_{39}N_5O_7.H_2O$

C, 62.35%; H, 6.50%; N, 11.02%.

Found: C, 62.10%; H, 6.40%; N, 10.92%.

Molecular Weight 635.7.

EXAMPLE 16

(2S,3S)-[3-(N-4′-Quinolinecarbonyl-L-asparaginyl)-amino-2-hydroxy-4-phenylbutyryl]-L-proline t-butyl ester (Compound No. 5)

Following the procedure described in step (b) of Example 3, 70 mg (0.15 mmol) of the t-butyl ester of (2S,3S)-(3-L-asparaginylamino-2-hydroxy-4-phenylbutyryl]-L-proline hydrochloride [obtained according to the procedure described in step (a) of Example 3] were treated with 30 mg (0.18 mmol) of quinoline-4-carboxylic acid to produce the title compound in a yield of 27 mg as a colourless powder, melting at 138-140°C.

Elemental analysis:

Calculated for $C_{33}H_{39}N_5O_7.3H_2O$

C, 59.00%; H, 6.75%; N, 10.43%.

Found: C, 58.83%; H, 6.10%; N, 9.79%.

Molecular Weight 671.7.

EXAMPLE 17

(2S,3S)-[3-(N-4′-Methoxyquinoline-2′-carbonyl-L-asparaginyl)amino-2-hydroxy-4-phenylbutyryl]-L-proline t-butyl ester (Compound No. 6)

Following the procedure described in step (b) of Example 3, 30 mg (0.05 mmol) of the t-butyl ester of (2S,3S)-(3-L-asparaginylamino-2-hydroxy-4-phenylbutyryl)- L-proline hydrochloride [obtained according to the procedure described in step (a) of Example 3] were treated with 16 mg (0.08 mmol) of 4-methoxy-2-quinolinecarboxylic acid, to produce the title compound in a yield of 27 mg as a colourless powder, melting at 118-119°C.

Elemental analysis:

Calculated for $C_{34}H_{41}N_5O_8.H_2O$

C, 61.34%; H, 6.51%; N, 10.52%.

Found: C, 61.15%; H, 6.30%; N, 10.29%.

Molecular Weight 665.7.

EXAMPLE 18

(2S,3S)-[3-(N-4′-Hydroxyquinoline-2′-carbonyl-L-asparaginyl)amino-2-hydroxy-4-pheny1butyryl]-L-proline t-butyl ester (Compound No. 13)

Following the procedure described in step (b) of Example 3, 70 mg (0.15 mmol) of the t-butyl ester of (2S,3S)-(3-L-asparaginylamino-2-hydroxy-4-phenylbutyryl)- L-proline hydrochloride [obtained according to the

procedure described in step (a) of Example 3] were treated with 30 mg (0.18 mmol) of 4-hydroxyquinoline-2-carboxylic acid to produce 18 mg of the title compound as a colourless powder, melting at 100-103°C.

Elemental analysis:

Calculated for $C_{33}H_{39}N_5O_8.3\ 1/2H_2O$

C, 56.88%; H, 6.66%; N, 10.05%.

Found: C, 56.92%; H, 6.31%; N, 8.87%.

Molecular Weight 696.7.

EXAMPLE 19

(2S,3S)-[3-(N-2′-Quinoxalinecarbonyl-L-asparaginyl amino-2-hydroxy-4-phenylbutyryl]-L-proline t-butyl ester (Compound No. 10)

Following the procedure described in step (b) of Example 3, 70 mg (0.15 mmol) of the t-butyl ester of (2S,3S)-(3-L-asparaginylamino-2-hydroxy-4-phenylbutyryl) L-proline hydrochloride [obtained according to the procedure described in step (a) of Example 3] were treated with 30 mg (0.18 mmol) of quinoxaline-2-carboxylic acid to produce 47 mg of the title compound as a colourless powder, melting at 116-118°C.

Elemental analysis:

Calculated for $C_{32}H_{38}N_6O_7.H_2O$

C, 60.36%; H, 6.33%; N, 13.20%

Found C, 60.31%; H, 5.92%; N, 13.06%

Molecular Weight 636.7.

EXAMPLE 20

(2S,3S)-[3-(N-3′-Hydroxyquinoxaline-2′-carbonyl-L-asparaginyl)amino-2-hydroxy-4-phenylbutyryl]-L-proline t-butyl ester (Compound No. 14)

Following the procedure described in step (b) of Example 3, 70 mg (0.15 mmol) of the t-butyl ester of (2S,3S)-(3-L-asparaginylamino-2-hydroxy-4-phenylbutyryl)-L-proline hydrochloride [obtained according to the procedure described in step (a) of Example 3] were treated with 30 mg (0.18 mmol) of 3-hydroxy-2-quinoxalinecarboxylic acid to produce 30 mg of the title compound as a colourless powder, melting at 161-164°C.

Elemental analysis:

Calculated for $C_{32}H_{38}N_6O_8.3H_2O$

C, 55.80%; H, 6.44%; N, 12.20%.

Found: C, 55.51%; H, 5.75%; N, 12.25%.

Molecular Weight 688.7.

EXAMPLE 21

(2S,3S)-[3-(N-5′-Butylpicolyl-L-asparaginyl)amino-2-hydroxy-4-phenylbutyryl]-L-proline t-butyl ester (Compound No. 11)

Following a procedure similar to that described in step (b) of Example 3, but using 60 mg (0.12 mmol) of the t-butyl ester of (2S,3S)-(3-L-asparaginylamino-2-hydroxy-4-phenylbutyryl)-L-proline hydrochloride, prepared in a manner similar to that described in step (a) of Example 3, and 28 mg (0.16 mmol) of fusaric acid in place of quinaldic acid, 56 mg of the title compound was obtained as a colourless powder, melting at 83-85°C.

Elemental analysis:
Calculated for $C_{33}H_{45}N_5O_7$ 1/2$H_2O$
C, 62.64%; H, 7.33%; N, 11.07%
Found: C, 62.55%; H, 7.24%; N, 10.98%

Molecular Weight 632.7.

EXAMPLE 22

(2S,3S)-[3-(N-Picolyl-L-asparaginyl)amino-2-hydroxy-4-phenylbutyryl]-L-proline t-butyl ester (Compound No. 2)

Following a procedure similar to that described in step (b) of Example 3, but using 60 mg (0.12 mmol) of the t-butyl ester of (2S,3S)-(3-L-asparaginylamino-2-hydroxy-4-phenylbutyryl)-L-proline hydrochloride, prepared in a manner similar to that described in step (a) of Example 3, and 18 mg (0.14 mmol) of picolinic acid in place of quinaldic acid, 55 mg of the title compound were obtained as a colourless powder, melting at 104-106°C.

Elemental analysis:
Calculated for $C_{29}H_{37}N_5O_7$.1/4$H_2O$
C, 60.88%; H, 6.61%; N, 12.24%
Found: C, 60.80: H, 6.65%; N, 11.97%

Molecular Weight 572.1.

EXAMPLE 23

(2S,3S)-[3-(N-Nicotinoyl-L-asparaginyl)amino-2-hydroxy-4-phenylbutyryl]-L-proline t-butyl ester (Compound No. 92)

Following a procedure similar to that described in step (b) of Example 3, but using 70 mg (0.15 mmol) of the t-butyl ester of (2S,3S)-(3-L-asparaginylamino-2-hydroxy-4-phenylbutyryl)-L-proline hydrochloride, prepared in a manner similar to that described in step (a) of Example 3, and 20 mg (0.18 mmol) of nicotinic acid in place of quinaldic acid, 56 mg of the title compound were obtained as a colourless powder, melting at 109-111°C.

Elemental analysis:
Calculated for $C_{29}H_{37}N_5O_7$ 1.1/2$H_2O$
C, 58.57%; H, 6.78%; N, 11.78%
Found: C, 58.28%; H, 6.37%; N, 11.50%

Molecular Weight 594.7.

EXAMPLE 24

(2S,3S)-[3-(N-Pyrazinecarbonyl-L-asparaginyl)amino-2-hydroxy-4-phenylbutyryl]-L-proline t-butyl ester (Compound No. 12)

Following a procedure similar to that described in step (b) of Example 3, but using 70 mg (0.15 mmol) of

the t-butyl ester of (2S,3S)-(3-L-asparaginylamino-2-hydroxy-4-phenylbutyryl)-L-proline hydrochloride, prepared in a manner similar to that described in step (a) of Example 3, and 20 mg (0.18 mmol) of pyrazine-2-carboxylic acid in place of quinaldic acid, 41 mg of the title compound were obtained as a colourless powder, melting at 107-110°C.

Elemental analysis:

Calculated for $C_{28}H_{36}N_6O_7.1/2H_2O$

C, 58.22%; H, 6.46%; N, 14.55%

Found: C, 57.88%; H, 6.21%; N, 14.53%

Molecular Weight 577.6.

EXAMPLE 25

(2S,3S)-{3-[N-(2-Thenoyl)-L-asparaginyl]amino-2-hydroxy-4-phenylbutyryl}-L-proline t-butyl ester (Compound No. 93)

Following a procedure similar to that described in step (b) of Example 3, but using 70 mg (0.15 mmol) of the t-butyl ester of (2S,3S)-(3-L-asparaginylamino-2-hydroxy-4-phenylbutyryl)-L-proline hydrochloride, prepared in a manner similar to that described in step (a) of Example 3, and 20 mg (0.18 mmol) of 2-thenoic acid in place of quinaldic acid, 50 mg of the title compound were obtained as a colourless powder, melting at 114-115°C.

Elemental analysis:

Calculated for $C_{28}H_{36}N_4O_7S.H_2O$

C, 56.93%; H, 6.48%; N, 9.49%; S, 5.43%

Found: C, 57.22%; H, 6.09%; N, 9.42%; S, 5.42%

Molecular Weight 590.7.

EXAMPLE 26

(2S,3S)-[3-(N-Benzoyl-L-asparaginyl)amino-2-hydroxy-4-phenylbutyryl]-L-proline t-butyl ester (Compound No. 16)

Following a procedure similar to that described in step (b) of Example 3, but using 60 mg (0.12 mmol) of the t-butyl ester of (2S,3S)-(3-L-asparaginylamino-2-hydroxy-4-phenylbutyryl)-L-proline hydrochloride, prepared in a manner similar to that described in step (a) of Example 3, and 18 mg (0.14 mmol) of benzoic acid in place of quinaldic acid, 45 mg of the title compound were obtained as a colourless powder, melting at 109-110°C.

Elemental analysis:

Calculated for $C_{30}H_{38}N_4O_7.1/2H_2O$

C, 62.59%; H, 6.83%; N, 9.73%

Found: C, 62.51%; H, 6.85%; N, 9.56%

Molecular Weight 575.6.

EXAMPLE 27

(2S,3S)-[3-(N-Benzylaminothiocarbonyl-L-asparaginyl)-amino-2-hydroxy-4-phenylbutyryl]-L-proline t-butyl ester (Compound No. 18)

60 mg (0.12 mmol) of the t-butyl ester of (2S,3S)-(3-L-asparaginylamino-2-hydroxy-4-phenylbutyryl)-L-proline hydrochloride [prepared in a similar procedure to that described in Example 3] and 23 mg (0.16 mmol) of benzyl isothiocyanate were dissolved in 1 ml DMF. The resulting solution was placed on an ice bath, 16 mg (0.16 mmol) of triethylamine were added and the mixture stirred for 14 hours at room temperature. The reaction mixture was then condensed by evaporation under reduced pressure. The residue from the condensation was purified by preparative thin layer chromatography, using a 10 : 1 by volume mixture of methylene chloride and methanol as the developing solvent, and 64 mg of the title compound were obtained as a colourless powder, melting at 107-109°C.

Elemental analysis:

Calculated for $C_{31}H_{41}N_5O_6S.1/4H_2O$

C, 60.42%; H, 6.79%; N, 11.37%; S, 5.20%

Found: C, 60.34%; H, 6.70%; N, 11.13%; S, 5.04%

Molecular Weight 616.3.

EXAMPLE 28

(2S,3S)-[3-(N-Benzylaminocarbonyl-L-asparaginyl)amino-2-hydroxy-4-phenylbutyryl]-L-proline t-butyl ester (Compound No. 17)

Following a procedure similar to that described in step (b) of Example 3, but using 60 mg (0.12 mmol) of the t-butyl ester of (2S,3S)-(3-L-asparaginylamino-2-hydroxy-4-phenylbutyryl)-L-proline hydrochloride, prepared in a manner similar to that described in step (a) of Example 3, and 18 mg (0.13 mmol) of benzyl isocyanate in place of quinaldic acid, 66 mg of the title compound were obtained as a colourless powder, melting at 179-180°C.

Elemental analysis:

Calculated for $C_{31}H_{41}N_5O_7$

C, 62.50, H, 6.94%; N, 11.76%

Found: C, 62.19%; H, 6.93%; N, 11.59%

Molecular Weight 595.7.

EXAMPLE 29

(2S,3S)-{3-[N-(2-Naphthalenesulphonyl)-L-asparaginyl]-amino-2-hydroxy-4-phenylbutyryl}-L-proline t-butyl ester (Compound No. 15)

Following a procedure similar to that described in step (b) of Example 3, but using 60 mg (0.12 mmol) of the t-butyl ester of (2S,3S)-(3-L-asparaginylamino-2-hydroxy-4-phenylbutyryl)-L-proline hydrochloride, prepared in a manner similar to that described in step (a) of Example 3, and 30 mg (0.13 mmol) of 2-naphthalenesulphonyl chloride in place of quinaldic acid, 69 mg of the title compound were obtained as colourless crystals, melting at 115-117°C.

Elemental analysis:

Calculated for $C_{33}H_{40}N_4O_8S.1/2H_2O$

C, 59.89%; H, 6.25%; N, 8.47%; S, 4.85%

Found: C, 59.86%; H, 6.19%; N, 8.32%; S, 4.85%

Molecular Weight 661.8.

EXAMPLE 30

(2S,3S)-[3-(N-Methoxycarbonylcarbonyl-L-asparaginyl)- amino-2-hydroxy-4-phenylbutyryl]-L-proline t-butyl ester (Compound No. 113)

Following a procedure similar to that described in step (b) of Example 3, but using 60 mg (0.12 mmol) of the t-butyl ester of (2S,3S)-(3-L-asparaginylamino-2-hydroxy-4-phenylbutyryl)-L-proline hydrochloride, prepared in a manner similar to that described in step (a) of Example 3, and 18 mg (0.14 mmol) of methyloxalyl chloride in place of quinaldic acid, 54 mg of the title compound were obtained as colourless crystals, melting at 98-100°C.

Elemental analysis:

Calculated for $C_{26}H_{36}N_4O_9.1/2H_2O$

C, 56.00%; H, 6.69%; N, 10.05%

Found: C, 56.00%; H, 6.52%; N, 10.03%

Molecular Weight 557.6.

EXAMPLE 31

(2S,3S)-[3-(L-Prolyl-L-asparaginyl)amino-2-hydroxy-4-phenylbutyryl]-L-proline t-butyl ester (Compound No. 89)

(a) (2S,3S)-{3-[N-(Benzyloxycarbonyl-L-prolyl)-L-asparaginyl]amino-2-hydroxy-4-phenylbutyryl}-L-proline t-butyl ester

Following a procedure similar to that described in step (b) of Example 3, but using 80 mg (0.16 mmol) of the t-butyl ester of (2S,3S)-(3-L-asparaginylamino-2-hydroxy-4-phenylbutyryl)-L-proline hydrochloride, prepared in a manner similar to that described in step (a) of Example 3, and 52 mg (0.21 mmol) of N-benzyloxycarbonyl-L-proline in place of quinaldic acid, 90 mg of the title compound were obtained as a colourless powder, melting at 97-99°C.

Elemental analysis:

Calculated for $C_{36}H_{47}N_5O_9.H_2O$

C, 60.74%; H, 6.94%; N, 9.84%

Found: C, 60.97%; H, 6.78%; N, 9.80%

Molecular Weight 711.8.

(b) t-Butyl ester of (2S,3S)-[3-(L-prolyl-L-asparaginyl)amino-2-hydroxy-4-phenylbutyryl]-L-proline hydrochloride

50 mg (0.07 mmol) of (2S,3S)-{3-[N-(benzyloxycarbonyl-L-prolyl)-L-asparaginyl]amino-2-hydroxy-4-phenylbutyryl}-L-proline t-butyl ester [obtained in (a) above] were dissolved in 2 ml of methanol. 0.08 ml of 1M aqueous hydrochloric acid and 20 mg of 10% w/w palladium-on-carbon were added to the resulting solution.

Hydrogen gas was then bubbled through the solution for 1 hour, after which the catalyst was removed by filtration. The filtrate was condensed to dryness under reduced pressure. Addition of diethyl ether to the residue afforded 40 mg of the title compound as colourless crystals, melting at 148-151°C.

Elemental analysis:
Calculated for $C_{28}H_{41}N_5O_7.5/2H_2O$ HCl
C, 52.45%; H, 7.39%; N, 10.92%
Found: C, 52.27%; H, 6.71%; N, 10.84%

Molecular Weight 641.2.

EXAMPLE 32

t-Butyl ester of (2S,3S)-[3-(L-pipecolyl-L-asparaginyl)amino-2-hydroxy-4-phenylbutyryl]-L-proline hydrochloride (Compound No. 90)

Following a procedure similar to that described in step (b) of Example 3, but using 80 mg (0.16 mmol) of the t-butyl ester of (2S,3S)-(3-L-asparaginylamino-2-hydroxy-4-phenylbutyryl)-L-proline hydrochloride, prepared in a manner similar to that described in step (a) of Example 3, and 51 mg (0.19 mmol) of N-benzyloxycarbonyl-L-pipecolic acid in place of quinaldic acid, 50 mg of (2S,3S)-{3-[N-(benzyloxycarbonyl-L-pipecolyl)-L-asparaginyl]amino-2-hydroxy-4-phenylbutyryl}-L-proline t-butyl ester were obtained as colourless crystals. The benzyloxycarbonyl group was removed from 45 mg of this compound in a procedure similar to that of Example 1, yielding 34 mg of the title compound as colourless crystals, melting at 171-173°C.

Elemental analysis:
Calculated for $C_{29}H_{43}N_5O_7.5/2H_2O$ HCl
C, 53.16%; H, 7.54%; N, 10.69%
Found: C, 52.97%; H, 6.93%; N, 10.62%

Molecular Weight 655.2.

EXAMPLE 33

(2S,3S)-[3-(N-Benzyloxycarbonyl-L-glutaminyl)amino-2-hydroxy-4-phenylbutyryl]-L-proline t-butyl ester (Compound No. 26)

Following a procedure similar to that described in step (b) of Example 1, but using 100 mg (0.26 mmol) of the t-butyl ester of (2S,3S)-3-amino-2-hydroxy-4-phenylbutyryl-L-proline hydrochloride, prepared in a manner similar to that described in step (a) of Example 1, and 157 mg (0.39 mmol) of N-benzyloxycarbonyl-L-glutamine p-nitrophenyl ester in place of N-benzyloxycarbonyl-L-asparagine p-nitrophenyl ester, 117 mg of the title compound were obtained as a colourless powder, melting at 92-94°C.

Elemental analysis:
Calculated for $C_{32}H_{42}N_4O_8.1/2H_2O$
C, 62.02%; H, 6.99%; N, 9.04%
Found: C, 61.89%; H, 6.65%; N, 8.82%

Molecular Weight 619.7.

EXAMPLE 34

(2S,3S)-[3-(N-Benzyloxycarbonyl-L-phenylalanyl)amino-2-hydroxy-4-phenylbutyryl]-L-proline t-butyl ester (Compound No. 99)

Following a procedure similar to that described in step (b) of Example 3, but using 77 mg (0.20 mmol) of the t-butyl ester of (2S,3S)-3-amino-2-hydroxy-4-phenyl-butyryl-L-proline hydrochloride as obtained in Example 1, and 60 mg (0.20 mmol) of N-benzyloxycarbonyl-L-phenylalanine, prepared in a manner similar to that described in step (a) of Example 3, in place of quinaldic acid, 100 mg of the title compound were obtained as a colourless powder, melting at 66-69°C.

Elemental analysis:
Calculated for $C_{36}H_{43}N_{3}O_{7}$
C, 68.66%; H, 6.88%; N, 6.67%
Found: C, 68.75%; H, 7.17%; N, 6.44%

Molecular Weight 629.8.

The compounds of the following Examples 35 to 40 were obtained in a manner similar to that described in Example 34.

EXAMPLE 35

(2S,3S)-[3-(N-Benzyloxycarbonyl-L-seryl)amino-2-hydroxy-4-phenylbutyryl]-L-proline t-butyl ester (Compound No. 100) Melting at: 63-65°C.

Elemental analysis:
Calculated for $C_{30}H_{39}N_{3}O_{8}.H_{2}O$
C, 61.32%; H, 7.03%; N, 7.15%
Found: C, 61.42%; H, 6.63%; N, 7.41%

Molecular Weight 587.7.

EXAMPLE 36

(2S,3S)-[3-(N-Benzyloxycarbonylglycyl)amino-2-hydroxy-4-phenylbutyryl]-L-proline t-butyl ester (Compound No. 98)

Melting at: 65-68°C.

Elemental analysis:
Calculated for $C_{29}H_{37}N_{3}O_{7}.1/2H_{2}O$
C, 63.49%; H, 6.98%; N, 7.66%
Found: C, 63.65%; H, 6.90%; N, 7.70%

Molecular Weight 548.7.

EXAMPLE 37

(2S,3S)-{3-[N-Benzyloxycarbonyl-3-(4-thiazolyl)-L-alanyl]amino-2-hydroxy-4-phenylbutyryl}-L-proline t-butyl ester (Compound No. 101)

Melting at: 84-86°C.

Elemental analysis:
Calculated for $C_{33}H_{40}N_4O_7S$
C, 62.25%; H, 6.33%; N, 8.80%; S, 5.04%
Found: C, 62.19%; H, 6.31%; N, 8.83%; S, 5.03%

Molecular Weight 636.8.

EXAMPLE 38

(2S,3S)-{3-[N-Benzyloxycarbonyl-(3-dimethylcarbamoyl)-L-alanyl]amino-2-hydroxy-4-phenylbutyryl}-L-proline t-butyl ester (Compound No. 74)

Melting at: 59-62°C.

Elemental analysis:
Calculated for $C_{33}H_{44}N_4O_8$
C, 63.44%; H, 7.10%; N, 8.97%
Found: C, 63.10%; H, 7.24%; N, 8.54%

Molecular Weight 624.7.

EXAMPLE 39

(2S,3S)-{3-[N-Benzyloxycarbonyl-3-(1-piperidine-carbonyl)-L-alanyl]amino-2-hydroxy-4-phenylbutyryl}-L-proline t-butyl ester (Compound No. 76)

Melting at: 64-66°C.

Elemental analysis:
Calculated for $C_{36}H_{48}N_4O_8 \cdot 1/2H_2O$
C, 64.17%; H, 7.33%; N, 8.31%
Found: C, 64.01%; H, 7.37%; N, 8.09%

Molecular Weight 673.8.

EXAMPLE 40

(2S,3S)-[3-(N-Benzyloxycarbonyl-L-histidyl)amino-2-hydroxy-4-phenylbutyryl]-L-proline t-butyl ester (Compound No. 102)

Melting at: 94-98°C.

Elemental analysis:
Calculated for $C_{33}H_{41}N_5O_7$
C, 63,96%; H, 6.67%; N, 11.30%
Found: C, 63.74%; H, 6.63%; N, 11.44%

Molecular Weight 619.7.

EXAMPLE 41

(2S,3S)-[3-(N-Benzyloxycarbonyl-D-asparaginyl)amino-2-hydroxy-4-phenylbutyryl]-L-proline t-butyl ester (Compound No. 1)

Melting at: 87-89°C.

Elemental analysis:
Calculated for $C_{31}H_{40}N_4O_8.1/2H_2O$
C, 61.47%; H, 6.82%; N, 9.25%.
Found: C, 61.78%; H, 6.84%; N, 9.22%.

Molecular weight: 605.69.

EXAMPLE 42

(2S,3S)-[3-(N-Benzyloxycarbonyl-L-cyanoalanyl)amino-2-hydroxy-4-phenylbutyryl]-L-proline t-butyl ester (Compound No. 34)

Melting at: 76-77°C.

Elemental analysis:
Calculated for $C_{31}H_{38}N_4O_7.2H_2O$
C, 64.57%; H, 6.89%; N, 9.12%.
Found: C, 64.34%; H, 6.62%; N, 9.68%.

Molecular weight: 614.68.

EXAMPLE 43

(2S,3S)-[3-(N-Benzyloxycarbonyl-3-morpholinocarbonyl-L-alanyl)amino-2-hydroxy-4-phenylbutyryl]-L-proline t-butyl ester (Compound No. 75)

Melting at: 73-76°C.

Elemental analysis:
Calculated for $C_{35}H_{46}N_4O_9$
C, 63.05%; H, 6.95%; N, 8.40%.
Found: C, 62.86%; H, 7.16%; N, 8.22%.

Molecular weight: 666.78.

EXAMPLE 44

(2S,3S)-[3-(N-Benzyloxycarbonyl-3-methylcarbamoyl-L-alanyl)amino-2-hydroxy-4-phenylbutyryl]-L-proline t-butyl ester (Compound No. 96)

Melting at: 162-164°C.

Elemental analysis:
Calculated for $C_{32}H_{42}N_4O_8.1/2H_2O$
C, 62.02%; H, 6.99%; N, 9.04%.
Found: C, 62.14%; H, 6.91%; N, 9.02%.

Molecular weight: 619.79.

EXAMPLE 45

(2S,3S)-[3-(N-Benzyloxycarbonyl-3-ethylcarbamoyl-L-alanyl)amino-2-hydroxy-4-phenylbutyryl]-L-proline t-butyl ester (Compound No. 97)

Melting at: 149-152°C.

Elemental analysis:
Calculated for $C_{33}H_{44}N_4O_8.4H_2O$
C, 56.88%; H, 7.52%; N, 8.04%.
Found: C, 56.79%; H, 7.26%; N, 7.80%.

Molecular weight: 696.78.

EXAMPLE 46

(2S,3S)-[3-(N-Benzyloxycarbonyl-L-asparaginyl)amino-2-hydroxy-4-phenylbutyryl]-L-proline ethyl ester (Compound No. 114)

Following a similar procedure to that described in Example, but using the ethyl ester hydrochloride of L-proline, the title compound was obtained.
Melting at: 133-135°C.

Elemental analysis:

Calculated for $C_{29}H_{36}N_4O_8$

C, 61.26%; H, 6.38%; N, 9.85%.

Found: C, 61.11%; H, 6.40%; N, 9.78%.

Molecular weight: 568.63.

EXAMPLE 47

(2S,3S)-[3-(N-Indoline-2′-carbonyl-L-asparaginyl)amino-2-hydroxy-4-phenylbutyryl]-L-proline t-butyl acetate (Compound No. 108)

Following a similar procedure to that described in Example 32, but using a 16:4:1 v/v mixture of chloroform, methanol and acetic acid as the developing solvent, the title compound was obtained.
Melting at: 215-218°C (with decomposition).

Elemental analysis:

Calculated for $C_{32}H_{41}N_5O_7.C_2H_4O_2.H_2O$

C, 59.55%; H, 6.91%; N, 10.21%.

Found: C, 59.83%; H, 6.62%; N, 10.03%.

Molecular weight: 685.8.

EXAMPLE 48

(2S,3S)-[3-(N-Phenoxyacetyl-L-asparaginyl)amino-2-hydroxy-4-phenylbutyryl]-L-proline t-butyl ester (Compound No. 115)

Following a procedure similar to that of Example 3, the title compound was obtained.
Melting at: 87-90°C.

Elemental analysis:

Calculated for $C_{31}H_{40}N_4O_8.1/2H_2O$

C, 61.47%; H, 6.82%; N, 9.25%.

Found: C, 61.53%; H, 6.76%; N, 9.08%.

Molecular weight: 605.7.

EXAMPLE 49

N-{(2S,3S)-[3-(N-4′-Methoxyhenoxyacetyl-L-asparaginyl)-amino-2-hydroxy-4-phenylbutyryl]-L-prolyl}-N-t-butylamine (Compound No. 116)

Following a procedure similar to that of Example 13, the title compound was obtained.
Melting at: 97-99°C.

Elemental analysis:

Calculated for $C_{32}H_{43}N_5O_8.H_2O$

C, 59.70%; H, 7.05%; N, 10.88%.

Found: C, 59.62%; H, 6.93%; N, 10.66%.

Molecular weight: 643.7.

EXAMPLE 50

N={(2S,3S)-[3-(N-5′-Methoxyindole-2′-carbonyl-L-asparaginyl)amino-2-hydroxy-4-phenylbutyryl]-L-prolyl}-N-t-butylamine (Compound No. 117)

Following a procedure similar to that of Example 13, the title compound was obtained.
Melting at: 146-149°C.

Elemental analysis:
Calculated for $C_{33}H_{42}N_6O_7.H_2O$
C, 60.72%; H, 6.79%; N, 12.88%.
Found: C, 60.76%; H, 6.80%; N, 12.65%.

Molecular weight: 652.7.

EXAMPLE 51

N-{(2S,3S)-[3-(N-5′-Hydroxyindole-2′-carbonyl-L-asparaginyl)amino-2-hydroxy-4-phenylbutyryl]-L-prolyl}-N-t-butylamine (Compound No. 118)

Following a procedure similar to that of Example 13, the title compound was obtained.
Melting at: 166-168°C.

Elemental analysis:
Calculated for $C_{32}H_{40}N_6O_7.H_2O$
C, 60.17%; H, 6.63%; N, 13.16%.
Found: C, 59.85%; H, 6.86%; N, 12.93%.

Molecular weight: 638.7.

EXAMPLE 52

N-{(2S,3S)-[3-(N-5′-Acetoxyindole-2′-carbonyl-L-asparaginyl)amino-2-hydroxy-4-phenylbutyryl]-L-prolyl}-N-t-butylamine (Compound No. 119)

50 mg (0.081 mmol) of N-{(2S,3S)-[3-(N-5′-hydroxyindole-2′-carbonyl-L-asparaginyl)amino-2-hydroxy-4-phenylbutyryl]-L-prolyl}-N-t-butylamine (prepared in Example 51) was added to 1 ml of N,N-dimethylformamide to prepare a solution, and to this were added 10 mg (0.097 mmol) of acetic anhydride and 8.5 mg (0.081 mmol) of sodium carbonate. The resulting mixture was stirred at room temperature for 14 hours. After this time, the solvent was removed by distillation under reduced pressure and the organic residue was mixed with ethyl acetate. The organic layer was separated and washed with a 5% w/v aqueous solution of citric acid, a 5% w/v aqueous solution of sodium hydrogencarbonate and a saturated aqueous solution of sodium chloride, in that order, and dried over anhydrous sodium sulphate. The solvent was then removed by distillation under reduced pressure, and the residue was purified by preparative thin layer chromatography, using a 7:1 by volume mixture of methylene chloride and methanol as the developing solvent, to give 23 mg of the title compound as a colourless powder, melting at 151-152°C.

Elemental analysis:

Calculated for $C_{34}H_{42}N_6O_8.H_2O$

C, 59.98%; H, 6.52%; N, 12.35%.

Found: C, 60.05%; H, 6.34%; N, 12.30%.

Molecular weight: 680.7.

EXAMPLE 53

N-{(2S,3S)-[3-(N-5′-Aminoacetoxyindole-2′-carbonyl-L-asparaginyl)amino-2-hydroxy-4-phenylbutyryl]-L-prolyl}-N-t-butylamine (Compound No. 120)

70 mg (0.11 mmol) of N-{(2S,3S)-[3-(N-5′-hydroxy-indole-2′-carbonyl-L-asparaginyl)amino-2-hydroxy-4-phenylbutyryl]-L-prolyl}-N-t-butylamine (prepared in Example 51) was added to 1 ml of N,N-dimethylformamide to prepare a solution, and to this were added 37 mg (0.14 mmol) of N-t-butoxycarbonylglycine N-hydroxysuccinimide and 35 μl (0.25 mmol) of triethylamine. The resulting mixture was stirred at room temperature for 48 hours. After this time, the solvent was distilled off under reduced pressure and the organic residue was mixed with ethyl acetate. The resulting organic solution was washed with a 5% w/v aqueous solution of citric acid, a 5% w/v aqueous solution of sodium hydrogencarbonate and a saturated aqueous solution of sodium chloride, in that order, and dried over anhydrous sodium sulphate. The solvent was then distilled off under reduced pressure, and the residue was purified by preparative thin layer chromatography, using a 7:1 by volume mixture of methylene chloride and methanol as the developing solvent, to give 41 mg of NN-{(2S,3S)-[3-(N-5′-t-butoxycarbonylaminoacetoxy-indole-2′-carbonyl-L-asparaginyl)amino-2-hydroxy-4-phenylbutyryl]-L-prolyl}-N-t-butylam ine as a colourless powder in a yield of 20 mg (0.026 mmol). The whole of this was then dissolved in 1 ml of methanol and the resulting solution was placed on an ice bath. 2 ml of a 4 N solution of hydrogen chloride in dioxane was added to the solution with stirring, and the stirring was continued for 30 minutes. The solvent was then distilled off under reduced pressure. Diethyl ether was added to the residue to give 18 mg of the hydrochloride of the title compound as a colourless powder, melting at 186-188°C.

Elemental analysis:

Calculated for $C_{34}H_{43}N_7O_8.3H_2O.HCl$

C, 53.15%; H, 6.56%; N, 12.76%; Cl, 4.62%.

Found: C, 53.04%; H, 6.07%; N, 12.41%; Cl, 4.98%.

Molecular weight: 768.3.

EXAMPLE 54

N-{(2S,3S)-[3-(N-Benzyloxycarbonyl-L-asparaginyl)amino-2-hydroxy-4-phenylbutyryl]-L-prolyl}-N-benzylamine (Compound No. 106)

68 mg (0.13 mmol) of (3S,6S,1′S)-3-[1′-(N-benzyloxycarbonyl-L-asparaginyl)amino-2′-phenylethyl]-1-aza-4-oxabicyclo[4.3.0]nonan-2,5-dione (prepared as described in Preparation 1) were dissolved in 1 ml of N,N-dimethylformamide. 71 μl (0.65 mmol) of benzylamine was added to the solution, and the resulting mixture was allowed to stand at room temperature for 14 hours. After this time, the solvent was distilled off under reduced pressure, and the residue was mixed with ethyl acetate. The organic layer was separated and washed with a 5% w/v aqueous solution of citric acid, a 5% w/v aqueous solution of sodium hydrogencarbonate and a saturated aqueous solution of sodium chloride, in that order, and dried over anhydrous sodium sulphate. The solvent was then distilled off under reduced pressure. The organic residue was purified by preparative thin layer chromatography, using a 8:1 by volume mixture of methylene chloride and methanol as the developing solvent, to give 52 mg of the title compound as a colourless powder, melting at 97-99°C.

Elemental analysis:
Calculated for $C_{34}H_{39}N_5O_7$
C, 64.85%; H, 6.24%; N, 11.12%.
Found: C, 64.80%; H, 6.18%; N, 11.18%.

Molecular weight: 629.7.

The compounds of Examples 55 to 67 were prepared in a similar manner.

EXAMPLE 55

N-{(2S,3S)-[3-(N-Benzyloxycarbonyl-L-asparaginyl)amino-2-hydroxy-4-phenylbutyryl]-L-prolyl}-N-butylamine (Compound No. 81)

Melting at: 91-93°C.

Elemental analysis:
Calculated for $C_{31}H_{41}N_5O_7.1/4H_2O$
C, 62.03%; H, 6.97%; N, 11.67%.
Found: C, 61.98%; H, 6.87%; N, 11.53%.

Molecular weight: 600.2.

EXAMPLE 56

N-{(2S,3S)-[3-(N-Benzyloxycarbonyl-L-asparaginyl)amino-2-hydroxy-4-phenylbutyryl]-L-prolyl}-N,N-dimethylamine (Compound No. 105)

Melting at: 100-102°C.

Elemental analysis:
Calculated for $C_{29}H_{37}N_5O_7.3/2H_2O$
C, 58.57%; H, 6.78%; N, 11.78%.
Found: C, 58.91%; H, 6.42%; N, 11.43%.

Molecular weight: 594.7.

EXAMPLE 57

(2S,3S)-[3-(N-Benzyloxycarbonyl-L-asparaginyl)amino-2-hydroxy-4-phenylbutyryl]-L-prolyl-L-isoleucinol (Compound No. 103)

Melting at: 100-103°C.

Elemental analysis:

Calculated for $C_{33}H_{45}N_5O_8.1/2H_2O$

C, 61.09%; H, 7.15%; N, 10.80%.

Found: C, 60.99%; H, 7.16%; N, 10.39%.

Molecular weight: 648.7.

EXAMPLE 58

N-{(2S,3S)-[3-(N-Benzyloxycarbonyl-L-asparaginyl)amino-2-hydroxy-4-phenylbutyryl]-L-prolyl}-N-phenethylamine (Compound No. 121)

Melting at: 93-95°C.

Elemental analysis:

Calculated for $C_{35}H_{41}N_5O_7$

C, 65.38%; H, 6.42%; N, 10.88%.

Found: C, 65.28%; H, 6.39%; N, 10.94%.

Molecular weight: 643.7.

EXAMPLE 59

N-{(2S,3S)-[3-(N-Benzyloxycarbonyl-L-asparaginyl)amino-2-hydroxy-4-phenylbutyryl]-L-prolyl}-N-benzhydrylamine (Compound No. 122)

Melting at: 105-107°C.

Elemental analysis:

Calculated for $C_{40}H_{43}N_5O_7.5/2H_2O$

C, 63.98%; H, 6.44%; N, 9.33%.

Found: C, 63.90%; H, 6.13%; N, 9.50%.

Molecular weight: 750.8.

EXAMPLE 60

N-{(2S,3S,1′R)-[3-(N-Benzyloxycarbonyl-L-asparaginyl)-amino-2-hydroxy-4-phenylbutyryl]-L-prolyl}-N-(1-phenylethyl)amine (Compound No. 123)

Melting at: 100-102°C.

Elemental analysis:

Calculated for $C_{35}H_{41}N_5O_7.H_2O$

C, 63.52%; H, 6.55%; N, 10.58%.

Found: C, 63.35%; H, 6.20%; N, 10.40%.

Molecular weight: 661.7.

EXAMPLE 61

N-{(2S,3S,1'S)-[3-(N-Benzyloxycarbonyl-L-asparaginyl)-amino-2-hydroxy-4-phenylbutyryl]-L-prolyl}-N-(1-phenylethyl)amine (Compound No. 123)

Melting at: 98-99°C.

Elemental analysis:
Calculated for $C_{35}H_{41}N_5O_7.1/2H_2O$
C, 64.40%; H, 6.49%; N, 10.73%.
Found: C, 64.29%; H, 6.50%; N, 10.55%.

Molecular weight: 652.7.

EXAMPLE 62

N-{(2S,3S)-[3-(N-Benzyloxycarbonyl-L-asparaginyl)amino-2-hydroxy-4-phenylbutyryl]-L-prolyl}-N-butyl-N-methylamine (Compound No. 124)

Melting at: 84-86°C.

Elemental analysis:
Calculated for $C_{32}H_{43}N_5O_7.H_2O$
C, 61.22%; H, 7.23%; N, 11.16%.
Found: C, 61.19%; H, 7.03%; N, 11.19%.

Molecular weight: 627.7.

EXAMPLE 63

N-{(2S,3S)-[3-(N-Benzyloxycarbonyl-L-asparaginyl)amino-2-hydroxy-4-phenylbutyryl]-L-prolyl}-N-(2-pyridyl)-methylamine (Compound No. 82)

Melting at: 97-99°C.

Elemental analysis:
Calculated for $C_{33}H_{38}N_6O_7.1/2H_2O$
C, 61.96%; H, 6.07%; N, 13.14%.
Found: C, 61.73%; H, 6.29%; N, 13.08%.

Molecular weight: 639.7.

EXAMPLE 64

N-{(2S,3S)-[3-(N-Benzyloxycarbonyl-L-asparaginyl)amino-2-hydroxy-4-phenylbutyryl]-L-prolyl}-N-(3-pyridyl)-methylamine (Compound No. 125)

Melting at: 109-111°C.

Elemental analysis:
Calculated for $C_{33}H_{38}N_6O_7.H_2O$
C, 61.10%; H, 6.22%; N, 12.96%.
Found: C, 61.20%; H, 6.28%; N, 12.64%.

Molecular weight: 648.7.

EXAMPLE 65

N-{(2S,3S)-[3-(N-Benzyloxycarbonyl-L-asparaginyl)amino-2-hydroxy-4-phenylbutyryl]-L-prolyl}-N-(4-pyridyl)-methylamine (Compound No. 126)

Melting at: 109-111°C.

Elemental analysis:
Calculated for $C_{33}H_{38}N_6O_7.H_2O$
C, 61.10%; H, 6.22%; N, 12.96%.
Found: C, 61.41%; H, 6.37%; N, 12.67%.

Molecular weight: 648.7.

EXAMPLE 66

N-{(2S,3S)-[3-(N-Benzyloxycarbonyl-L-asparaginyl)amino-2-hydroxy-4-phenylbutyryl]-L-prolyl}-N-(1-ethylpyrrolidin-2-yl)methylamine (Compound No. 127)

Melting at: 118-120°C.

Elemental analysis:
Calculated for $C_{34}H_{46}N_6O_7.3H_2O$
C, 57.94%; H, 7.44%; N, 11.93%.
Found: C, 57.57%; H, 7.00%; N, 11.69%.

Molecular weight: 704.8.

EXAMPLE 67

N-{(2S,3S)-[3-(N-Benzyloxycarbonyl-L-asparaginyl)amino-2-hydroxy-4-phenylbutyryl]-L-prolyl}-N-(3-hydroxypropyl)amine (Compound No. 128)

Melting at: 90-92°C.

Elemental analysis:

Calculated for $C_{30}H_{39}N_5O_8.H_2O$

C, 58.52%; H, 6.71%; N, 11.38%.

Found: C, 58.72%; H, 6.57%; N, 11.49%.

Molecular weight: 615.7.

EXAMPLE 68

N-{(2S,3S)-[3-(N-Benzyloxycarbonyl-L-asparaginyl)amino-2-hydroxy-4-phenylbutyryl]-L-prolyl}-N-t-butylamine (Compound No. 19)

67 mg (0.15 mmol) of (2S,3S)-3-(N-benzyloxycarbonyl-L-asparaginyl)amino-2-hydroxy-4-phenylbutyric acid (prepared as described in Preparation 2), 47 mg (0.23 mmol) of L-proline-t-butylamide hydrochloride and 24 mg (0.18 mmol) of 1-hydroxybenzotriazole were dissolved in 1 ml of N,N-dimethylformamide. 83 µl (0.60 mmol) of triethylamine and 35 mg (0.18 mmol) of 1-ethyl-3-(3-di- methylaminopropyl)carbodiimide hydrochloride were added to the resulting solution with stirring. The resulting mixture was kept at 4°C with continuous stirring for 24 hours. After this time, the solvent was distilled off under reduced pressure and the organic residue was mixed with ethyl acetate. The organic layer was separated and washed with a 5% w/v aqueous solution of citric acid, a 5% w/v aqueous solution of sodium hydrogencarbonate and a saturated aqueous solution of sodium chloride, in that order, and dried over anhydrous sodium sulphate. The solvent was then distilled off under reduced pressure and the residue purified by thin layer chromatography, using a 10:1 by volume mixture of methylene chloride and methanol as the developing solvent, to give 52 mg of the title compound as a colourless powder, melting at 101-102°C.

Elemental analysis:

Calculated for $C_{31}H_{41}N_5O_7.3/2H_2O$

C, 59.79%; H, 7.12%; N, 11.25%.

Found: C, 60.00%; H, 6.83%; N, 11.30%.

Molecular weight: 622.7.

EXAMPLE 69

N-{(2S,3S)-[3-(N-Benzyloxycarbonyl-L-asparaginyl)-amino-2-hydroxy-4-phenylbutyryl]-D-proline-t-butylamine (Compound No. 19)

The title compound was obtained by following the procedure described in Example 68, but using N-D-proline-N-t-butylamine hydrochloride.
Melting at: 110-112°C.

Elemental analysis:

Calculated for $C_{31}H_{41}N_5O_7.3/2H_2O$

C, 59.79%; H, 7.12%; N, 11.25%.

Found: C, 59.88%; H, 6.63%; N, 11.14%.

Molecular weight: 622.7.

EXAMPLE 70

N-{(2S,3S)-[3-(N-Benzyloxycarbonyl-L-asparaginyl)-amino-2-hydroxy-4-phenylbutyryl]-L-prolyl}-N-amine (Compound No. 110)

The title compound was obtained by following the procedure described in Example 68, but using L-prolinamide hydrochloride.
Melting at: 122-124°C.

Elemental analysis:
Calculated for $C_{27}H_{33}N_5O_7.2H_2O$
C, 55.47%; H, 6.55%; N, 11.98%.
Found: C, 55.07%; H, 6.82%; N, 11.59%.

Molecular weight: 584.64.

EXAMPLE 71

N-{(2S,3S)-[3-(N-Indole-2′-carbonyl-L-asparaginyl)-amino-2-hydroxy-4-phenylbutyryl]-L-prolyl}-N-t-butylamine (Compound No. 25)

The title compound was obtained by following the procedure described Example 13, but using indole-2-carboxylic acid.
Melting at: 152-154°C.

Elemental analysis:
Calculated for $C_{32}H_{40}N_6O_6.H_2O$
C, 61.72%; H, 6.80%; N, 13.50%.
Found: C, 62.03%; H, 6.61%; N, 13.30%.

Molecular weight: 613.7.

EXAMPLE 72

(2S,3S)-[3-(N-Benzyloxycarbonyl-L-aspartyl)amino-2-hydroxy-4-butyryl]-L-proline t-butyl ester (Compound No. 129)

72(a) (2S,3S)-[3-(N-Benzyloxycarbonyl-β-benzyl- L-aspartyl)amino-2-hydroxy-4-phenylbutyryl]-L-proline t-butyl ester

Following a procedure similar to that described in Example 3, but using 115 mg (0.3 mmol) of (2S,3S)-(3-amino-2-hydroxy-4-phenylbutyryl)-L-proline t-butyl ester hydrochloride (as prepared in Example 1) and 129 mg (0.36 mmol) of β-benzyl benzyloxycarbonyl-L-aspartate, 160 mg of title compound was obtained as a colourless powder. The structure of the product was confirmed by nuclear magnetic resonance.
Nuclear Magnetic Resonance Spectrum ($CDCl_3$) δ ppm:
1.43 (9H, singlet);
1.89-2.32 (4H, multiplet);
2.60-2.93 (4H, multiplet);
3.56-3.92 (3H, multiplet);
4.31-4.62 (3H, multiplet);
5.09 (4H, doublet, J=6Hz);
5.66 (1H, broad doublet, J=8.5Hz);
6.87 (1H, broad doublet, J=8.5Hz);
7.12-7.40 (15H, multiplet).

72(b) (2S,3S)-[3-(N-Benzyloxycarbonyl-L-aspartyl)amino-2-hydroxy-4-phenylbutyryl]-L-prolyl}-N-t-butylamine

In order to remove the benzyloxycarbonyl and benzyl groups, the whole of the compound obtained in a) above [160 mg (0.23 mmol)] was dissolved in 5 ml of methanol and subjected to catalytic hydrogenolysis in the presence of 20 mg of 10% w/w palladium-on-charcoal for 5 hours. After this time, the catalyst was filtered off and the filtrate was concentrated to dryness under reduced pressure. The organic residue was dissolved in 5 ml of ethyl acetate. 57 mg (0.23 mmol) of N-(benzyloxycarbonyl)succinimide and 23 mg (0.23 mmol) of N-methylmorpholine were added to the resulting solution. After the reaction mixture had been stirred at room temperature for 11.5 hours, it was washed with water and dried over anhydrous anhydrous sodium sulphate, and the solvent was distilled off under reduced pressure. The residue was purified by preparative thin layer chromatography through silica gel, using a 6:1 by volume mixture of methylene chloride and methanol as the developing solvent, to give 55 mg of the title compound as a colourless powder, melting at 121-123°C.

Elemental analysis:

Calculated for $C_{31}H_{39}N_3O_9.2H_2O$

C, 58.75%; H, 6.84%; N, 6.63%.

Found: C, 58.91%; H, 6.33%; N, 6.52%.

Molecular weight: 633.68.

EXAMPLE 73

(2S,3S)-[3-(N-Benzyloxycarbonyl-L-glutamyl)amino-2-hydroxy-4-phenylbutyryl]-L-proline t-butyl ester (Compound No. 130)

Following a similar procedure to that described in Example 72, but using benzyl N-benzyloxycarbonyl-L-glutamate, the title compound was obtained as a colourless powder, melting at 80-82°C.

Elemental analysis:

Calculated for $C_{32}H_{41}N_3O_9.1/2H_2O$

C, 61.92%; H, 6.82%; N, 6.77%.

Found: C, 61.99%; H, 6.83%; N, 6.85%.

Molecular weight: 620.68.

EXAMPLE 74

(2S,3S)-[3-(N-Benzimidazole-5'-carbonyl-L-asparaginyl)-amino-2-hydroxy-4-phenylbutyryl]-L-proline t-butyl ester (Compound No. 131)

Following a procedure similar to that described in Example 3, but using benzimidazole-5-carboxylic acid, the title compound was obtained as a colourless powder, melting at 162-164°C.

Elemental analysis:

Calculated for $C_{31}H_{38}N_6O_7.2H_2O$

C, 57.96%; H, 6.59%; N, 13.08%.

Found: C, 58.14%; H, 6.65%; N, 12.64%.

Molecular weight: 642.42.

EXAMPLE 75

(2S,3S)-N-{[3-(N-Benzyloxycarbonyl-L-asparaginyl)amino-2-hydroxy-4-phenylbutyryl]-L-prolyl}-2-methylalaninol (Compound No. 132)

75(a) N-(N-t-Butoxycarbonyl-L-prolyl)-2-methylalaninol

2.15 g (10 mmol) of N-t-butoxycarbonylproline was dissolved in 30 ml of dry tetrahydrofuran. 1.11 g (11 mmol) of N-methylmorpholine was added to the solution, and the resulting mixture was cooled to -20°C. 1.5 g (11 mmol) of isobutyl chloroformate were then added to the mixture, which was stirred at -10°C for 20 minutes. After this time, 0.98 g (11 mmol) of 2-amino-2-methyl-1-propanol was added to the mixture, which was then stirred at -10°C for 5 hours.

Subsequently, the reaction mixture was worked up as described in Example 68, to give 2.08 g of the title compound as colourless crystals, melting at 147-149°C

75 (b) (2S,3S)-N-{[3-(N-Benzyloxycarbonyl-L-asparaginyl)-amino-2-hydroxy-4-phenylbutyryl]-L-prolyl}-2-methyl-alaninol

Following a procedure similar to that described in Example 68, L-prolyl-2-methylalaninol was prepared by treating 344 mg (1.2 mmol) of N-(N-t-butoxycarbonyl-L-prolyl)-2-methylalaninol [prepared in (a) above] with 5 ml of a 4 N solution of hydrogen chloride in dioxane. The product was reacted with 443 mg (1 mmol) of (2S,3S)-3-(N-benzyloxycarbonyl-L-asparaginyl)amino-2-hydroxy-4-phenylbutyric acid (prepared as described in Preparation 2), to give 323 mg of the title compound as a colourless powder, melting at 112-114°C.

Elemental analysis:

Calculated for $C_{31}H_{41}N_5O_8.3/2H_2O$

C, 58.30%; H, 6.94%; N, 10.96%.

Found: C, 58.14%; H, 6.54%; N, 10.79%.

Molecular weight: 638.72.

EXAMPLE 76

(2S,3S)-N-[3-(N-Benzyloxycarbonyl-L-asparaginyl)amino-2-hydroxy-4-phenylbutyryl]-L-prolyl-L-alaninol (Compound No. 133)

76(a) N-t-Butoxycarbonyl-L-prolyl-L-alaninol

The title compound was obtained by following a procedure similar to that described in Example 75 (a), but using L-alaninol. The structure of the resulting compound was confirmed nuclear magnetic resonance.

Nuclear Magnetic Resonance Spectrum ($CDCl_3$) δ ppm:

1.17 (3H, doublet, J=6.4Hz);
1.48 (9H, singlet);
1.83-2.35 (5H, multiplet);
3.32-3.60 (3H, multiplet);
3.63-3.73 (1H, multiplet);
3.96-4.12 (1H, multiplet);
4.25 (1H, broad singlet).

76(b) (2S,3S)-N-[3-(N-Benzyloxycarbonyl-L-asparaginyl)-amino-2-hydroxy-4-phenylbutyryl]-L-prolyl-L-alaninol

Following a procedure similar to that described in Example 75 (b), but using all of the compound prepared in step (a) above, the title compound was obtained as a colourless powder, melting at 103-105°C.

Elemental analysis:

Calculated for $C_{30}H_{39}N_5O_8.3/2H_2O$

C, 57.68%; H, 6.78%; N, 11.21%.

Found: C, 57.88%; H, 6.67%; N, 10.96%.

Molecular weight: 624.7.

EXAMPLE 77

2,2-Bis(hydroxymethyl)-2-{[(2S,3S)-3-(N-benzyloxycarbonyl-L-asparaginyl)amino-2-hydroxy-4-phenylbutyryl]-L-prolylamino}ethanol (Compound No. 134)

77(a) 2,2-Bis(hydroxymethyl)-2-(N-benzyloxycarbonyl-L-prolylamino)ethanol

0.36 g (3 mmol) of tris(hydroxymethyl)aminomethane was dissolved in 10 ml of N,N-dimethylformamide, and 1.04 g (3 mmol) of N-benzyloxycarbonylproline N-hydroxysuccinimide were added to the resulting solution. The mixture thus obtained was then stirred at room temperature for 24 hours. At the end of this time, the reaction mixture was concentrated by evaporation under reduced pressure, and the residue was purified by preparative thin layer chromatography through silica gel, using a 10:1 by volume mixture of methylene chloride and methanol as the developing solvent, to give 0.52 g of the title compound as a colourless powder, melting at 122-123°C.

Mass spectrum: $M^+$ 352 ($C_{17}H_{24}N_2O_6$)

77(b) 2,2-Bis(hydroxymethyl)-2-{[(2S,3S)-3-(N-benzyloxycarbonyl-L-asparaginyl)amino-2-hydroxy-4-phenylbutyryl]-L-prolylamino}ethanol

The benzyloxycarbonyl group was removed from 70.5 mg (0.2 mmol) of 2,2-bis(hydroxymethyl)-2-(N-benzyloxycarbonyl-L-prolylamino)ethanol [prepared as described in step (a) above], by the procedure described in step (a) of Example 3, to give 2,2-bis(hydroxymethyl)-2-(L-prolylamino)ethanol hydrochloride. The whole of this compound was treated as described in step (b) of Example 75 to give 27 mg of the title compound as a colourless powder, melting at 113-115°C.

Elemental analysis:

Calculated for $C_{31}H_{41}N_5O_{10}.H_2O$

C, 56.27%; H, 6.55%; N, 10.58%.

Found: C, 55.84%; H, 6.54%; N, 10.58%.

Molecular weight: 661.72.

EXAMPLE 78

(2S,3S)-N-{[3-(N-Quinoxaline-2′-carbonyl-L-asparaginyl)-amino-2-hydroxy-4-phenylbutyryl]-L-prolyl}-2-methylalaninol (Compound No. 135)

The benzyloxycarbonyl group was removed from 306 mg (0.5 mmol) of (2S,3S)-[3-(N-benzyloxycarbonyl-L-asparaginyl)amino-2-hydroxy-4-phenylbutyryl]-L-prolyl 2-methylalaninol (as prepared in Example 75), by the procedure of step (a) of Example 3, to give 240 mg of (2S,3S)-[(3-L-asparaginylamino-2-hydroxy-4-phenylbutyryl)]-L-prolyl-2-methylalaninol hydrochloride. 139 mg (0.27 mmol) of the resulting colourless powder was reacted with 52 mg (0.27 mmol) of quinoxaline-2-carboxylic acid in a manner similar to that described in Example 3, to give 79 mg of the title compound as a pale yellow powder, melting at 124-126°C.

Elemental analysis:

Calculated for $C_{32}H_{39}N_7O_7.3/2H_2O$

C, 58.17%; H, 6.41%; N, 14.84%.

Found: C, 58.60%; H, 6.42%; N, 14.47%.

Molecular weight: 660.7.

EXAMPLE 79

N-{(2S,3S)-[3-(N-Benzyloxycarbonyl-L-asparaginyl]amino-2-hydroxy-4-phenylbutyryl]-(3S,4aS,8aS)-decahydroisoquinoline-3-carbonyl}-N-t-butylamine (Compound No. 60)

The title compound was obtained by a procedure similar to that described in Example 68. Melting at: 110-115°C.

Elemental analysis:

Calculated for $C_{36}H_{49}N_5O_7.1/2H_2O$

C, 64.27%; H, 7.49%; N, 10.41%.

Found: C, 64.41%; H, 7.63%; N, 10.22%.

Molecular weight: 672.84.

EXAMPLE 80

N-{(2S,3S)-[3-(N-2′-Quinoxalinecarbonyl-L-aspartyl)amino-2-hydroxy-4-phenylbutyryl]-L-prolyl}-N-t-butylamine (Compound No. 208)

The title compound was obtained by a procedure similar procedure to that of Example 3. Melting at: 136-138°C.

Elemental analysis:

Calculated for $C_{32}H_{38}N_6O_7.H_2O$

C, 60.37%; H, 6.33%; N, 13.20%.

Found: C, 60.53%; H, 6.60%; N, 13.12%.

Molecular weight: 636.71.

PREPARATION 1

(3S,6S,1′S)-3-[1′-(N-Benzyloxycarbonyl-L-asparaginyl)-amino-2′-phenylethyl]-1-aza-4-oxabicyclo-[4.3.0]nonan-2,5-dione

380 mg (0.64 mmol) of (2S,3S)-[3-(N-benzyloxycarbonyl-L-asparaginyl)amino-2-hydroxy-4-phenylbutyryl]-L-proline t-butyl ester (as prepared in Example 1) were placed over ice, and 0.21 ml (1.91 mmol) of anisole and 5 ml of trifluoroacetic acid were added. The resulting mixture was stirred at room temperature for 4.5 hours. After this time, the solvent was distilled off under reduced pressure. The organic residue was mixed with diethyl ether to give 300 mg of the title compound as a colourless powder, melting at 260-262°C.

Elemental analysis:

Calculated for $C_{27}H_{30}N_4O_7.1/4H_2O$

C, 61.53%; H, 5.83%; N, 10.63%.

Found: C, 61.37%; H, 5.78%; N, 10.51%.

Molecular weight: 527.0.

PREPARATION 2

(2S,3S)-3-(N-Benzyloxycarbonyl-L-asparaginyl)amino-2-hydroxy-4-phenylbutyric acid

A solution of 900 mg (3.9 mmol) of the hydrochloride of (2S,3S)-3-amino-2-hydroxy-4-phenylbutyric acid was prepared in 15 ml of N,N-dimethylformamide and placed on an ice bath. 2.26 g (5.8 mmol) of benzyloxycarbonyl-L-asparagine p-nitrophenyl ester and 1.89 ml (13.6 mmol) of triethylamine were added to the solution, and the resulting mixture was stirred at 4°C for 2 days. After this time, the solvent was distilled off under reduced pressure. 1 N aqueous hydrochloric acid was added to the organic residue, and precipitated crystals were collected by filtration and washed with water and ethyl acetate, in that order. 1.52 g of the title compound was obtained as a colourless powder, melting at 225-227°C.

Elemental analysis:

Calculated for $C_{22}H_{25}N_3O_7.H_2O$

C, 57.26%; H, 5.90%; N, 9.11%.

Found: C, 57.33%; H, 5.61%; N, 9.18%.

Molecular weight: 461.5.

Test Example 1

Assay of inhibition of HIV pol protease

The ability of the compounds of the invention to inhibit the activity of HIV pol protease may be demonstrated by assaying HIV protease, expressed in E. coli, using a synthetic substrate. The $K_i$, i.e. the dissociation constant of the enzyme-inhibitor complex, measured according to the equation

$$K_i = \frac{[E][I]}{[EI]}$$

[in which [E] is the concentration of enzyme, [I] is the concentration of inhibitor and [EI] is the concentration of E + I],
is a measure of the activity of the compounds of the invention.

a) Construction of the expression vector

Using the appropriate restriction enzymes, the sequence between the ClaI site in the gag region and the EcoRI site in the pol region was excised from clone BH10. The BH10 clone [Flossie Wong-Staal et al., Nature (1985), 313, 277-284] contains the main part of the HTLV IIIB provirus. The fragment obtained was cloned into plasmid pBR322 at the corresponding restriction sites (ClaI - EcoRI), with deletion of the existing fragment in pBR322 at that location.

The sequence between the BamHI and ClaI sites, upstream of the ClaI to EcoRI fragment, was excised using the appropriate restriction enzymes, and the cleaved plasmid was then ligated with a synthetic nucleotide sequence, designated TE-1. The fragment TE-1 has the following base sequence and contains a translation initiation codon where indicated.

TE-1

GATCCTACCA AGTGATGGGT GCGAGAGCGT CAGTATTAAG CGGGGGAGAA
TTAGATGATG GTTCACTACC CACGCTCTCG CAGTCATAAT TCGCCCCCTC
TTAATCTAGC

The BglII to BamHI fragment of the T7 promoter region [Barbara A. Moffatt et al., J. Mol. Biol. (1986), 189, 113-130] was subsequently inserted into the BamHI site of the resulting plasmid, thereby being positioned upstream of the TE-1 sequence.

In order to enhance expression of the gag and pol regions, a frame shift mutation was then introduced into the plasmid as follows. The plasmid was digested with BglII, and the recessed 3′ ends filled in by use of the Klenow fragment. The resulting blunt ends of the linearized plasmid were then religated with T4 DNA ligase to give the expression vector, pT7HIV.GP(-), containing a part of HIV gag and pol regions.

b) Expression in Escherichia coli

pT7HIV.GP(-) was introduced into an E. coli host containing the T7 polymerase gene [(DE-3) Barbara A. Moffatt et al., J. Mol. Biol. (1986), 189, 113-130] using standard procedures. The resulting transformant was incubated at 37°C in M9CA-10% LB medium [9 parts M9CA medium ($Na_2HPO_4.12H_2$, 42 mM; $KH_2PO_4$, 22 mM; NaCl, 8.6 mM; $NH_4Cl$, 18.7 mM; Casamino acids, 2.0 g/l; $MgSO_4$, 2 mM; $CaCl_2$, 0.1 mM; glucose, 0.2% w/v) and 1 part LB medium (Bacto-tryptone, 10 g/l; Bacto-yeast extract, 5 g/l; NaCl, 10 g/l)], containing 200 μg/ml of ampicillin, until the absorbancy at 600 nm reached 2.

0.4 mM of isopropylthio-β-D-galactoside was added to the culture medium, and incubation was continued for a further 3 hours.

After this time, the bacterial cells were collected and preserved at -80°C.

c) Purification of the enzyme

Expression of the plasmid by the E. coli host results in the formation of a polyprotein comprising among others, the HIV protease. The polyprotein is self-digested in cells to produce HIV protease.

The pelleted bacterial cells obtained from 2 liters of culture medium were re-suspended in 60 ml of Buffer A [50 mM tris HCl (pH 7.5), 1 mM dithiothreitol, 0.7% w/v lysozyme, 10 μg/ml aprotinin, 5 mM ethylenediaminetetraacetic acid, 10 μg/ml benzamide, 1 mM fluorophenylmethylsulphonic acid, 10% v/v glycerin], and allowed to stand for 10 minutes at 0°C. Triton X-100 (0.1% w/v) was added to the suspension and the suspension was allowed to stand for a further 10 minutes at 0°C.

The suspension was then frozen and thawed four times. DNase (0.1 mg) and 10 mM magnesium chloride were added to the suspension to decompose any DNA present.

The suspension was then centrifuged at 10,000 x g for 15 minutes, and the supernatant obtained was run on a DEAE Sephadex (Trade Mark) A25 chromatography column (50 mm inner diameter by 200 mm) using, as the eluent, Buffer B [50 mM HEPES (N-2-hydroxyethylpiperazine-N′-2-ethanesulphonic acid) (pH 7.8), 1 mM dithiothreitol, 10 μg/ml aprotinin, 5 mM ethylenediaminetetraacetic acid, 10 μg/ml benzamide, 1 mM fluorophenylmethylsulphonic acid, 10% glycerin]. The column had been equilibrated with Buffer B.

Biologically active fractions were collected. Proteins were precipitated by addition of ammonium sulphate to a final concentration of 60% w/v. The precipitate was dissolved in 2 ml of Buffer C [50 mM tris HCl (pH 7.5), 1 mM dithiothreitol, 1 mM ethylenediaminetetraacetic acid, 200 mM sodium chloride] and the solution run on a TSK G2000SW (Trade Mark) gel filtration column (7.5 mm inner diameter by 600 mm, To-So Co., Tokyo, Japan), and eluted with Buffer C at a flow rate of 0.5 ml/min.

Biologically active fractions obtained were collected and concentrated to half of the original volume by use of a 10kD ultra-filter. The resulting enzyme solutions were stored at -80°C.

d) Activity assay

A reaction mixture containing 1 mM or 1.5 mM of the substrate, (TE-2; Ac-Ser-Gln-Asn-Tyr-Pro-Ile-Val-$NH_2$), 1 μl of the desired concentration of the compound to be tested (dissolved in dimethyl sulphoxide comprising 20% v/v water), 2 μl of the enzyme solution prepared in c), above, and buffer solution [50 mM tris HCl

(pH 6.0), 0.25 M sodium chloride, 0.1 mM ethylenediaminetetraacetic acid, 0.1 mM Triton X-100] to form 10 μl in total, was incubated for 30 minutes at 37°C.

The reaction was stopped by addition of 250 μl of 0.1% w/v aqueous trifluoroacetic acid and 10% v/v acetonitrile. The reaction mixture was subjected to Sep-pak light (Trade Mark - Waters Co., Milford, Ma., USA), and fractions passing through the column were collected.

TE-2 is decomposed by HIV protease to form a shortened peptide of four amino acids, (TE-3; Ac-Ser-Gln-Asn-Tyr). The amount of TE-3 formed was assayed quantitatively using HPLC [ODS-120T (Trade Mark) column, 4.6 mm in inner diameter X 250 mm, To-So Co.; solvent: 9% acetonitrile-0.05% trifluoroacetic acid].

The $K_i$ in nM was calculated according to the method of Michaelis-Menten using the equation given above.

| Compound No. | Ki (nM) |
|---|---|
| 3 | 6.3 |

TEST EXAMPLE 2

Assay for Anti-retrovirus activity

The anti-retroviral activity of the peptide derivatives of the present invention was determined by assaying their ability to inhibit focus formation of the murine retrovirus Mo-MSV in NIH3T3 cells.

NIH3T3 cells were grown up on a 35 mm petridish having Dulbecco's modified Eagle's medium supplemented with 10% v/v fetal calf serum. 1.5 x $10^5$ cells were placed in each dish, and incubated at 37°C overnight under an atmosphere containing 5% carbon dioxide. The next day, the dishes were seeded with virus (prepared in Dulbecco's'modified Eagle's medium) at a potency of 50 focus/dish. Polybrene (Trade Mark - hexadimethrine bromide - previously prepared as a stock aqueous solution at 2 mg/ml) was added to a final concentration of 10 μg/ml, and incubation was continued for a further 6 hours. After this time, the medium was replaced with fresh medium containing the peptide sample to be tested.

The dish was incubated for a further 4 days. Foci formation of transformed cells was determined microscopically.

The concentration of sample necessary to inhibit focus formation by 50% ($ED_{50}$) was calculated, and is given in the following Table.

Anti-retrovirus activity

| Compound No. | $ED_{50}$ (μM) |
|---|---|
| 3 | 0.15 |
| 4 | 0.50 |

TEST EXAMPLE 3

Anti-HIV activity

a) Inhibition of processing of HIV gag polyprotein

A Molt4 cell line chronically infected with HTLVIIIB was established (Molt4/HTLVIIIB). Molt4/HTLVIIIB cells were washed twice with growth medium (RPMI-1640 medium containing 10% v/v heat-inactivated fetal calf serum). The washed cells were then inoculated into growth medium at a density of 2 x $10^5$ cells/ml, in the presence of selected concentrations of test compounds, at 37°C, and cultured under an atmosphere of 5% carbon dioxide.

After 48 hours, the test cultures were spun down at 1000 x g for 3 minutes, and the supernatant was collected. Polyethylene glycol 6000 was added to the supernatant to a final concentration of 10% w/v, and left to stand for 6 hours on ice. Virions precipitated by this procedure were subsequently obtained as a pellet by centrifuging the treated supernatants for 30 minutes at 10000 x g. The resulting pelleted virion fractions were subjected to SDS-PAGE in the usual manner [Laemmli, U.K., Nature (1970), 227, 680]. p17, formed proteolytically from P55 gag polyprotein, was then assayed by immunoblotting, using anti-P17 monoclonal antibody (DuPont).

$IC_{100}$ (the minimum concentration of test compound sufficient to totally prevent p17 production, as measured by immunoblotting) was used as a measure of anti-HIV activity.

The results are shown in the following Table.

TABLE

| Compound No. | $IC_{100}$ (μg/ml) |
|---|---|
| 14 (ki, 11nM) | 0.6 |
| 13 (ki, 27nM) | 3 |
| 3 (ki, 36nM) | 18 |
| Ro 31-8959 (ki, 58nM) | 20 |

Ro 31-8959 is 3-t-butylaminocarbonyl-2-{(2R, 3S)-3-[(N-2'-quinolinecarbonyl-L-asparaginyl)amino]-2-hydroxy-4-phenylbutyl}-(3S, 4aS, 8aS)-decahydroisoquinoline, and has the formula:

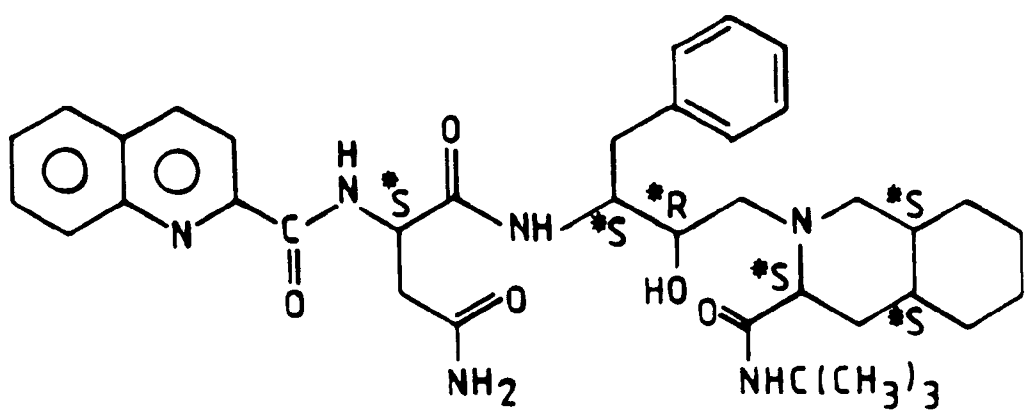

RO 31-8959

b) Inhibition of virion release

Supernatant of Molt4 cells chronically infected with HTLVIIIB was prepared in a manner similar to that described in a) above. HIV antigen was then determined using a commercial enzyme-linked immunosorbent assay system (HIV antigen EIA kit, Abbott).

In this Example, the measure of anti-HIV activity is $IC_{50}$, wherein $IC_{50}$ is defined as the minimum concentration required to reduce HIV antigen levels by 50% as determined by the assay system.

The results are shown in the following Table.

TABLE

| Compound No. | $IC_{50}$ (μg/ml) |
|---|---|
| 14 | 0.56 |
| Ro 31-8959 | >20 |

c) Inhibition of HIV Proliferation

CEM cells were inoculated in growth medium as defined in a) above, to a density of $2 \times 10^4$ cells/ml. The inoculum was then seeded with a similar volume of serial 1 : 3 dilutions of the viral stock [a filtered preparation of the 48-hour culture supernatant of Molt4/HTLVIIIB cells prepared as described in Test Example 3 a) above]. The seeded inoculum was then cultured at 37°C under a 5% carbon dioxide atmosphere in the presence of 1 μM of the test compound (compound no. 14). After 1 week, the culture was spun down and the supernatant was collected. HIV antigen in the supernatant was determined as described in b) above.

The results are shown in the accompanying Figure, wherein the graph indicated by "□" shows virus released by cells incubated with the test compound, while the graph indicated by "○" shows virus released by cells incubated without the test compound.

From the results, it can be seen that 1 μM (0.6 μg/ml) of the test compound was sufficient to block the spread of HIV almost completely in CEM cells.

SEQUENCE LISTING

SEQ. ID NO: (TE-1)
SEQ. TYPE: nucleotide
SEQ. LENGTH 117 nucleotides

STRANDEDNESS: single
TOPOLOGY: linear
SOURCE: synthetic
PROPERTIES: fragment comprises initiation codon (ATC)

GATCCTACCA AGTGATGGGT GCGAGAGCGT CAGTATTAAG CGGGGGAGAA (50
TTAGATGATG GTTCACTACC CACGCTCTCG CAGTCATAAT TCGCCCCCTC (100
TTAATCTAGC (110

SEQ. ID NO: (TE-2)
SEQ. TYPE: amino acid
SEQ. LENGTH: 7 amino acids
PROPERTIES: HIV protease substrate

Ac-Ser Gln Asn Tyr Pro Ile Val-$NH_2$
5

SEQ. ID NO: (TE-3)
SEQ. TYPE: amino acid
SEQ. LENGTH: 4 amino acids
PROPERTIES: digestion product

Ac-Ser Gln Asn Tyr
4

## Claims

**1.** A compound of formula (I):

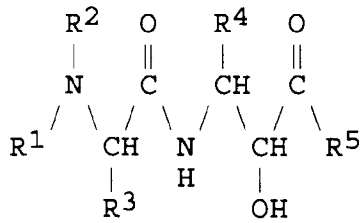

(I)

wherein:

$R^1$ represents a group of formula

R (Z) $_x$A-

wherein R represents

a hydrogen atom,

an alkyl group having from one to six carbon atoms,

an alkyl group having from one to six carbon atoms and being substituted by at least one of substituents (a) below,

an alkenyl group having from two to seven carbon atoms,

an alkenyl group having from two to seven carbon atoms and being substituted by at least one of substituents (a) below,

an alkynyl group having from two to seven carbon atoms,

an alkynyl group having from two to seven carbon atoms and being substituted by at least one of substituents (a) below,

a cycloalkyl group having from three to ten carbon atoms,

a cycloalkyl group having from three to ten carbon atoms and being substituted by at least one of substituents (b) below,

a carbocyclic aryl group having from six to fourteen carbon atoms,

a carbocyclic aryl group having from six to fourteen carbon atoms and being substituted by at least one of substituents (b) below, or

a heterocyclic group having from 3 to 10 ring atoms of which from 1 to 4 are nitrogen and/or oxygen and/or sulphur heteroatoms, said heterocyclic group being unsubstituted or substituted by at least one of substituents (b) below,

or R represents a group of formula $-NR^aR^b$

wherein $R^a$ and $R^b$ are independently selected from hydrogen atoms,

alkyl groups having from 1 to 4 carbon atoms, cycloalkyl groups having from three to ten carbon atoms,

cycloalkyl groups having from three to ten carbon atoms and being substituted by at least one of substituents (b) below,

heterocyclic groups having from 3 to 10 ring atoms of which from 1 to 4 are nitrogen and/or oxygen and/or sulphur heteroatoms, said heterocyclic group being unsubstituted or substituted by at least one of substituents (b) below,

carbocyclic aryl groups having from 6 to 14 carbon atoms,

carbocyclic aryl groups having from 6 to 14 carbon atoms and being substituted by at least one of substituents (b) below,

aralkenyl groups in which the aryl group has from 6 to 14 carbon atoms and the alkenyl group has from 2 to 7 carbon atoms, and

aralkyl groups in which the aryl group has from 6 to 14 carbon atoms and the alkyl group has from 1 to 3 carbon atoms;

Z represents oxygen or sulphur;

A represents a group of formula -CO-, -COCO-, -SO-, $-SO_2-$ or -CS-; and

x is the cipher 0 or the integer 1;

$R^2$ represents a hydrogen atom, an alkyl group having from one to six carbon atoms or an alkyl group having from one to six carbon atoms and being substituted with at least one of substituents (a) below;

$R^3$ represents

a hydrogen atom,

an alkyl group having from one to six carbon atoms,

an alkyl group having from one to six carbon atoms and being substituted by at least one of substituents (c) below,

a cycloalkyl group having from three to ten carbon atoms,

a cycloalkyl group having from three to ten carbon atoms and being substituted by at least one of substituents (b) below,

an alkenyl group having from two to seven carbon atoms,

an alkenyl group having from two to seven carbon atoms and being substituted by at least one of substituents (a) below,

an alkynyl group having from two to seven carbon atoms,

an alkynyl group having from two to seven carbon atoms and being substituted by at least one of substituents (a) below,

a carbocyclic aryl group having from six to fourteen carbon atoms,

a carbocyclic aryl group having from six to fourteen carbon atoms and being substituted by at least one of substituents (b) below, or

a heterocyclic group having from 3 to 10 ring atoms of which from 1 to 4 are nitrogen and/or oxygen and/or sulphur heteroatoms, said heterocyclic group being unsubstituted or substituted by at least one of substituents (b) below;

$R^4$ represents a hydrogen atom,

an alkyl group having from one to six carbon atoms,

an alkyl group having from one to six carbon atoms and being substituted by at least one of substituents (a) below,

a cycloalkyl group having from three to ten carbon atoms,

a cycloalkyl group having from three to ten carbon atoms and being substituted by at least one of substituents (b) below,

a carbocyclic aryl group having from six to fourteen carbon atoms,

a carbocyclic aryl group having from six to fourteen carbon atoms and being substituted by at least one of substituents (b) below, or

a heterocyclic group having from 3 to 10 ring atoms of which from 1 to 4 are nitrogen and/or oxygen and/or sulphur heteroatoms, said heterocyclic group being unsubstituted or substituted by at least one of substituents (b) below;

and

$R^5$ represents a group of formula $-B-(CO)-Y-R^c_y$ , wherein

B represents a heterocyclic group having from 3 to 10 ring atoms of which from 1 to 4 are nitrogen and/or oxygen and/or sulphur heteroatoms, at least one of which is a nitrogen heteroatom, said heterocyclic group being unsubstituted or substituted with at least one alkyl group having from one to six carbon atoms;

Y represents an oxygen atom, a nitrogen atom or a sulphur atom;

y is the integer 1 when Y represents an oxygen atom or a sulphur atom, and 2 when Y represents a nitrogen atom; and

$R^c$ represents

a hydrogen atom,

an alkyl group having from one to six carbon atoms,

an alkyl group having from one to six carbon atoms and being substituted by at least one of substituents (a) below,

a cycloalkyl group having from three to ten carbon atoms,

a cycloalkyl group having from three to ten carbon atoms and being substituted by at least one of substituents (b) below,

an alkenyl group having from two to seven carbon atoms,

an alkenyl group having from two to seven carbon atoms and being substituted by at least one substituent selected from substituents (a) below,

an alkynyl group having from two to seven carbon atoms,

an alkynyl group having from two to seven carbon atoms and being substituted by at least one of substituents (a) below,

a carbocyclic aryl group having from six to fourteen carbon atoms,

a carbocyclic aryl group having from six to fourteen carbon atoms and being substituted by at least one of substituents (b) below, or

a heterocyclic group having from 3 to 10 ring atoms of which from 1 to 4 are nitrogen and/or oxygen and/or sulphur heteroatoms, said heterocyclic group being unsubstituted or substituted by at least one of substituents (b) below;

and, where there are two groups or atoms represented by $R^c$, they are the same or different; and

<u>substituents (a):</u>

hydroxy groups, $C_3$-$C_{10}$ cycloalkyl groups, $C_1$-$C_6$ alkoxy groups, $C_1$-$C_6$ aliphatic acyloxy groups, $C_1$-$C_6$ aliphatic acyl groups, carboxy groups, $C_2$-$C_6$ alkoxycarbonyl groups, sulpho groups, halogen atoms, amino groups, $C_2$-$C_4$ aliphatic acylamino groups, alkylamino groups in which the alkyl part is $C_1$-$C_6$ alkyl, dialkylamino groups in which each alkyl part is $C_1$-$C_3$ alkyl, carbocyclic aryl groups having from six to fourteen carbon atoms, carbocyclic aryl groups having from six to fourteen carbon atoms and being substituted by at least one of substituents (b) below, carbocyclic aryloxy groups having from six to fourteen carbon atoms, carbocyclic aryloxy groups having from six to fourteen carbon atoms and being substituted by at least one of substituents (b) below, and heterocyclic groups having from 3 to 10 ring atoms of which from 1 to 4 are nitrogen and/or oxygen and/or sulphur heteroatoms, said heterocyclic group being unsubstituted or substituted by at least one of substituents (b) below;

<u>substituents (b):</u>

$C_1$-$C_6$ alkyl groups having from 0 to 3 of substituents (a), $C_1$-$C_6$ haloalkyl groups, $C_1$-$C_6$ aliphatic acyl groups having from 0 to 3 of substituents (a), $C_1$-$C_6$ alkylenedioxy groups, aralkyl groups wherein the alkyl part is $C_1$-$C_6$ alkyl and the aryl part is $C_6$-$C_{14}$ carbocyclic aryl which has from 0 to 3 of substituents (b), aralkyloxycarbonyl groups wherein the alkyl part is $C_1$-$C_6$ alkyl and the aryl part is $C_6$-$C_{14}$ carbocyclic aryl which has from 0 to 3 of substituents (b), hydroxy groups, $C_1$-$C_6$ alkoxy groups, $C_6$-$C_{14}$ carbocyclic aryl groups having from 0 to 3 of substituents (b), aralkyloxy groups where the alkyl part is $C_1$-$C_6$ alkyl and the aryl part is $C_6$-$C_{14}$ carbocyclic aryl which has from 0 to 3 of substituents (b), $C_6$-$C_{14}$ carbocyclic aryloxy groups having from 0-3 of substituents (b), heterocyclic groups having from 3 to 10 ring atoms of which 1 to 4 are nitrogen and/or oxygen and/or sulphur, said heterocyclic group being unsubstituted or substituted by at least one of substituents (b), halogen atoms, nitro groups, cyano groups, carboxy groups, alkoxycarbonyl groups having a total of from 2 to 7 carbon atoms, amino groups, $C_1$-$C_6$ alkylamino groups, dialkylamino groups wherein each alkyl part is $C_1$-$C_6$ alkyl, aliphatic or carbocyclic aromatic carboxylic acylamino groups, carbamoyl groups, alkylcarbamoyl groups where the alkyl part is $C_1$-$C_6$ alkyl, dialkylcarbamoyl groups where each alkyl part is $C_1$-$C_6$ alkyl, mercapto groups, $C_1$-$C_6$ alkylthio groups, $C_6$-$C_{14}$ carbocyclic arylthio groups, $C_1$-$C_6$ alkylsulphonyl groups, $C_6$-$C_{14}$ carbocyclic arylsulphonyl groups wherein the aryl part has from 0 to 3 $C_1$-$C_6$ alkyl substituents, aminosulphonyl groups, $C_1$-$C_6$ alkylsulphinyl groups and $C_6$-$C_{14}$ carbocyclic arylsulphinyl groups wherein the aryl part has from 0 to 3 $C_1$-$C_6$ alkyl substituents;

<u>substituents (c):</u>

hydroxy groups, $C_1$-$C_6$ alkoxy groups, $C_6$-$C_{14}$ carbocyclic aryl groups having from 0 to 3 of substituents (b), aralkyloxy groups where the alkyl part is $C_1$-$C_6$ alkyl and the carbocyclic aryl part is $C_6$-$C_{14}$ carbocyclic aryl which has from 0 to 3 of substituents (b), $C_6$-$C_{14}$ carbocyclic aryloxy groups having from 0-3 of substituents (b), heterocyclic groups having from 3 to 10 ring atoms of which 1 to 4 are nitrogen and/or oxygen and/or sulphur, said heterocyclic group being unsubstituted or substituted by at least one of substituents (b), halogen atoms, nitro groups, cyano groups, carboxy groups, alkoxycarbonyl groups having a total of from 2 to 7 carbon atoms, amino groups, $C_1$-$C_6$ alkylamino groups, dialkylamino groups wherein each alkyl part is $C_1$-$C_6$ alkyl, aliphatic or carbocyclic aromatic carboxylic acylamino groups, carbamoyl groups, alkylcarbamoyl groups where the alkyl part is $C_1$-$C_6$ alkyl, dialkylcarbamoyl groups where each alkyl part is $C_1$-$C_6$ alkyl, mercapto groups, $C_1$-$C_6$ alkylthio groups, $C_6$-$C_{14}$ carbocyclic arylthio groups, $C_1$-$C_6$ alkylsulphonyl groups, $C_6$-$C_{14}$ carbocyclic arylsulphonyl groups wherein the aryl part has from 0 to 3 $C_1$-$C_6$ alkyl substituents, aminosulphonyl groups, $C_1$-$C_6$ alkylsulphinyl groups and $C_6$-$C_{14}$ carbocyclic arylsulphinyl groups wherein the aryl part has from 0 to 3 $C_1$-$C_6$ alkyl substituents;

provided that, where a substituent (a) is substituted by a substituent (b), then substituent (b) is not further substituted by a substituent (a); that where a substituent (b) is substituted by a substituent (a), then substituent (a) is not further substituted by a substituent (b); that, where substituent (b) is a group which is itself substituted by a further substituent (b), that further substituent (b) is not itself substituted; and that where a substituent (c) is substituted by a substituent (b), that further substituent (b) is not itself substituted, and pharmaceutically acceptable salts and esters thereof.

2. A compound according to Claim 1, wherein A represents a group of formula -CO- or $-SO_2-$ .

3. A compound according to Claim 1, wherein A represents a group of formula -CO-.

4. A compound according to any preceding Claim, wherein $R^1$ represents a group of formula

$$R(Z)_xA-$$

wherein R represents

a hydrogen atom,

an alkyl group having from one to six carbon atoms,

an alkyl group having from one to six carbon atoms and being substituted by at least one of substituents (a′) below,

a carbocyclic aryl group having from six to fourteen carbon atoms,

a carbocyclic aryl group having from six to fourteen carbon atoms and being substituted by at least one of substituents (b′) below,

or

a heterocyclic group having from 3 to 10 ring atoms of which from 1 to 4 are nitrogen and/or oxygen and/or sulphur atoms, said heterocyclic group being unsubstituted or substituted by at least one of substituents (b′) below;

Z represents oxygen;

A represents a group of formula -CO- or $-SO_2-$; and

x is the cipher 0 or the integer 1;

substituents (a′):

hydroxy groups, $C_3$-$C_{10}$ cycloalkyl groups, $C_1$-$C_6$ alkoxy groups, $C_1$-$C_6$ aliphatic acyloxy groups, $C_1$-$C_6$ aliphatic acyl groups, carboxy groups, $C_2$-$C_6$ alkoxycarbonyl groups, sulpho groups, halogen atoms, amino groups, $C_2$-$C_4$ aliphatic acylamino groups, alkylamino groups in which the alkyl part is $C_1$-$C_3$ alkyl, dialkylamino groups in which each alkyl part is $C_1$-$C_3$ alkyl, carbocyclic aryl groups having from six to fourteen carbon atoms, carbocyclic aryl groups having from six to fourteen carbon atoms and being substituted by at least one of substituents (b′) below, carbocyclic aryloxy groups having from six to fourteen carbon atoms, carbocyclic aryloxy groups having from six to fourteen carbon atoms and being substituted by at least one of substituents (b′) below, and heterocyclic groups having from 3 to 10 ring atoms of which from 1 to 4 are nitrogen and/or oxygen and/or sulphur heteroatoms, said heterocyclic group being unsubstituted or substituted by at least one of substituents (b′) below;

substituents (b′):

$C_1$-$C_6$ alkyl groups having from 0 to 3 of substituents (a′), $C_1$-$C_2$ haloalkyl groups, $C_1$-$C_6$ aliphatic carboxylic acyl groups having from 0 to 3 of substituents (a′), hydroxy groups, $C_1$-$C_6$ alkoxy groups, $C_6$-$C_{14}$ carbocyclic aryl groups having from 0 to 3 of substituents (b′), halogen atoms, nitro groups, cyano groups, carboxy groups, amino groups, carbamoyl groups, mercapto groups, $C_1$-$C_6$ alkylthio groups, $C_1$-$C_6$ alkylsulphonyl groups and aminosulphonyl groups;

provided that, where a substituent (a′) is substituted by a substituent (b′), then substituent (b′) is not further substituted by a substituent (a′); that where a substituent (b′) is substituted by a substituent (a′), then substituent (a′) is not further substituted by a substituent (b′); and that, where substituent (b′) is a group which is itself substituted by a further substituent (b′), that further substituent (b′) is not itself substituted.

5. A compound according to any preceding Claim, wherein $R^1$ represents a group of formula

$$R(Z)_xA-$$

wherein R represents

an alkyl group having from one to six carbon atoms and being substituted by at least one of substituents (a′) as defined in Claim 4,

a carbocyclic aryl group having from six to fourteen carbon atoms,

a carbocyclic aryl group having from six to fourteen carbon atoms and being substituted by at least one substituent selected from $C_{1-6}$ alkyl groups, $C_{1-6}$ alkoxy groups and hydroxy groups,

a heterocyclic group having from 3 to 10 ring atoms of which from 1 to 4 are nitrogen and/or oxygen and/or sulphur heteroatoms, said heterocyclic group being unsubstituted or substituted by at least one substituent selected from $C_1$-$C_6$ alkyl groups, $C_1$-$C_6$ alkoxy groups and hydroxy groups,

Z represents oxygen;

A represents a group of formula -CO- or $-SO_2-$; and

x is the cipher 0 or the integer 1.

6. A compound according to any preceding Claim, wherein $R^1$ represents a group of formula

$$R(Z)_xA-$$

wherein R represents a group of formula $-NR^aR^b$ wherein $R^a$ and $R^b$ are independently selected from heterocyclic groups having from 3 to 10 ring atoms of which from 1 to 4 are nitrogen and/or oxygen and/or sulphur heteroatoms, said heterocyclic group being unsubstituted or substituted by at least one $C_1$-$C_6$ alkyl group, $C_1$-$C_6$ alkoxy group or hydroxy group; carbocyclic aryl groups having from 6 to 14 carbon atoms; and aralkyl groups in which the aryl part has from 6 to 14 carbon atoms and the alkyl part has from 1 to 3 carbon atoms;

A represents a group of formula -CO-; and

x is the cipher 0.

**7.** A compound according to any preceding Claim, wherein $R^1$ represents a group of formula

$$R(Z)_xA-$$

wherein R represents

an alkyl group having from one to six carbon atoms and being substituted by at least one of phenyl, naphthyl, phenoxy and naphthoxy, or phenyl, naphthyl, phenoxy or naphthoxy substituted by at least one substituent selected from methyl, hydroxy, methoxy and phenoxy groups,

a carbocyclic aryl group having from six to fourteen carbon atoms,

a heterocyclic group having from 3 to 10 ring atoms of which from 1 to 4 are nitrogen and/or oxygen and/or sulphur heteroatoms, said heterocyclic group being unsubstituted or substituted by at least one substituent selected from $C_1$-$C_6$ alkyl groups, $C_1$-$C_6$ alkoxy groups and hydroxy groups,

A represents a group of formula -CO-;

Z represents an oxygen atom; and

x is the cipher 0 or the integer 1.

**8.** A compound according to any preceding Claim, wherein $R^1$ represents a group of formula

$$R(Z)_xA-$$

wherein R represents

an alkyl group having from one to six carbon atoms and being substituted by at least one of phenyl, phenoxy, 4-methoxyphenyl, 4-methoxyphenoxy, 3-phenylphenoxy, naphthyl and naphthoxy, a carbocyclic aryl group having from six to fourteen carbon atoms,

a benzofuranyl, indolyl, quinolyl or quinoxalinyl group, which is unsubstituted or substituted by at least one substituent selected from $C_1$-$C_6$ alkyl groups, $C_1$-$C_6$ alkoxy groups and hydroxy groups,

A represents a group of formula -CO-;

Z represents an oxygen atom; and

x is the cipher 0 or the integer 1.

**9.** A compound according to any preceding Claim, wherein $R^2$ represents a hydrogen atom, an alkyl group having from one to four carbon atoms or an alkyl group having from one to four carbon atoms and being substituted with at least one of substituents (a′) as defined in Claim 4.

**10.** A compound according to Claim 9, wherein $R^2$ represents a hydrogen atom.

**11.** A compound according to any preceding Claim, wherein $R^3$ represents

an alkyl group having from one to six carbon atoms;

or

an alkyl group having from one to six carbon atoms and being substituted by at least one of substituents (c′) below;

<u>substituents (c′):</u>

hydroxy groups, $C_1$-$C_6$ alkoxy groups, $C_6$-$C_{14}$ carbocyclic aryl groups having from 0 to 3 of substituents (b′) as defined in Claim 4, heterocyclic groups having from 3 to 10 ring atoms of which 1 to 4 are nitrogen and/or oxygen and/or sulphur, said heterocyclic group being unsubstituted or substituted by at least one of substituents (b′) as defined in Claim 4, halogen atoms, cyano groups, carboxy groups, amino groups, $C_1$-$C_6$ alkylamino groups, dialkylamino groups wherein each alkyl part is $C_1$-$C_6$ alkyl, carbamoyl groups, alkylcarbamoyl groups where the alkyl part is $C_1$-$C_6$ alkyl, dialkylcarbamoyl groups where each alkyl part is $C_1$-$C_6$ alkyl, $C_1$-$C_6$ alkylthio groups, $C_6$-$C_{14}$ carbocyclic arylthio groups, $C_1$-$C_6$ alkylsulphonyl groups, $C_6$-$C_{14}$ carbocyclic arylsulphonyl groups wherein the aryl part has from 0 to 3 $C_1$-$C_6$ alkyl substituents, aminosulphonyl groups, $C_1$-$C_6$ alkylsulphinyl groups and $C_6$-$C_{14}$ carbocyclic arylsulphinyl groups wherein the aryl part has from 0 to 3 $C_1$-$C_6$ alkyl substituents;

provided that, where a substituent (c′) is substituted by a substituent (b′), that further substituent (b′) is not itself substituted.

**12.** A compound according to any preceding Claim, wherein $R^3$ represents

an alkyl group having from one to six carbon atoms;

or

an alkyl group having from one to six carbon atoms and being substituted by at least one of a cyano group, a carbamoyl group, a cycloalkyl group having from three to ten carbon atoms, a carbocyclic aryl group having from six to fourteen carbon atoms, a heterocyclic group having from 3 to 10 ring atoms, of which from 1 to 4 are nitrogen and/or oxygen and/or sulphur heteroatoms, a hydroxy group, a halogen atom, an amino group, a $C_1$-$C_6$ alkylthio group, a $C_1$-$C_6$ alkylsulphonyl group, an aminosulphonyl group and a carboxy group.

**13.** A compound according to any preceding Claim, wherein $R^3$ is selected from an alkyl group having from one to six carbon atoms and an alkyl group having from one to six carbon atoms and being substituted by at least one substituent selected from: cyano groups, hydroxy groups, carboxy groups, carbamoyl groups, $C_1$-$C_6$, mono- or di- alkylcarbamoyl groups, $C_1$-$C_6$ alkylthio groups, $C_1$-$C_6$ alkylsulphonyl groups and aminosulphonyl groups.

**14.** A compound according to Claim 13, wherein $R^3$ is selected from: carbamoylmethyl, 2-carbamoylethyl, dimethylcarbamoylmethyl, hydroxymethyl, 2-hydroxyethyl, cyanomethyl, 2-cyanoethyl, carboxymethyl, 2-carboxyethyl, methylthiomethyl, 2-methylthioethyl, methanesulphonylmethyl, 2-methanesulphonylethyl, sulphamoylmethyl and 2-sulphamoylethyl.

**15.** A compound according to any preceding Claim, wherein $R^3$ represents an alkyl group having from one to six carbon atoms and being substituted by at least one of a cyano group, a hydroxy group, a carbamoyl group, a $C_1$-$C_6$ mono- or di- alkylcarbamoyl group, a $C_1$-$C_6$ alkylthio group, a $C_1$-$C_6$ alkylsulphonyl group, an aminosulphonyl group and a carboxy group.

**16.** A compound according to any preceding Claim, wherein $R^4$ represents

an alkyl group having from one to six carbon atoms;

or

an alkyl group having from one to six carbon atoms and being substituted by at least one of substituents (a′) as defined in Claim 4.

**17.** A compound according to any preceding Claim, wherein $R^4$ represents an alkyl group having from one to six carbon atoms and being substituted by at least one substituent selected from

cycloalkyl groups having from three to ten carbon atoms,

carbocyclic aryl groups having from six to fourteen carbon atoms,

heterocyclic groups having from 3 to 10 ring atoms of which from 1 to 4 are nitrogen and/or oxygen and/or sulphur heteroatoms,

hydroxy groups,

halogen atoms, and

amino groups.

**18.** A compound according to any preceding Claim, wherein $R^4$ represents an alkyl group having from one to six carbon atoms and being substituted by at least one substituent selected from

cycloalkyl groups having from three to ten carbon atoms,

carbocyclic aryl groups having from six to fourteen carbon atoms, and

heterocyclic groups having from 3 to 10 ring atoms of which from 1 to 4 are nitrogen and/or oxygen and/or sulphur heteroatoms.

**19.** A compound according to any preceding Claim, wherein $R^4$ represents an alkyl group having one or two carbon atoms and being substituted by at least one of a cycloalkyl group having from three to six carbon atoms, and a carbocyclic aryl group having from six to ten carbon atoms.

**20.** A compound according to any preceding Claim, wherein $R^4$ represents an alkyl group having one or two carbon atoms and being substituted by a carbocyclic aryl group having six or ten carbon atoms.

**21.** A compound according to any preceding Claim, wherein $R^4$ represents a benzyl or a cyclohexylmethyl group.

**22.** A compound according to any preceding Claim, wherein $R^5$ represents a group of formula -B-(CO)-Y-$R^c_y$, wherein

B represents a heterocyclic group having from 3 to 10 ring atoms of which from 1 to 4 are nitrogen and/or oxygen and/or sulphur heteroatoms, at least one of which is a nitrogen heteroatom, said heterocyclic group being unsubstituted or substituted with at least one alkyl group having from one to six carbon atoms;

Y represents an oxygen atom or a nitrogen atom;

y is the integer 1 when Y represents an oxygen atom and 2 when Y represents a nitrogen atom; and

$R^c$ represents

a hydrogen atom,

an alkyl group having from one to six carbon atoms,

an alkyl group having from one to six carbon atoms and being substituted by at least one of substituents (a′) as defined in Claim 4;

and, where there are two groups or atoms represented by $R^c$, they are the same or different.

**23.** A compound according to any preceding Claim, wherein $R^5$ represents a group of formula -B-(CO)-Y-$R^c$, wherein

B represents a heterocyclic group having from 3 to 10 ring atoms of which from 1 to 4 are nitrogen and/or oxygen and/or sulphur heteroatoms, at least one of which is a nitrogen heteroatom, said heterocyclic group being unsubstituted or substituted with at least one alkyl group having from one to six carbon atoms;

Y represents an oxygen atom; and

$R^c$ represents

a hydrogen atom,

an alkyl group having from one to six carbon atoms, or

an alkyl group having from one to six carbon atoms and being substituted by at least one substituent selected from:

cycloalkyl groups having from three to ten carbon atoms,

carbocyclic aryl groups having from six to fourteen carbon atoms,

heterocyclic groups having from 3 to 10 ring atoms of which from 1 to 4 are nitrogen and/or oxygen and/or sulphur heteroatoms, and hydroxy groups.

**24.** A compound according to any preceding Claim, wherein $R^5$ represents a group of formula -B-(CO)-Y-$R^c_y$, wherein

B represents a heterocyclic group having from 3 to 10 ring atoms of which from 1 to 4 are nitrogen and/or oxygen and/or sulphur heteroatoms, at least one of which is a nitrogen heteroatom, said heterocyclic group being unsubstituted or substituted with at least one alkyl group having from one to six carbon atoms;

Y represents a nitrogen atom;

y is the integer 2; and

$R^c$ represents

a hydrogen atom,

an alkyl group having from one to six carbon atoms, or

an alkyl group having from one to six carbon atoms and being substituted by at least one substituent selected from:

cycloalkyl groups having from three to ten carbon atoms,

carbocyclic aryl groups having from six to fourteen carbon atoms,

heterocyclic groups having from 3 to 10 ring atoms of which from 1 to 4 are nitrogen and/or oxygen and/or sulphur heteroatoms, and hydroxy groups;

and, where there are two groups or atoms represented by $R^c$, they are the same or different.

**25.** A compound according to any preceding Claim, wherein $R^5$ represents a group of formula -B-(CO)-Y-$R^c_y$, wherein

B represents a heterocyclic group having from 4 to 6 ring atoms of which from 1 to 4 are nitrogen

and/or oxygen and/or sulphur heteroatoms, at least one of which is a nitrogen heteroatom, said heterocyclic group being unsubstituted or substituted with at least one alkyl group having from one to six carbon atoms;

Y represents a nitrogen atom;

y is the integer 2; and

$R^c$ represents

a hydrogen atom,

an alkyl group having from one to six carbon atoms, or

an alkyl group having from one to six carbon atoms and being substituted by at least one substituent selected from:

cycloalkyl groups having from five to ten carbon atoms,

carbocyclic aryl groups having six or ten carbon atoms,

heterocyclic groups having from 4 to 6 ring atoms of which from 1 to 4 are nitrogen and oxygen heteroatoms, and

hydroxy groups;

and, where there are two groups or atoms represented by $R^c$, they are the same or different.

**26.** A compound according to any preceding Claim, wherein $R^5$ represents a group of formula $-B-(CO)-Y-R^c_y$, wherein

B represents a heterocyclic group selected from pyrrolidinyl, thiazolidinyl and oxazolidinyl;

Y represents a nitrogen atom;

y is the integer 2; and

$R^c$ represents

a hydrogen atom,

an alkyl group having from one to six carbon atoms, or

an alkyl group having from one to six carbon atoms and being substituted by at least one substituent selected from:

cycloalkyl groups having from five to ten carbon atoms,

carbocyclic aryl groups having six or ten carbon atoms,

heterocyclic groups having from 4 to 6 ring atoms of which from 1 to 4 are nitrogen and oxygen heteroatoms, and

hydroxy groups;

and, where there are two groups or atoms represented by $R^c$, they are the same or different.

**27.** A compound according to Claim 1, which is [3-(N-2′-quinolinecarbonyl-L-asparaginyl)amino-2-hydroxy-4-phenylbutyryl]-L-proline t-butyl ester.

**28.** A compound according to Claim 1, which is [3-(N-3′-quinolinecarbonyl-L-asparaginyl)amino-2-hydroxy-4-phenylbutyryl]-L-proline t-butyl ester.

**29.** A compound according to Claim 1, which is [3-(N-2′-benzofurancarbonyl-L-asparaginyl)amino-2-hydroxy-4-phenylbutyryl]-L-proline t-butyl ester.

**30.** A compound according to Claim 1, which is [3-(N-2′-indolecarbonyl-L-asparaginyl)amino-2-hydroxy-4-phenylbutyryl]-L-proline t-butyl ester.

**31.** A compound according to Claim 1, which is N-{[3-(N-2′-quinolinecarbonyl-L-asparaginyl)amino-2-hydroxy-4-phenylbutyryl]-L-prolyl}-N-t-butylamine.

**32.** A compound according to Claim 1, which is N-{[3-(N-3′-quinolinecarbonyl-L-asparaginyl)amino-2-hydroxy-4-phenylbutyryl]-L-prolyl}-N-t-butylamine.

**33.** A compound according to Claim 1, which is N-{[3-(N-2′-quinoxalinecarbonyl-L-asparaginyl)amino-2-hydroxy-4-phenylbutyryl]-L-prolyl}-N-t-butylamine.

**34.** A compound according to Claim 1, which is N-{[3-(N-p-methoxyphenoxyacetyl-L-asparaginyl)amino-2-hydroxy-4-phenylbutyryl]-L-prolyl}-N-t-butylamine.

**35.** A compound according to Claim 1, which is N-{[3-(N-quinoxaline-2′-carbonyl-L-asparaginyl)amino-2-hydroxy-4-phenylbutyryl]-L-prolyl}-2-methylalaninol.

**36.** A compound according to Claim 1, which is 4-t-butylaminocarbonyl-1-[3-(N-2′-indolecarbonyl-L-asparaginyl)amino-2-hydroxy-4-phenylbutyryl]thiazolidine

**37.** A compound according to Claim, which is 4-t-butylaminocarbonyl-5,5-dimethyl-1-[3-(N-quinoxaline-2′-carbonyl-L-asparaginyl)amino-2-hydroxy-4-phenylbutyryl]-thiazolidine.

**38.** A compound according to Claim 1, which is 4-t-butylaminocarbonyl-5,5-dimethyl-1-[3-(N-2′-indolecarbonyl-L-asparaginyl)amino-2-hydroxy-4-phenylbutyryl]thiazolidine.

**39.** A compound according to Claim 1, which is 4-t-butylaminocarbonyl-1-[3-(N-2′-indolecarbonyl-L-3-methylthioalanyl)amino-2-hydroxy-4-phenylbutyryl]thiazolidine.

**40.** A compound according to Claim 1, which is N-{[2-hydroxy-3-(N-p-methylphenoxyacetyl-L-3-methanesulphonylalanyl)amino-4-phenylbutyryl]-L-prolyl}-N-t-butylamine.

**41.** A compound according to Claim 1, which is N-{[2-hydroxy-4-phenylbutyryl-3-(N-2′-quinolinecarbonyl-L-3-methanesulphonylalanyl)amino]-L-prolyl}-N-t-butylamine.

**42.** A compound according to Claim 1, which is 4-t-butylaminocarbonyl-1-[2-hydroxy-4-phenylbutyryl-3-(N-2′-quinolinecarbonyl-L-3-methanesulphonylalanyl)amino] thiazolidine.

**43.** A compound according to Claim 1, which is 4-t-butylaminocarbonyl-5,5-dimethyl-1-{3-[N-(1-naphthyloxy)-acetyl-L-3-sulphamoylalanyl]amino-2-hydroxy-4-phenylbutyryl}thiazolidine.

**44.** A pharmaceutical composition for the treatment of acquired immunodeficiency syndrome in a mammal 1, which comprises a therapeutically effective amount of a compound of formula (I) as defined in any preceding Claim, or a pharmaceutically acceptable salt or ester thereof, in admixture with a pharmaceutically acceptable carrier, diluent or excipient.

**45.** A compound as defined in any of Claims 1 to 43 for use in therapy.

**46.** Use of a compound as defined in any of Claims 1 to 43 in the manufacture of a medicament for the treatment or prophylaxis of AIDS.

**47.** A process for the preparation of a compound as defined in any of Claims 1 to 43, which process comprises either:

a) reacting a compound of formula (V):

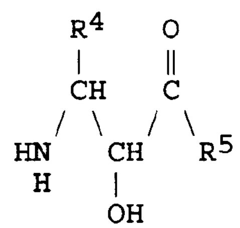

(V)

(in which: $R^4$ and $R^5$ are as defined above) with a compound of formula (VI):

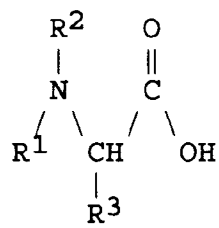

(VI)

(VI)

(in which $R^1$, $R^2$ and $R^3$ are as defined above);
or

b) reacting a compound of formula (IX):

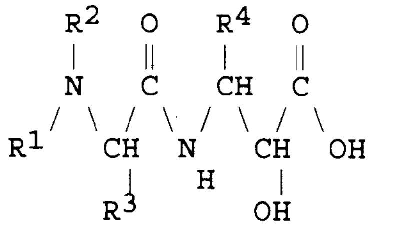

(IX)

(in which $R^1$, $R^2$, $R^3$ and $R^4$ are as defined above) with a group of formula (III):

$R^5$-H (III)

(in which $R^5$ is as defined above);
optionally removing any protecting group; and/or
optionally esterifying the compound; and/or
optionally salifying the compound.

**Claims for the Following Contracting States: ES, GR**

**1.** A process for preparing a compound of formula (I):

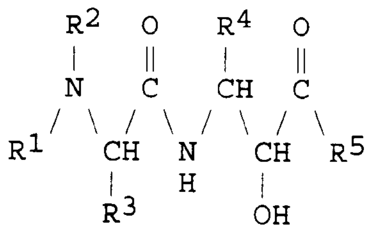

(I)

wherein:
$R^1$ represents a group of formula

$R(Z)_xA$-

wherein R represents
a hydrogen atom,
an alkyl group having from one to six carbon atoms,
an alkyl group having from one to six carbon atoms and being substituted by at least one of substituents (a) below,
an alkenyl group having from two to seven carbon atoms,
an alkenyl group having from two to seven carbon atoms and being substituted by at least one of substituents (a) below,
an alkynyl group having from two to seven carbon atoms,
an alkynyl group having from two to seven carbon atoms and being substituted by at least one of substituents (a) below,
a cycloalkyl group having from three to ten carbon atoms,
a cycloalkyl group having from three to ten carbon atoms and being substituted by at least one of substituents (b) below,
a carbocyclic aryl group having from six to fourteen carbon atoms,
a carbocyclic aryl group having from six to fourteen carbon atoms and being substituted by at least one of substituents (b) below, or
a heterocyclic group having from 3 to 10 ring atoms of which from 1 to 4 are nitrogen and/or oxygen and/or sulphur heteroatoms, said heterocyclic group being unsubstituted or substituted by at least one of substituents (b) below,
or R represents a group of formula $-NR^aR^b$ wherein $R^a$ and $R^b$ are independently selected from hydrogen atoms,
alkyl groups having from 1 to 4 carbon atoms, cycloalkyl groups having from three to ten carbon atoms,

cycloalkyl groups having from three to ten carbon atoms and being substituted by at least one of substituents (b) below,

heterocyclic groups having from 3 to 10 ring atoms of which from 1 to 4 are nitrogen and/or oxygen and/or sulphur heteroatoms, said heterocyclic group being unsubstituted or substituted by at least one of substituents (b) below,

carbocyclic aryl groups having from 6 to 14 carbon atoms,

carbocyclic aryl groups having from 6 to 14 carbon atoms and being substituted by at least one of substituents (b) below,

aralkenyl groups in which the aryl group has from 6 to 14 carbon atoms and the alkenyl group has from 2 to 7 carbon atoms, and

aralkyl groups in which the aryl group has from 6 to 14 carbon atoms and the alkyl group has from 1 to 3 carbon atoms;

Z represents oxygen or sulphur;

A represents a group of formula -CO-, -COCO-, -SO-, $-SO_2-$ or -CS-; and

x is the cipher 0 or the integer 1;

$R^2$ represents a hydrogen atom, an alkyl group having from one to six carbon atoms or an alkyl group having from one to six carbon atoms and being substituted with at least one of substituents (a) below;

$R^3$ represents

a hydrogen atom,

an alkyl group having from one to six carbon atoms, an alkyl group having from one to six carbon atoms and being substituted by at least one of substituents (c) below,

a cycloalkyl group having from three to ten carbon atoms,

a cycloalkyl group having from three to ten carbon atoms and being substituted by at least one of substituents (b) below,

an alkenyl group having from two to seven carbon atoms,

an alkenyl group having from two to seven carbon atoms and being substituted by at least one of substituents (a) below,

an alkynyl group having from two to seven carbon atoms,

an alkynyl group having from two to seven carbon atoms and being substituted by at least one of substituents (a) below,

a carbocyclic aryl group having from six to fourteen carbon atoms,

a carbocyclic aryl group having from six to fourteen carbon atoms and being substituted by at least one of substituents (b) below, or

a heterocyclic group having from 3 to 10 ring atoms of which from 1 to 4 are nitrogen and/or oxygen and/or sulphur heteroatoms, said heterocyclic group being unsubstituted or substituted by at least one of substituents (b) below;

$R^4$ represents a hydrogen atom,

an alkyl group having from one to six carbon atoms,

an alkyl group having from one to six carbon atoms and being substituted by at least one of substituents (a) below,

a cycloalkyl group having from three to ten carbon atoms,

a cycloalkyl group having from three to ten carbon atoms and being substituted by at least one of substituents (b) below,

a carbocyclic aryl group having from six to fourteen carbon atoms,

a carbocyclic aryl group having from six to fourteen carbon atoms and being substituted by at least one of substituents (b) below, or

a heterocyclic group having from 3 to 10 ring atoms of which from 1 to 4 are nitrogen and/or oxygen and/or sulphur heteroatoms, said heterocyclic group being unsubstituted or substituted by at least one of substituents (b) below;

and

$R^5$ represents a group of formula $-B-(CO)-Y-R^c_y$ , wherein

B represents a heterocyclic group having from 3 to 10 ring atoms of which from 1 to 4 are nitrogen and/or oxygen and/or sulphur heteroatoms, at least one of which is a nitrogen heteroatom, said heterocyclic group being unsubstituted or substituted with at least one alkyl group having from one to six carbon atoms;

Y represents an oxygen atom, a nitrogen atom or a sulphur atom;

y is the integer 1 when Y represents an oxygen atom or a sulphur atom, and 2 when Y represents

a nitrogen atom; and

$R^c$ represents

a hydrogen atom,

an alkyl group having from one to six carbon atoms,

an alkyl group having from one to six carbon atoms and being substituted by at least one of substituents (a) below,

a cycloalkyl group having from three to ten carbon atoms,

a cycloalkyl group having from three to ten carbon atoms and being substituted by at least one of substituents (b) below,

an alkenyl group having from two to seven carbon atoms,

an alkenyl group having from two to seven carbon atoms and being substituted by at least one of substituents (a) below,

an alkynyl group having from two to seven carbon atoms,

an alkynyl group having from two to seven carbon atoms and being substituted by at least one of substituents (a) below,

a carbocyclic aryl group having from six to fourteen carbon atoms,

a carbocyclic aryl group having from six to fourteen carbon atoms and being substituted by at least one of substituents (b) below, or

a heterocyclic group having from 3 to 10 ring atoms of which from 1 to 4 are nitrogen and/or oxygen and/or sulphur heteroatoms, said heterocyclic group being unsubstituted or substituted by at least one of substituents (b) below;

and, where there are two groups or atoms represented by $R^c$, they are the same or different; and

substituents (a):

hydroxy groups, $C_3$-$C_{10}$ cycloalkyl groups, $C_1$-$C_6$ alkoxy groups, $C_1$-$C_6$ aliphatic acyloxy groups, $C_1$-$C_6$ aliphatic acyl groups, carboxy groups, $C_2$-$C_6$ alkoxycarbonyl groups, sulpho groups, halogen atoms, amino groups, $C_2$-$C_4$ aliphatic acylamino groups, alkylamino groups in which the alkyl part is $C_1$-$C_6$ alkyl, dialkylamino groups in which each alkyl part is $C_1$-$C_3$ alkyl, carbocyclic aryl groups having from six to fourteen carbon atoms, carbocyclic aryl groups having from six to fourteen carbon atoms and being substituted by at least one of substituents (b) below, carbocyclic aryloxy groups having from six to fourteen carbon atoms, carbocyclic aryloxy groups having from six to fourteen carbon atoms and being substituted by at least one of substituents (b) below, and heterocyclic groups having from 3 to 10 ring atoms of which from 1 to 4 are nitrogen and/or oxygen and/or sulphur heteroatoms, said heterocyclic group being unsubstituted or substituted by at least one of substituents (b) below;

substituents (b):

$C_1$-$C_6$ alkyl groups having from 0 to 3 of substituents (a), $C_1$-$C_6$ haloalkyl groups, $C_1$-$C_6$ aliphatic acyl groups having from 0 to 3 of substituents (a), $C_1$-$C_6$ alkylenedioxy groups, aralkyl groups wherein the alkyl part is $C_1$-$C_6$ alkyl and the aryl part is $C_6$-$C_{14}$ carbocyclic aryl which has from 0 to 3 of substituents (b), aralkyloxycarbonyl groups wherein the alkyl part is $C_1$-$C_6$ alkyl and the aryl part is $C_6$-$C_{14}$ carbocyclic aryl which has from 0 to 3 of substituents (b), hydroxy groups, $C_1$-$C_6$ alkoxy groups, $C_6$-$C_{14}$ carbocyclic aryl groups having from 0 to 3 of substituents (b), aralkyloxy groups where the alkyl part is $C_1$-$C_6$ alkyl and the aryl part is $C_6$-$C_{14}$ carbocyclic aryl which has from 0 to 3 of substituents (b), $C_6$-$C_{14}$ carbocyclic aryloxy groups having from 0-3 of substituents (b), heterocyclic groups having from 3 to 10 ring atoms of which 1 to 4 are nitrogen and/or oxygen and/or sulphur, said heterocyclic group being unsubstituted or substituted by at least one of substituents (b), halogen atoms, nitro groups, cyano groups, carboxy groups, alkoxycarbonyl groups having a total of from 2 to 7 carbon atoms, amino groups, $C_1$-$C_6$ alkylamino groups, dialkylamino groups wherein each alkyl part is $C_1$-$C_6$ alkyl, aliphatic or carbocyclic aromatic carboxylic acylamino groups, carbamoyl groups, alkylcarbamoyl groups where the alkyl part is $C_1$-$C_6$ alkyl, dialkylcarbamoyl groups where each alkyl part is $C_1$-$C_6$ alkyl, mercapto groups, $C_1$-$C_6$ alkylthio groups, $C_6$-$C_{14}$ carbocyclic arylthio groups, $C_1$-$C_6$ alkylsulphonyl groups, $C_6$-$C_{14}$ carbocyclic arylsulphonyl groups wherein the aryl part has from 0 to 3 $C_1$-$C_6$ alkyl substituents, aminosulphonyl groups, $C_1$-$C_6$ alkylsulphinyl groups and $C_6$-$C_{14}$ carbocyclic arylsulphinyl groups wherein the aryl part has from 0 to 3 $C_1$-$C_6$ alkyl substituents;

substituents (c):

hydroxy groups, $C_1$-$C_6$ alkoxy groups, $C_6$-$C_{14}$ carbocyclic aryl groups having from 0 to 3 of substituents (b), aralkyloxy groups where the alkyl part is $C_1$-$C_6$ alkyl and the carbocyclic aryl part is $C_6$-$C_{14}$ carbocyclic aryl which has from 0 to 3 of substituents (b), $C_6$-$C_{14}$ carbocyclic aryloxy groups having from 0-3 of substituents (b), heterocyclic groups having from 3 to 10 ring atoms of which 1 to 4 are nitrogen and/or oxygen and/or sulphur, said heterocyclic group being unsubstituted or substituted by at least one of substituents (b), halogen atoms, nitro groups, cyano groups, carboxy groups, alkoxycarbonyl groups having a total of

from 2 to 7 carbon atoms, amino groups, $C_1$-$C_6$ alkylamino groups, dialkylamino groups wherein each alkyl part is $C_1$-$C_6$ alkyl, aliphatic or carbocyclic aromatic carboxylic acylamino groups, carbamoyl groups, alkylcarbamoyl groups where the alkyl part is $C_1$-$C_6$ alkyl, dialkylcarbamoyl groups where each alkyl part is $C_1$-$C_6$ alkyl, mercapto groups, $C_1$-$C_6$ alkylthio groups, $C_6$-$C_{14}$ carbocyclic arylthio groups, $C_1$-$C_6$ alkylsulphonyl groups, $C_6$-$C_{14}$ carbocyclic arylsulphonyl groups wherein the aryl part has from 0 to 3 $C_1$-$C_6$ alkyl substituents, aminosulphonyl groups, $C_1$-$C_6$ alkylsulphinyl groups and $C_6$-$C_{14}$ carbocyclic arylsulphinyl groups wherein the aryl part has from 0 to 3 $C_1$-$C_6$ alkyl substituents;
provided that, where a substituent (a) is substituted by a substituent (b), then substituent (b) is not further substituted by a substituent (a); that where a substituent (b) is substituted by a substituent (a), then substituent (a) is not further substituted by a substituent (b); that, where substituent (b) is a group which is itself substituted by a further substituent (b), that further substituent (b) is not itself substituted; and that where a substituent (c) is substituted by a substituent (b), that further substituent (b) is not itself substituted, and pharmaceutically acceptable salts and esters thereof,
which process comprises either:

a) reacting a compound of formula (V):

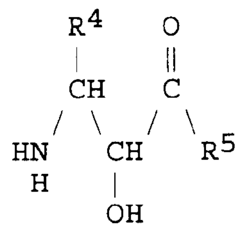

(V)

(in which: $R^4$ and $R^5$ are as defined above) with a compound of formula (VI):

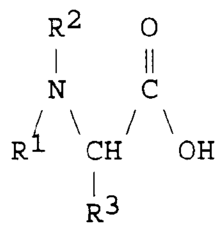

(VI)

(VI)

(in which $R^1$, $R^2$ and $R^3$ are as defined above);
or
b) reacting a compound of formula (IX):

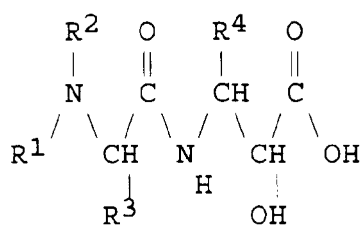

(IX)

(in which $R^1$, $R^2$, $R^3$ and $R^4$ are as defined above) with a group of formula (III):

$R^5$-H (III)

(in which $R^5$ is as defined above);
optionally removing any protecting group;and/or
optionally esterifying the compound; and/or
optionally salifying the compound.

2. A process according to claim 1, in which the reagents and reaction conditions are so chosen as to prepare a compound of formula (I) or a salt or ester thereof, wherein A represents a group of formula -CO- or $-SO_2-$ .

3. A process according to Claim 1, in which the reagents and reaction conditions are so chosen as to prepare a compound of formula (I) or a salt or ester thereof, wherein A represents a group of formula -CO-.

4. A process according to any preceding Claim, wherein the reagents and reaction conditions are so chosen as to prepare a compound of formula (I) or a salt or ester thereof, in which $R^1$ represents a group of formula

$$R(Z)_xA-$$

wherein R represents

a hydrogen atom,

an alkyl group having from one to six carbon atoms,

an alkyl group having from one to six carbon atoms and being substituted by at least one of substituents (a′) below,

a carbocyclic aryl group having from six to fourteen carbon atoms,

a carbocyclic aryl group having from six to fourteen carbon atoms and being substituted by at least one of substituents (b′) below,

or

a heterocyclic group having from 3 to 10 ring atoms of which from 1 to 4 are heteroatoms selected from nitrogen and/or oxygen and/or sulphur atoms, said heterocyclic group being unsubstituted or substituted by at least one of substituents (b′) below;

Z represents oxygen;

A represents a group of formula -CO- or $-SO_2-$; and

x is the cipher 0 or the integer 1;

substituents (a′):

hydroxy groups, $C_3$-$C_{10}$ cycloalkyl groups, $C_1$-$C_6$ alkoxy groups, $C_1$-$C_6$ aliphatic acyloxy groups, $C_1$-$C_6$ aliphatic acyl groups, carboxy groups, $C_2$-$C_6$ alkoxycarbonyl groups, sulpho groups, halogen atoms, amino groups, $C_2$-$C_4$ aliphatic acylamino groups, alkylamino groups in which the alkyl part is $C_1$-$C_3$ alkyl, dialkylamino groups in which each alkyl part is $C_1$-$C_3$ alkyl, carbocyclic aryl groups having from six to fourteen carbon atoms, carbocyclic aryl groups having from six to fourteen carbon atoms and being substituted by at least one of substituents (b′) below, carbocyclic aryloxy groups having from six to fourteen carbon atoms, carbocyclic aryloxy groups having from six to fourteen carbon atoms and being substituted by at least one of substituents (b′) below, and heterocyclic groups having from 3 to 10 ring atoms of which from 1 to 4 are nitrogen and/or oxygen and/or sulphur heteroatoms, said heterocyclic group being unsubstituted or substituted by at least one of substituents (b′) below;

substituents (b′):

$C_1$-$C_6$ alkyl groups having from 0 to 3 of substituents (a′), $C_1$-$C_2$ haloalkyl groups, $C_1$-$C_6$ aliphatic carboxylic acyl groups having from 0 to 3 of substituents (a′), hydroxy groups, $C_1$-$C_6$ alkoxy groups, $C_6$-$C_{14}$ carbocyclic aryl groups having from 0 to 3 of substituents (b′), halogen atoms, nitro groups, cyano groups, carboxy groups, amino groups, carbamoyl groups, mercapto groups, $C_1$-$C_6$ alkylthio groups, $C_1$-$C_6$ alkylsulphonyl groups and aminosulphonyl groups;

provided that, where a substituent (a′) is substituted by a substituent (b′), then substituent (b′) is not further substituted by a substituent (a′); that where a substituent (b′) is substituted by a substituent (a′), then substituent (a′) is not further substituted by a substituent (b′); and that, where substituent (b′) is a group which is itself substituted by a further substituent (b′), that further substituent (b′) is not itself substituted.

5. A process according to any preceding Claim, wherein the reagents and reaction conditions are so chosen as to prepare a compound of formula (I) or a salt or ester thereof, in which $R^1$ represents a group of formula

$$R (Z)_xA-$$

wherein R represents

an alkyl group having from one to six carbon atoms and being substituted by at least one of substituents (a′) as defined in Claim 4,

a carbocyclic aryl group having from six to fourteen carbon atoms,

a carbocyclic aryl group having from six to fourteen carbon atoms and being substituted by at least one of $C_{1-6}$ alkyl groups, $C_{1-6}$ alkoxy groups and hydroxy groups,

a heterocyclic group having from 3 to 10 ring atoms of which from 1 to 4 are nitrogen and/or oxygen and/or sulphur heteroatoms, said heterocyclic group being unsubstituted or substituted by at least one of $C_1$-$C_6$ alkyl groups, $C_1$-$C_6$ alkoxy groups and hydroxy groups,

Z represents oxygen;
A represents a group of formula -CO- or $-SO_2-$; and
x is the cipher 0 or the integer 1.

6. A process according to any preceding Claim, wherein the reagents and reaction conditions are so chosen as to prepare a compound of formula (I) or a salt or ester thereof, in which $R^1$ represents a group of formula

$$R(Z)_xA-$$

wherein R represents a group of formula $-NR^aR^b$ wherein $R^a$ and $R^b$ are independently selected from
heterocyclic groups having from 3 to 10 ring atoms of which from 1 to 4 are nitrogen and/or oxygen and/or sulphur heteroatoms, said heterocyclic group being unsubstituted or substituted by at least one $C_1$-$C_6$ alkyl group, $C_1$-$C_6$ alkoxy group or hydroxy group;
carbocyclic aryl groups having from 6 to 14 carbon atoms; and
aralkyl groups in which the aryl part has from 6 to 14 carbon atoms and the alkyl part has from 1 to 3 carbon atoms;
A represents a group of formula -CO-; and
x is the cipher 0.

7. A process according to any preceding Claim, wherein the reagents and reaction conditions are so chosen as to prepare a compound of formula (I) or a salt or ester thereof, in which $R^1$ represents a group of formula

$$R (Z)_xA-$$

wherein R represents
an alkyl group having from one to six carbon atoms and being substituted by at least one of phenyl, naphthyl, phenoxy and naphthoxy, or phenyl, naphthyl, phenoxy or naphthoxy substituted by at least one of methyl, hydroxy, methoxy and phenoxy groups,
a carbocyclic aryl group having from six to fourteen carbon atoms,
a heterocyclic group having from 3 to 10 ring atoms of which from 1 to 4 are heteroatoms selected from nitrogen and/or oxygen and/or sulphur heteroatoms, said heterocyclic group being unsubstituted or substituted by at least one of $C_1$-$C_6$ alkyl groups, $C_1$-$C_6$ alkoxy groups and hydroxy groups,
A represents a group of formula -CO-;
Z represents an oxygen atom; and
x is the cipher 0 or the integer 1.

8. A process according to any preceding Claim, wherein the reagents and reaction conditions are so chosen as to prepare a compound of formula (I) or a salt or ester thereof, in which $R^1$ represents a group of formula

$$R (Z)_xA-$$

wherein R represents
an alkyl group having from one to six carbon atoms and being substituted by at least one of phenyl, phenoxy, 4-methoxyphenyl, 4-methoxyphenoxy, 3-phenylphenoxy, naphthyl and naphthoxy groups,
a carbocyclic aryl group having from six to fourteen carbon atoms,
a benzofuranyl, indolyl, quinolyl and quinoxalinyl group, which is unsubstituted or substituted by at least one of $C_1$-$C_6$ alkyl groups, $C_1$-$C_6$ alkoxy groups and hydroxy groups,
A represents a group of formula -CO-;
Z represents an oxygen atom; and
x is the cipher 0 or the integer 1.

9. A process according to any preceding Claim, wherein the reagents and reaction conditions are so chosen as to prepare a compound of formula (I) or a salt or ester thereof, in which $R^2$ represents a hydrogen atom, an alkyl group having from one to four carbon atoms or an alkyl group having from one to four carbon atoms and being substituted with at least one of substituents (a′) as defined in Claim 4.

10. A process according to Claim 9, wherein the reagents and reaction conditions are so chosen as to prepare a compound of formula (I) or a salt or ester thereof, in which $R^2$ represents a hydrogen atom.

11. A process according to any preceding Claim, wherein the reagents and reaction conditions are so chosen as to prepare a compound of formula (I) or a salt or ester thereof, in which $R^3$ represents
an alkyl group having from one to six carbon atoms;
or
an alkyl group having from one to six carbon atoms and being substituted by at least one of substituents (c′) below;
substituents (c′):
hydroxy groups, $C_1$-$C_6$ alkoxy groups, $C_6$-$C_{14}$ carbocyclic aryl groups having from 0 to 3 of substituents (b′) as defined in Claim 4, heterocyclic groups having from 3 to 10 ring atoms of which 1 to 4 are nitrogen and/or oxygen and/or sulphur, said heterocyclic group being unsubstituted or substituted by at least one of substituents (b′) as defined in Claim 4, halogen atoms, cyano groups, carboxy groups, amino groups, $C_1$-$C_6$ alkylamino groups, dialkylamino groups wherein each alkyl part is $C_1$-$C_6$ alkyl, carbamoyl groups, alkylcarbamoyl groups where the alkyl part is $C_1$-$C_6$ alkyl, dialkylcarbamoyl groups where each alkyl part is $C_1$-$C_6$ alkyl, $C_1$-$C_6$ alkylthio groups, $C_6$-$C_{14}$ carbocyclic arylthio groups, $C_1$-$C_6$ alkylsulphonyl groups, $C_6$-$C_{14}$ carbocyclic arylsulphonyl groups wherein the aryl part has from 0 to 3 $C_1$-$C_6$ alkyl substituents, aminosulphonyl groups, $C_1$-$C_6$ alkylsulphinyl groups and $C_6$-$C_{14}$ carbocyclic arylsulphinyl groups wherein the aryl part has from 0 to 3 $C_1$-$C_6$ alkyl substituents;
provided that, where a substituent (c′) is substituted by a substituent (b′), that further substituent (b′) is not itself substituted.

**12.** A process according to any preceding Claim, wherein the reagents and reaction conditions are so chosen as to prepare a compound of formula (I) or a salt or ester thereof, in which $R^3$ represents
an alkyl group having from one to six carbon atoms;
or
an alkyl group having from one to six carbon atoms and being substituted by at least one of a cyano group, a carbamoyl group, a cycloalkyl group having from three to ten carbon atoms, a carbocyclic aryl group having from six to fourteen carbon atoms, a heterocyclic group having from 3 to 10 ring atoms, of which from 1 to 4 are nitrogen and/or oxygen and/or sulphur heteroatoms, a hydroxy group, a halogen atom, an amino group, a $C_1$-$C_6$ alkylthio group, a $C_1$-$C_6$ alkylsulphonyl group, an aminosulphonyl group and a carboxy group.

**13.** A process according to any preceding Claim, wherein the reagents and reaction conditions are so chosen as to prepare a compound of formula (I) or a salt or ester thereof, in which $R^3$ is selected from an alkyl group having from one to six carbon atoms and an alkyl group having from one to six carbon atoms and being substituted by at least-one substituent selected from: cyano groups, hydroxy groups, carboxy groups, carbamoyl groups, $C_1$-$C_6$, mono- or di-alkylcarbamoyl groups, $C_1$-$C_6$ alkylthio groups, $C_1$-$C_6$ alkylsulphonyl groups and aminosulphonyl groups.

**14.** A process according to Claim 13, wherein the reagents and reaction conditions are so chosen as to prepare a compound of formula (I) or a salt or ester thereof, in which $R^3$ is selected from: carbamoylmethyl, 2-carbamoylethyl, dimethylcarbamoylmethyl, hydroxymethyl, 2-hydroxyethyl, cyanomethyl, 2-cyanoethyl, carboxymethyl, 2-carboxyethyl, methylthiomethyl, 2-methylthioethyl, methanesulphonylmethyl, 2-methanesulphonylethyl, sulphamoylmethyl and 2-sulphamoylethyl.

**15.** A process according to any preceding Claim, wherein the reagents and reaction conditions are so chosen as to prepare a compound of formula (I) or a salt or ester thereof, in which $R^3$ represents an alkyl group having from one to six carbon atoms and being substituted by at least one of a cyano group, a hydroxy group, a carbamoyl group, a $C_1$-$C_6$ mono- or di-alkylcarbamoyl group, a $C_1$-$C_6$ alkylthio group, a $C_1$-$C_6$ alkylsulphonyl group, an aminosulphonyl group and a carboxy group.

**16.** A process according to any preceding Claim, wherein the reagents and reaction conditions are so chosen as to prepare a compound of formula (I) or a salt or ester thereof, in which $R^4$ represents
an alkyl group having from one to six carbon atoms;
or
an alkyl group having from one to six carbon atoms and being substituted by at least one of substituents (a′) as defined in Claim 4.

**17.** A process according to any preceding Claim, wherein the reagents and reaction conditions are so chosen as to prepare a compound of formula (I) or a salt or ester thereof, in which $R^4$ represents an alkyl group having from one to six carbon atoms and being substituted by at least one substituent selected from
cycloalkyl groups having from three to ten carbon atoms,
carbocyclic aryl groups having from six to fourteen carbon atoms,
heterocyclic groups having from 3 to 10 ring atoms of which from 1 to 4 are nitrogen and/or oxygen and/or sulphur heteroatoms,

hydroxy groups,
halogen atoms, and
amino groups.

**18.** A process according to any preceding Claim, wherein the reagents and reaction conditions are so chosen as to prepare a compound of formula (I) or a salt or ester thereof, in which $R^4$ represents an alkyl group having from one to six carbon atoms and being substituted by at least one of
cycloalkyl groups having from three to ten carbon atoms,
carbocyclic aryl groups having from six to fourteen carbon atoms, and
heterocyclic groups having from 3 to 10 ring atoms of which from 1 to 4 are nitrogen and/or oxygen and/or sulphur heteroatoms.

**19.** A process according to any preceding Claim, wherein the reagents and reaction conditions are so chosen as to prepare a compound of formula (I) or a salt or ester thereof, in which $R^4$ represents an alkyl group having one or two carbon atoms and being substituted by at least one of a cycloalkyl group having from three to six carbon atoms, and a carbocyclic aryl group having from six to ten carbon atoms.

**20.** A process according to any preceding Claim, wherein the reagents and reaction conditions are so chosen as to prepare a compound of formula (I) or a salt or ester thereof, in which $R^4$ represents an alkyl group having one or two carbon atoms and being substituted by a carbocyclic aryl group having six or ten carbon atoms.

**21.** A process according to any preceding Claim, wherein the reagents and reaction conditions are so chosen as to prepare a compound of formula (I) or a salt or ester thereof, in which $R^4$ represents a benzyl or a cyclohexylmethyl group.

**22.** A process according to any preceding Claim, wherein the reagents and reaction conditions are so chosen as to prepare a compound of formula (I) or a salt or ester thereof, in which $R^5$ represents a group of formula $-B-(CO)-Y-R^c_y$ , wherein
B represents a heterocyclic group having from 3 to 10 ring atoms of which from 1 to 4 are nitrogen and/or oxygen and/or sulphur heteroatoms, at least one of which is a nitrogen heteroatom, said heterocyclic group being unsubstituted or substituted with at least one alkyl group having from one to six carbon atoms;
Y represents an oxygen atom or a nitrogen atom;
y is the integer 1 when Y represents an oxygen atom and 2 when Y represents a nitrogen atom; and
$R^c$ represents
a hydrogen atom,
an alkyl group having from one to six carbon atoms,
an alkyl group having from one to six carbon atoms and being substituted by at least one of substituents (a′) as defined in Claim 4;
and, where there are two groups or atoms represented by $R^c$, they are the same or different.

**23.** A process according to any preceding Claim, wherein the reagents and reaction conditions are so chosen as to prepare a compound of formula (I) or a salt or ester thereof, in which $R^5$ represents a group of formula $-B-(CO)-Y-R^c$, wherein
B represents a heterocyclic group having from 3 to 10 ring atoms of which from 1 to 4 are nitrogen and/or oxygen and/or sulphur heteroatoms, at least one of which is a nitrogen heteroatom, said heterocyclic group being unsubstituted or substituted with at least one alkyl group having from one to six carbon atoms;
Y represents an oxygen atom; and
$R^c$ represents
a hydrogen atom,
an alkyl group having from one to six carbon atoms, or
an alkyl group having from one to six carbon atoms and being substituted by at least one substituent selected from:
cycloalkyl groups having from three to ten carbon atoms,
carbocyclic aryl groups having from six to fourteen carbon atoms,

heterocyclic groups having from 3 to 10 ring atoms of which from 1 to 4 are heteroatoms selected from nitrogen and/or oxygen and/or sulphur heteroatoms, and
hydroxy groups.

**24.** A process according to any preceding Claim, wherein the reagents and reaction conditions are so chosen as to prepare a compound of formula (I) or a salt or ester thereof, in which $R^5$ represents a group of formula $-B-(CO)-Y-R^c_y$ , wherein

B represents a heterocyclic group having from 3 to 10 ring atoms of which from 1 to 4 are nitrogen and/or oxygen and/or sulphur heteroatoms, at least one of which is a nitrogen heteroatom, said heterocyclic group being unsubstituted or substituted with at least one alkyl group having from one to six carbon atoms;
Y represents a nitrogen atom;
y is the integer 2; and
$R^c$ represents
a hydrogen atom,
an alkyl group having from one to six carbon atoms, or
an alkyl group having from one to six carbon atoms and being substituted by at least one substituent selected from:
cycloalkyl groups having from three to ten carbon atoms,
carbocyclic aryl groups having from six to fourteen carbon atoms,
heterocyclic groups having from 3 to 10 ring atoms of which from 1 to 4 are heteroatoms selected from nitrogen and/or oxygen and/or sulphur heteroatoms, and
hydroxy groups;
and, where there are two groups or atoms represented by $R^c$, they are the same or different.

**25.** A process according to any preceding Claim, wherein the reagents and reaction conditions are so chosen as to prepare a compound of formula (I) or a salt or ester thereof, in which $R^5$ represents a group of formula $-B-(CO)-Y-R^c_y$ , wherein

B represents a heterocyclic group having from 4 to 6 ring atoms of which from 1 to 4 are nitrogen and/or oxygen and/or sulphur heteroatoms, at least one of which is a nitrogen heteroatom, said heterocyclic group being unsubstituted or substituted with at least one alkyl group having from one to six carbon atoms;
Y represents a nitrogen atom;
y is the integer 2; and
$R^c$ represents
a hydrogen atom,
an alkyl group having from one to six carbon atoms, or
an alkyl group having from one to six carbon atoms and being substituted by at least one substituent selected from:
cycloalkyl groups having from five to ten carbon atoms,
carbocyclic aryl groups having six or ten carbon atoms,
heterocyclic groups having from 4 to 6 ring atoms of which from 1 to 4 are nitrogen and
oxygen heteroatoms, and
hydroxy groups;
and, where there are two groups or atoms represented by $R^c$, they are the same or different.

**26.** A process according to any preceding Claim, wherein the reagents and reaction conditions are so chosen as to prepare a compound of formula (I) or a salt or ester thereof, in which $R^5$ represents a group of formula $-B-(CO)-Y-R^c_y$ , wherein

B represents a pyrrolidinyl, thiazolidinyl or a oxazolidinyl group;
Y represents a nitrogen atom;
y is the integer 2; and
$R^c$ represents
a hydrogen atom,
an alkyl group having from one to six carbon atoms, or
an alkyl group having from one to six carbon atoms and being substituted by at least one substituent selected from:

cycloalkyl groups having from five to ten carbon atoms,
carbocyclic aryl groups having six or ten carbon atoms,
heterocyclic groups having from 4 to 6 ring atoms of which from 1 to 4 are nitrogen and oxygen heteroatoms, and
hydroxy groups;
and, where there are two groups or atoms represented by $R^c$, they are the same or different.

**27.** A process according to Claim 1, wherein the reagents and reaction conditions are so chosen as to prepare a compound of formula (I) or a salt or ester thereof, in which one of the following compounds is prepared:
[3-(N-2′-quinolinecarbonyl-L-asparaginyl)amino-2-hydroxy-4-phenylbutyryl]-L-proline t-butyl ester;
[3-(N-3′-quinolinecarbonyl-L-asparaginyl)amino-2-hydroxy-4-phenylbutyryl]-L-proline t-butyl ester;
[3-(N-2′-benzofurancarbonyl-L-asparaginyl)amino-2-hydroxy-4-phenylbutyryl]-L-proline t-butyl ester;
[3-(N-2′-indolecarbonyl-L-asparaginyl)amino-2-hydroxy-4-phenylbutyryl]-L-proline t-butyl ester;
N-{[3-(N-2′-quinolinecarbonyl-L-asparaginyl)amino-2-hydroxy-4-phenylbutyryl]-L-prolyl}-N-t-butylamine;
N-{[3-(N-3′-quinolinecarbonyl-L-asparaginyl)amino-2-hydroxy-4-phenylbutyryl]-L-prolyl}-N-t-butylamine;
N-{[3-(N-2′-quinoxalinecarbonyl-L-asparaginyl)amino-2-hydroxy-4-phenylbutyryl]-L-prolyl}-N-t-butylamine;
N-{[3-(N-p-methoxyphenoxyacetyl-L-asparaginyl)amino-2-hydroxy-4-phenylbutyryl]-L-prolyl}-N-t-butylamine;
N-{[3-(N-quinoxaline-2′-carbonyl-L-asparaginyl)amino-2-hydroxy-4-phenylbutyryl]-L-prolyl}-2-methylalaninol;
4-t-butylaminocarbonyl-1-[3-(N-2′-indolecarbonyl-L-asparaginyl)amino-2-hydroxy-4-phenylbutyryl]thiazolidine;
4-t-butylaminocarbonyl-5,5-dimethyl-1-[3-(N-quinoxaline-2′-carbonyl-L-asparaginyl)amino-2-hydroxy-4-phenylbutyryl]thiazolidine;
4-t-butylaminocarbonyl-5,5-dimethyl-1-[3-(N-2′-indolecarbonyl-L-asparaginyl)amino-2-hydroxy-4-phenylbutyryl]thiazolidine;
4-t-butylaminocarbonyl-1-[3-(N-2′-indolecarbonyl-L-3-methyl-thioalanyl)amino-2-hydroxy-4-phenylbutyryl] thiazolidine;
N-{[2-hydroxy-3-(N-p-methylphenoxyacetyl-L-3-methanesulphonylalanyl)amino-4-phenylbutyryl]-L-prolyl}-N-t-butylamine;
N-{[2-hydroxy-4-phenylbutyryl-3-(N-2′-quinolinecarbonyl-L-3-methanesulphonyl-alanyl)amino]-L-prolyl}-N-t-butylamine;
4-t-butylaminocarbonyl-1-[2-hydroxy-4-phenylbutyryl-3-(N-2′-quinolinecarbonyl-L-3-methanesulphonylalanyl)amino]thiazolidine; and
4-t-butylaminocarbonyl-5,5-dimethyl-1-{3-[N-(1-naphthyloxy)acetyl-L-3-sulphamoylalanyl]amino-2-hydroxy-4-phenylbutyryl}thiazolidine.

**28.** Use of a compound according to any preceding claim, in the manufacture of a medicament for the treatment or prophylaxis of AIDS.

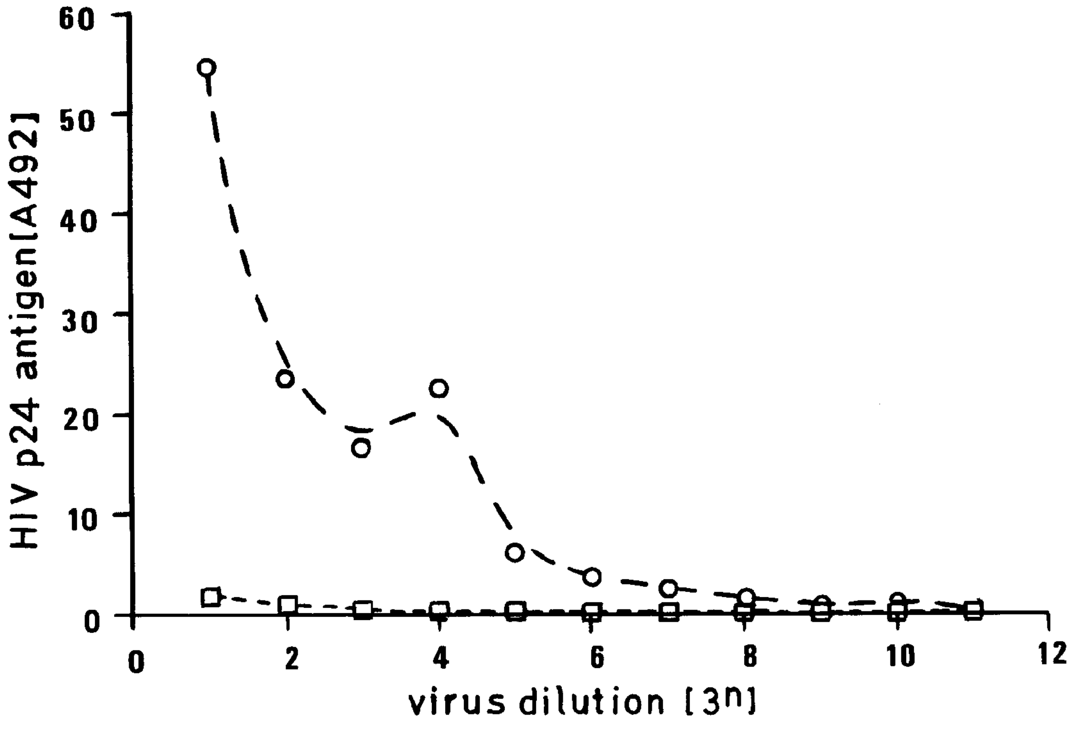
60
50
40
30
20
10
0
HIV p24 antigen [A492]
0
2
4
6
8
10
12
virus dilution [3n]

European Patent Office

# PARTIAL EUROPEAN SEARCH REPORT

which under Rule 45 of the European Patent Convention shall be considered, for the purposes of subsequent proceedings, as the European search report

Application Number

EP 92 30 1100

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A,D | EP-A-0 346 847 (HOFFMANN-LA ROCHE)<br>* Entire document *<br>--- | 1-4 | C 07 D 207/16<br>C 07 K 5/06<br>C 07 D 401/12<br>C 07 D 403/12<br>C 07 D 405/12<br>C 07 D 409/12<br>A 61 K 31/40<br>C 07 D 413/12<br>C 07 D 417/12<br>C 07 D 217/26 |
| P,A | CHEM. PHARM. BULL., vol. 39, no. 9, pages 2465-2467; T. MIMOTO et al.: "Rational design and synthesis of a novel class of active site-targeted HIV protease inhibitors containing a hydroxymethylcarbonyl isotere. Use of phenylnorstatine or allophenylnorstatine as a transition-state mimic"<br>* Entire document; table 1 *<br>--- | 1-4 | |
| A | JOURNAL OF MEDICINAL CHEMISTRY, vol. 20, no. 4, 1977, pages 510-515; R. NISHIZAWA et al.: "Synthesis and structure-activity relationships of bestatin analogues, inhibitors of aminopeptidase B"<br>* Entire document; compounds with RN'S 62023-67-0,62023-44-3 *<br>--- -/- | 1-4 | |

TECHNICAL FIELDS SEARCHED (Int. Cl.5)

C 07 D 207/00
C 07 K 5/00
C 07 D 401/00
C 07 D 403/00
C 07 D 405/00
C 07 D 409/00
C 07 D 413/00
C 07 D 417/00
C 07 D 217/00

## INCOMPLETE SEARCH

The Search Division considers that the present European patent application does not comply with the provisions of the European Patent Convention to such an extent that it is not possible to carry out a meaningful search into the state of the art on the basis of some of the claims

Claims searched completely : 26-43

Claims searched incompletely : 1-25,44-47

Claims not searched :

Reason for the limitation of the search:

see sheet -C-

| Place of search | Date of completion of the search | Examiner |
|---|---|---|
| THE HAGUE | 19-03-1992 | KISSLER B.E. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0407)

European Patent Office

# PARTIAL EUROPEAN SEARCH REPORT

Application Number
EP 92 30 1100

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (Int. Cl.5) |
| A | CHEMICAL ABSTRACTS, vol. 104, 1986, abstract no. 19822g, Columbus, Ohio, US; & CA-A-1 182 110 (MICROBIOCHEMICAL RESEARCH FOUNDATION) 05-02-1985 * Abstract; compound with RN 99429-59-1 * --- | 1-4 | |
| D,A P | J. MED. CHEM., vol. 34, no. 3, 1991, pages 1222-1225; D.H. RICH et al.: "Effect of hydroxyl group configuration in hydroxyethylamine dipeptide isosteres on HIV protease inhibition. Evidence for multiple binding modes" * Entire document * --- | 1-4 | TECHNICAL FIELDS SEARCHED (Int. Cl.5) |
| D,A | J. MED. CHEM., vol. 33, no. 5, 1990, pages 1285-1288; D.H. RICH et al.: "Hydroxyethylamine analogues of the p17/p24 substrate cleavage site are tight-binding inhibitors of HIV protease" * Entire document * ----- | 1-46 | |

EPO FORM 1503 03.82 (P0410)

EP 92 30 1100

-C-

Lack of conciseness
---------------------

The definition of the following substituent(s) is too general and/or encompasses too broad a range of totally different chemical groups, only partly supported by examples given in the descriptive part of the application:

R1, R2, R3, R4, R5

The vast number of theoretically conceivable compounds resulting from the combination of all claimed substituents of above list precludes a comprehensive search. Guided by the spirit of the application and the inventive concept as disclosed in the descriptive part of the present application the search has been limited to the following case(s):

compounds where R5 = -N-C-C=O and N-C bond is cyclic R1, R2, R3 and R4 undefined).

(Cf. Arts. 83, 84 EPC, Rule 45 EPC, Guidelines Exam. Part B, Chapt. III, 3.6, 3.7)